# EUROPEAN PATENT SPECIFICATION

(11) **EP 2 993 215 B1**
(45) Date of publication and mention of the grant of the patent: **19.06.2019**
(21) Application number: 14183598.3
(22) Date of filing: 04.09.2014
(51) Int. Cl.: C09K 11/06, H05B 33/10, H01L 51/00, C07D 487/00

(54) **Azabenzimidazo[2,1-a]benzimidazoles for electronic applications**
Azabenzimidazo[2,1-a]benzimidazole für elektronische Anwendungen
Azabenzimidazo[2,1-a]benzimidazoles pour applications électroniques

(43) Date of publication of application: 09.03.2016
(73) Proprietor: Idemitsu Kosan Co., Ltd., Chiyoda-ku Tokyo 100-8321 (JP)
(72) Inventor: Groarke, Michell, 4125 Riehen (CH); Schäfer, Thomas, 4410 Liestal (CH); Nagashima, Hideaki, 4057 Basel (CH); Raimann, Thomas, 4334 Sisseln (CH)
(74) Representative: Hollah, Dorothee

(56) References cited:
- WO-A1-2012/130709
- DE-A1-102010 024 542
- US-A1- 2012 241 681

## Description

The present invention relates to compounds of formulae (Ia) and (Ib), a process for their production and their use in electronic devices, especially electroluminescent devices. When used as charge transport material and/or host material for phosphorescent emitters in electroluminescent devices, the compounds of formulae (Ia) and (Ib) may provide improved efficiency, stability, manufacturability and/or spectral characteristics of electroluminescent devices.

DE102012000064 describes compounds of formula 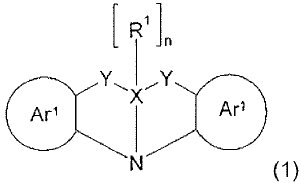 and their use in organic light emitting devices (OLEDs). Among others X can be C. If X is C, n is 1.

WO2012110182 describes compounds of formula 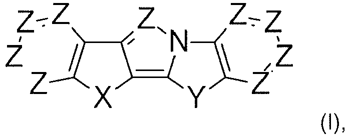 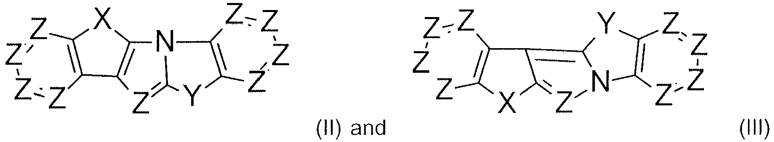 and their use in electronic devices, especially OLEDs. X and Y can be S and Z is CR², or N.

WO2012139692 relates to electronic devices which comprise an anode, a cathode and at least one organic layer, where the organic layer comprises one or more substituted benzene compounds of formula 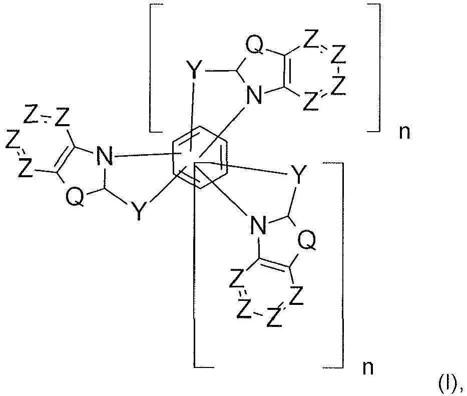 or 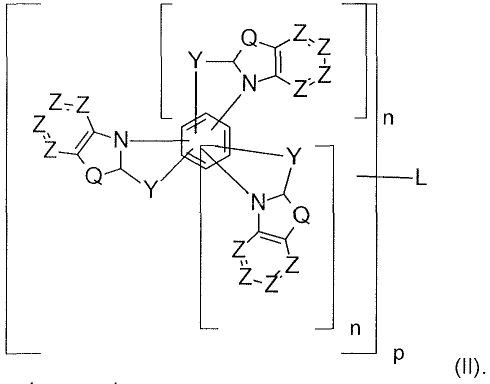 Y can be N and n can be 0 or 1, Z is CR¹ or N. R¹ can be an aromatic or hetero aromatic ring system.

B is 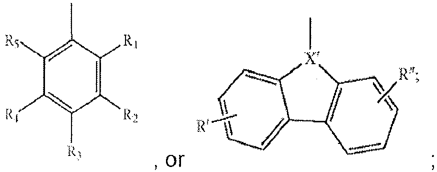 and their use in OLEDs.

WO2011160757 relates to an electronic device comprising an anode, cathode and at least one organic layer which may contain a compound of formulae 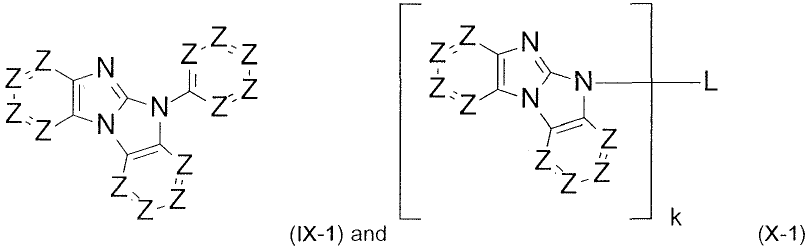 and their use in electronic devices, especially OLEDs. Z is CR², or N. The following 4H-Imidazo[1,2-a]imidazole compounds are explicitly disclosed: 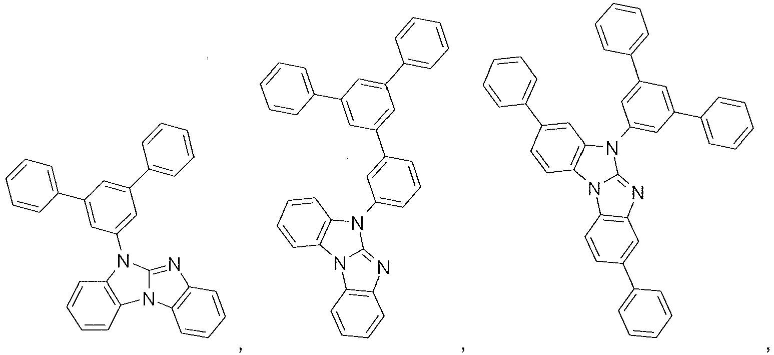 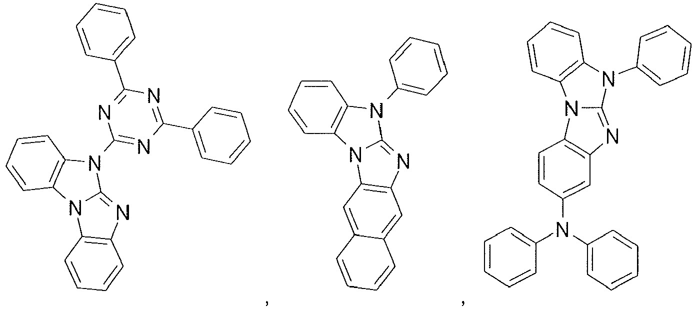 and

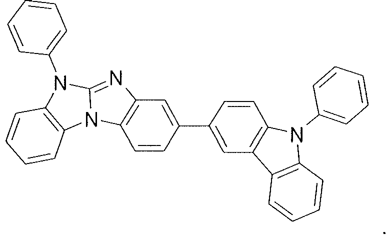

WO2012/130709 relates to 4H-Imidazo[1,2-a]imidazoles, 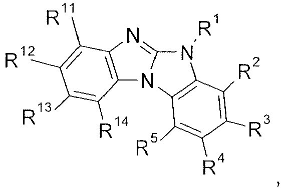 such as, for example, 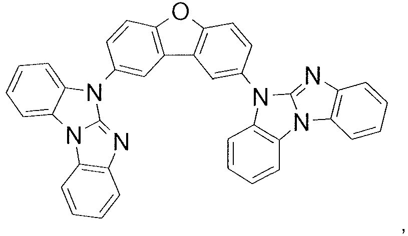 a process for their production and their use in electronic devices, especially electroluminescent devices.

WO2013/050401 describes 4H-imidazo[1,2-a]imidazoles of formula 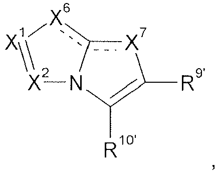 wherein X⁶ is -N= and X⁷ is -NR⁶-, or X⁷ is =N- and X⁶ is -NR⁶- R⁶ is a group of formula 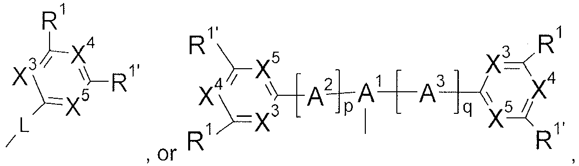 such as, for example, 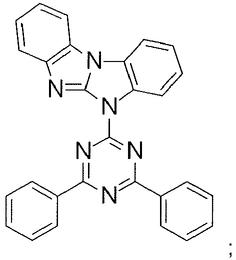 a process for their production and their use in electronic devices, especially electroluminescent devices.

WO2014/009317 relates to compounds of formula 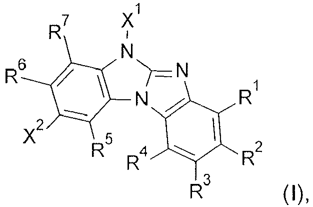 a process for their production and their use in electronic devices, especially electroluminescent devices. When used as host material for phosphorescent emitters in electroluminescent devices, the compounds of formula I may provide improved efficiency, stability, manufacturability, or spectral characteristics of electroluminescent devices.

WO2014/044722 relates to compounds of formula 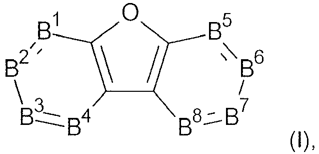 which are characterized in that they substituted by benzimidazo[1,2-a]benzimidazo-5-yl and/or benzimidazo[1,2-a]benzimidazo-2,5-ylene groups and in that at least one of the substituents B¹, B², B³, B⁴, B⁵, B⁶, B⁷ and B⁸ represents N; a process for their production and their use in electronic devices, especially electroluminescent devices.

El-Shafei et al reported the synthesis of benzimidazo[3,2:1',2']imidazo[4,5-b]quinoxaline, 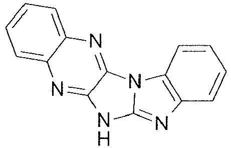 (Gazzetta Chimica Italiana, 1981, 409) by a condensation reaction with chloroazanaphthene.

Borisova et al reported the synthesis of 7-methylquinolino[4,5-b]imidazo[1,2-a]benzimidazole, 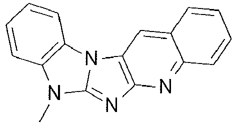 (Khimiya Geterotsiklicheskikh Soedinenii, 1973, 803) using a metal mediated reduction of nitrobenzilidene.

Tagdiwala et al reported the fluorescence details of imidazoquinoxalines derived from the reaction of 2,3-dichloroquinoxaline with 2-aminopyridine, 2-aminopyrimidine and 2-aminobenzimidazole (Indian Journal of Chemistry, Section B: Organic Chemistry Including Medicinal Chemistry 1986, 1057). Yamazoe et al investigated peroxidative activation of benzidine deriatives (Carcinogenesis, 1988, 1635).

DE 10 2010 024542 A1 concerns an electronic device wherein the organic layer comprises at least one compound of the following formulae (I) to (IV) 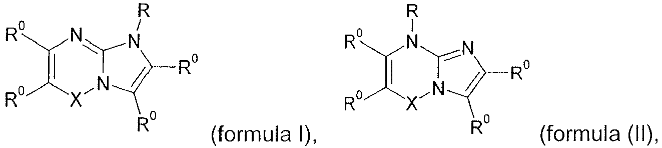 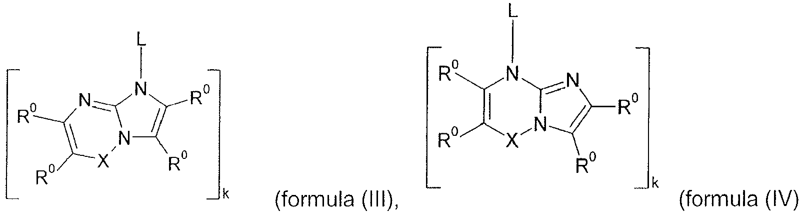 where the following applies to the symbols occurring:
X is on each occurrence, identically or differently, a single bond, C=O, C=S, C=NR¹, C(R¹)₂, C(R¹)₂-C(R¹)₂, CR¹=CR¹, Si(R¹)₂, NR¹, PR¹, P(=O)R¹, O, S, SO or SO₂;
L is a divalent, or in the case of k=3, 4, 5 or 6 a tri-, tetra-, penta- or hexavalent group respectively, selected from C=O, C=NR¹, Si(R¹)₂, P(=O)(R¹), SO, SO₂, alkylene groups having 1 to 20 C atoms, alkenylene or alkynylene groups having 2 to 20 C atoms, where, in the case of the groups mentioned, one or more CH₂ groups may be replaced by Si(R¹)₂, O, S, C=O, C=NR¹, C=O-O, C=O-NR¹, NR¹, P(=O)(R¹), SO or SO₂ and where one or more H atoms in the above-mentioned groups may be replaced by D, F, Cl, Br, I, CN or NO₂, aromatic or heteroaromatic ring systems having 5 to 60 aromatic ring atoms, each of which may be sub-stituted by one or more radicals R¹, and any desired combinations of 1, 2, 3, 4 or 5 identical or different groups selected from the above-mentioned groups; or L is a single bond, where k in this case must be equal to 2;
R⁰ is on each occurrence, identically or differently, H, D, F, Cl, Br, I, C(=O)R¹, OSO₂R¹, COOR¹, CON(R¹)₂, a straight-chain alkyl group having 1 to 40 C atoms or a branched or cyclic alkyl group having 3 to 40 C atoms or an alkenyl or alkynyl group having 2 to 40 C atoms, where the above-mentioned groups may each be substituted by one or more radicals R¹ and where one or more CH₂ groups in the above-mentioned groups may be replaced by Si(R¹)₂, Ge(R¹)₂, Sn(R¹)₂, C=O, C=S, C=Se, C=NR¹, P(=O)(R¹), SO, SO₂, NR¹, -O-, -S-, -COO- or -CONR¹- and where one or more H atoms in the above-mentioned groups may be replaced by D, F, Cl, Br, I, CN or NO₂, or an aromatic or heteroaromatic ring system having 5 to 60 aromatic ring atoms, which may in each case be substituted by one or more radicals R¹, or an aralkyl or heteroaralkyl group having 5 to 60 aromatic ring atoms, which may be substituted by one or more radicals R¹, or a combination of these systems, furthermore two or more adjacent radicals R⁰ may be linked to one another here and form an aliphatic or aromatic ring, or a radical R⁰ may be linked to an adjacent radical R via a single bond or a divalent group Y and form an aliphatic or aromatic ring;
R is equal to C(=O)R¹, OSO₂R¹, COOR¹, CON(R¹)₂, a straight-chain alkyl group having 1 to 40 C atoms or a branched or cyclic alkyl group having 3 to 40 C atoms or an alkenyl or alkynyl group having 2 to 40 C atoms, where the above-mentioned groups may each be substituted by one or more radicals R¹ and where one or more CH₂ groups in the above-mentioned groups may be replaced by Si(R¹)₂, Ge(R¹)₂, Sn(R¹)₂, C=O, C=S, C=Se, C=NR¹, P(=O)(R¹), SO, SO₂, NR¹, -O-, -S-, -COO- or -CONR¹- and where one or more H atoms in the above-mentioned groups may be replaced by D, F, Cl, Br, I, CN or NO₂, or an aromatic or heteroaromatic ring system having 5 to 60 aromatic ring atoms, which may in each case be substituted by one or more radicals R¹, or an aralkyl or heteroaralkyl group having 5 to 60 aromatic ring atoms, which may be substituted by one or more radicals R¹, or a combination of these systems, where furthermore the radical R may be linked to one or more adjacent radicals R⁰ via a single bond or a divalent group Y;
Y is on each occurrence, identically or differently, a divalent group selected from C=O, C=S, C=NR¹, C(R¹)₂, Si(R¹)₂, NR¹, PR¹, P(=O)R¹, O, S, SO and SO₂;
R¹ is on each occurrence, identically or differently, H, D, F, Cl, Br, I, CHO, N(R²)₂, C(=O)R², P(=O)(R²)₂, S(=O)R², S(=O)₂R², CN, NO₂, Si(R²)₃, B(OR²)₂, OSO₂R², OH, COOR², CON(R²)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, where the above-mentioned groups may each be substituted by one or more radicals R² and where one or more CH₂ groups in the above-mentioned groups may be replaced by Si(R²)₂, Ge(R²)₂, Sn(R²)₂, C=O, C=S, C=Se, C=NR², P(=O)(R²), SO, SO₂, NR², -O-, -S-, -COO- or -CONR²- and where one or more H atoms in the above-mentioned groups may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a combination of these systems, where two or more radicals R¹ may be linked to one another and may form an aliphatic or aromatic ring;
R² is, identically or differently on each occurrence, H, D, F or an aliphatic, aromatic and/or heteroaromatic organic radical having 1 to C atoms, in which, in addition, one or more H atoms may be replaced by D or F; two or more substituents R² here may also be linked to one another and form an aliphatic or aromatic ring; and
k is equal to 2, 3, 4, 5 or 6.

Notwithstanding these developments, there remains a need for organic light emitting devices comprising new charge transport materials to provide improved efficiency, stability, manufacturability, and/or spectral characteristics of electroluminescent devices.

Accordingly, it is an object of the present invention, with respect to the aforementioned prior art, to provide further materials suitable for use in OLEDs and further applications in organic electronics. More particularly, it should be possible to provide charge transport materials, charge/exciton blocker materials and matrix materials for use in OLEDs. The materials should be suitable especially for OLEDs which comprise at least one phosphorescence emitter, especially at least one green emitter or at least one blue emitter. Furthermore, the materials should be suitable for providing OLEDs which ensure good efficiencies, good operative lifetimes and a high stability to thermal stress, and a low use and operating voltage of the OLEDs.

Certain azabenzimidazo[1,2-a]benzimidazole derivatives are found to be suitable for use in organo-electroluminescent devices. In particular, said derivatives are suitable charge transport materials, or host materials for phosphorescent emitters with good efficiency and durability.

Said object has been solved by compounds of the formula 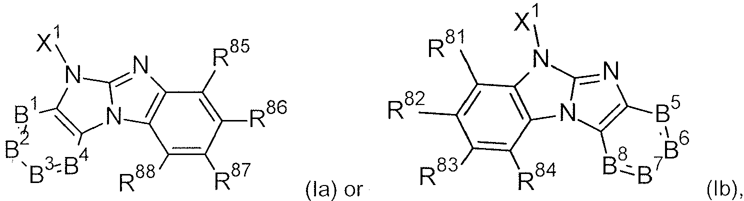 wherein
B¹ is N, or CR⁸¹,
B² is N, or CR⁸²,
B³ is N, or CR⁸³,
B⁴ is N, or CR⁸⁴,
B⁵ is N, or CR⁸⁵,
B⁶ is N, or CR⁸⁶,
B⁷ is N, or CR⁸⁷,
B⁸ is N, or CR⁸⁸,
R⁸¹, R⁸², R⁸³, R⁸⁴, R⁸⁵, R⁸⁶, R⁸⁷ and R⁸⁸ are H;
X¹ is a group of formula -(A¹)ₒ-(A²)ₚ-(A³)_{q}-(A⁴),-R¹⁶,
o is 0, or 1, p is 0, or 1, q is 0, or 1, r is 0, or 1,
R¹⁶ is H, -NR¹⁰R¹¹, or -Si(R¹²)(R¹³)(R¹⁴), a C₆-C₂₄aryl group, which can optionally be substituted by G; or a C₂-C₃₀heteroaryl group, which can optionally be substituted by G;
R¹⁰ and R¹¹ are independently of each other a C₆-C₂₄aryl group, which can optionally be substituted by G; or a C₂-C₃₀heteroaryl group, which can optionally be substituted by G;
R¹², R¹³ and R¹⁴ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G;
A¹, A², A³ and A⁴ are independently of each other a C₆-C₂₄arylen group, which can optionally be substituted by G, or a C₂-C₃₀heteroarylen group, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
E is -OR⁶⁹, -SR69, -NR⁶⁵R⁶⁶, -COR⁶⁸, -COOR⁶⁷, -CONR⁶⁵R⁶⁶, -CN, or F,
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R¹⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl, with the proviso that
one of the substituents B¹, B², B³, B⁴ in formula (Ia) and one of the substituents B⁵, B⁶, B⁷ and B⁸ in formula (Ib) represents N;
and R¹⁶ is different from H, if o is 0, p is 0, q is 0 and r is 0.

The predicted electron affinities of the azabenzimidazo[2,1-a]benzimidazole derivatives of the present invention is higher than the electron affinity of corresponding 6H-benzimidazolo[1,2-a]benzimidazole derivatives. Consequently, the azabenzimidazolo[1,2-a] derivatives of the present envention may have excellent electron injection and hole blocking properties respectively.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices, such as, for example, organic light-emitting diodes (OLEDs).

Accordingly, a further subject of the present invention is directed to an electronic device, comprising a compound according to the present invention. The electronic device is preferably an electroluminescent device.

The compounds of formulae (Ia) and (Ib) can in principal be used in any layer of an EL device, but are preferably used as host, charge transport and/or charge/exciton blocking material. Particularly, the compounds of formulae (Ia) and (Ib) are used as host material for green, especially blue light emitting phosphorescent emitters.

Hence, a further subject of the present invention is directed to a charge transport layer, comprising a compound of formula (Ia) or (Ib) according to the present invention.

A further subject of the present invention is directed to an emitting layer, comprising a compound of formula (Ia) or (Ib) according to the present invention. In said embodiment a compound of formula (Ia) or (Ib) is preferably used as host material in combination with a phosphorescent emitter.

A further subject of the present invention is directed to a charge/exciton blocking layer, comprising a compound of formula (Ia) or (Ib) according to the present invention.

D is preferably -CO-, -COO-, -S-, -SO-, -SO₂-, -O-, -NR⁶⁵-, wherein R⁶⁵ is C₁-C₁₈alkyl, such as methyl, ethyl, n-propyl, iso-propyl, n-butyl, isobutyl, or sec-butyl, or C₆-C₁₄aryl, such as phenyl, tolyl, naphthyl, or biphenylyl, or C₂-C₃₀heteroaryl, such as, for example, benzimidazo[1,2-a]benzimidazo-2-yl 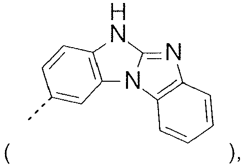 carbazolyl, dibenzofuranyl, which can be unsubstituted or substituted especially by C₆-C₁₀aryl, or C₆-C₁₀aryl, which is substituted by C₁-C₄alkyl; or C₂-C₁₀heteroaryl.

E is preferably -OR⁶⁹; -SR⁶⁹; -NR⁶⁵R⁶⁵; -COR⁶⁸; -COOR⁶⁷; -CONR⁶⁵R⁶⁵; or -CN; wherein R⁶⁵, R⁶⁷, R⁶⁸ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl.

G is preferably -OR⁶⁹; -SR⁶⁹; -NR⁶⁵R⁶⁵; a C₁-C₁₈alkyl group, a C₆-C₁₄aryl group, a C₆-C₁₄aryl group, which is substituted by F, or C₁-C₁₈alkyl; a C₂-C₁₀heteroaryl group, or a C₂-C₁₀heteroaryl group, which is substituted by F, or C₁-C₁₈alkyl; or -Si(R^{12'})(R^{13'})(R^{14'}); wherein R⁶⁵, R⁶⁷, R⁶⁸ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl; R^{12'}, R^{13'} and R^{14'} are independently of each other; a C₆-C₁₄aryl group, which can optionally be substituted by C₁-C₁₈alkyl ; or a C₂-C₁₀heteroaryl group, which can optionally be substituted by C₁-C₁₈alkyl.

A C₂-C₁₀heteroaryl group is for example, benzimidazo[1,2-a]benzimidazo-5-yl 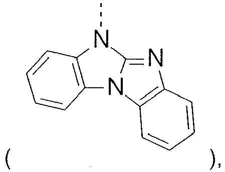 benzimidazo[1,2-a]benzimidazo-2-yl 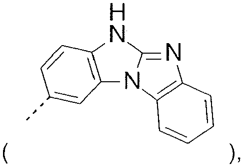 benzimidazolo[2,1-b][1,3]benzothiazolyl, carbazolyl, dibenzofuranyl, which can be unsubstituted or substituted, especially by C₆-C₁₀aryl, or C₆-C₁₀aryl, which is substituted by C₁-C₄alkyl; or C₂-C₁₀heteroaryl.

In a more preferred embodiment the present invention is directed to compounds of formula 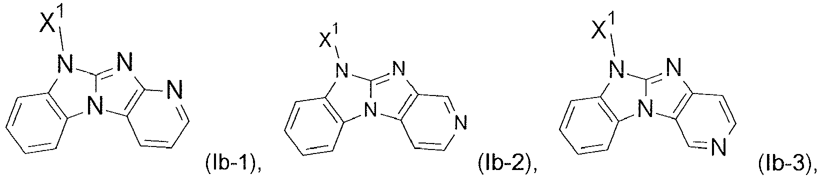 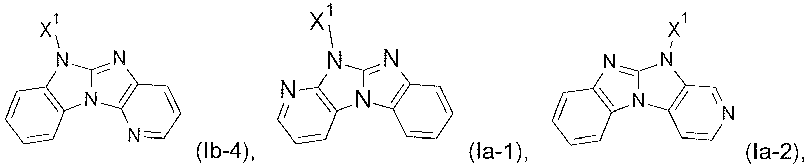 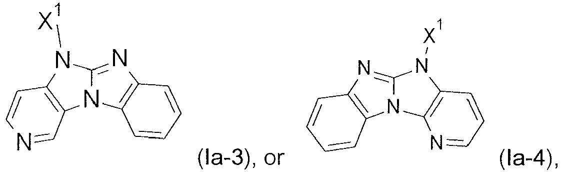 wherein X¹ is defined above.

Compounds of the formula (Ib-2), (Ib-4), (Ia-2) and (Ia-4) are preferred.

X¹ is a group of formula --(A¹)ₒ-(A²)ₚ-(A³)_{q}-(A⁴)ᵣ-R¹⁶.

For the group of formula --(A¹)ₒ-(A²)ₚ-(A³)_{q}-(A⁴)ᵣ₋R¹⁶ the following preferences apply.

A¹, A², A³ and A⁴ are independently of each other a C₆-C₂₄arylen group, which can optionally be substituted by G, or a C₂-C₃₀heteroarylen group, which can optionally be substituted by G.

The C₆-C₂₄arylen groups A¹, A², A³ and A⁴ which optionally can be substituted by G, are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted. The C₂-C₃₀heteroarylen groups A¹, A², A³ and A⁴, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated-electrons such as, for example, benzofuro[2,3-b]pyridylene 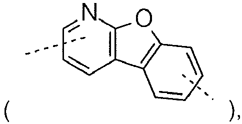 benzothiopheno[2,3-b]pyridylene 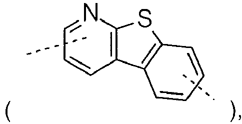 pyrido[2,3-b]indolylene 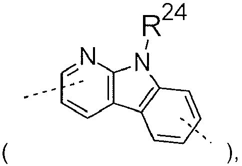 benzofuro[2,3-c]pyridylene 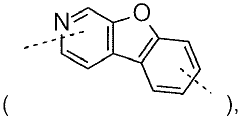 benzothiopheno[2,3-c]pyridylene 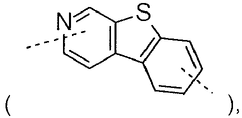 pyrido[2,3-c]indolylene 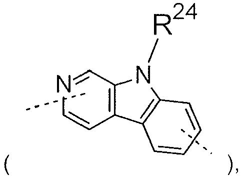 furo[3,2-b:4,5-b']dipyridylene 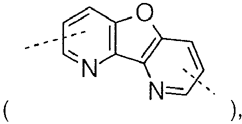 thieno[3,2-b:4,5-b']dipyridylene 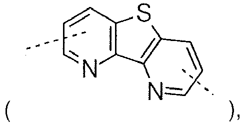 pyrrolo[3,2-b:4,5-b']dipyridylene 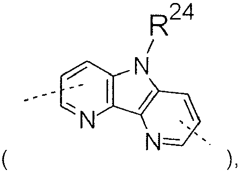 thienylene, benzothiophenylene, thianthrenylene, furylene, furfurylene, 2H-pyranylene, benzofuranylene, isobenzofuranylene, dibenzofuranylene 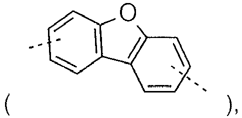 dibenzothiophenylene 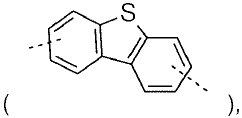 phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene 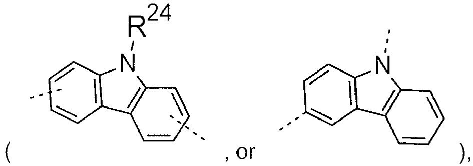 benzimidazo[1,2-a]benzimidazo-2,5-ylene 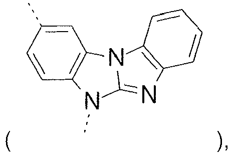 or phenoxazinylene, which can be unsubstituted or substituted.

Preferred C₆-C₂₄arylen groups are 1,3-phenylene, 3,3'-biphenylylene, 3,3'-m-terphenylene, 2- or 9-fluorenylene, phenanthrylene, which may be unsubstituted or substituted, especially by C₆-C₁₀aryl, C₆-C₁₀aryl which is substituted by C₁-C₄alkyl; or C₂-C₁₄heteroaryl.

Preferred C₂-C₃₀heteroarylen groups are pyridylene, triazinylene, pyrimidinylene, especially benzofuro[2,3-b]pyridylene, benzothiopheno[2,3-b]pyridylene, pyrido[2,3-b]indolylene, benzofuro[2,3-c]pyridylene, benzothiopheno[2,3-c]pyridylene, pyrido[2,3-c]indolylene furo[3,2-b:4,5-b']dipyridylene, thieno[3,2-b:4,5-b']dipyridylene, pyrrolo[3,2-b:4,5-b']dipyridylene, dibenzofuranylene, dibenzothiophenylene, carbazolylene and benzimidazo[1,2-a]benzimidazo-2,5-ylene, which can be unsubstituted or substituted, especially by C₆-C₁₄aryl, C₆-C₁₄aryl which is substituted by C₁-C₄alkyl; or C₂-C₁₀heteroaryl.

The C₆-C₂₄arylen and C₂-C₃₀heteroarylen groups may be substituted by G.

G is preferably C₁-C₁₈alkyl, such as methyl, ethyl, n-propyl, iso-propyl, n-butyl, isobutyl, sec-butyl, hexyl, octyl, or 2-ethyl-hexyl, -CF₃, a C₆-C₁₄aryl group, a C₆-C₁₄aryl group, which is substituted by F, or C₁-C₁₈alkyl; a C₂-C₁₀heteroaryl group, or a C₂-C₁₀heteroaryl group, which is substituted by F, or C₁-C₁₈alkyl.

Benzimidazo[1,2-a]benzimidazo-5-yl, benzimidazo[1,2-a]benzimidazo-2-yl, carbazolyl and dibenzofuranyl are examples of a C₂-C₁₀heteroaryl group. Phenyl, 1-naphthyl and 2-naphthyl are examples of a C₆-C₁₄aryl group.

Preferably, A¹, A², A³ and A⁴ are independently of each other a group of the formula A¹, A², A³ and A⁴ are independently of each other a group of formula 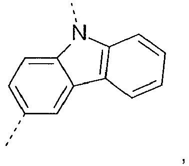 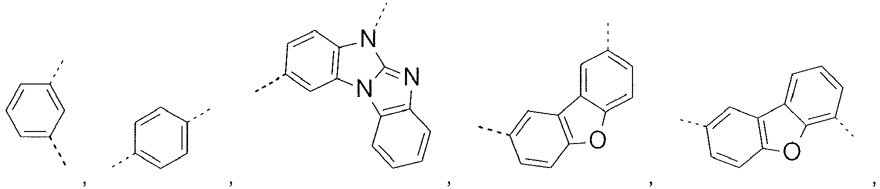 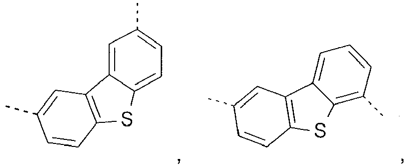 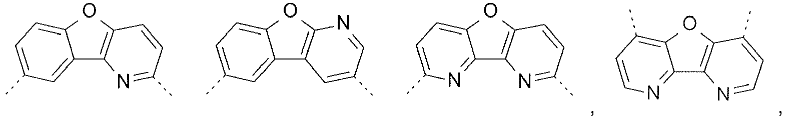 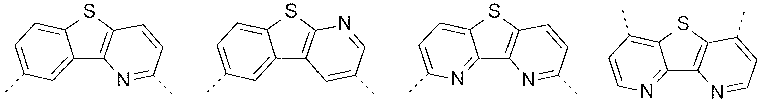 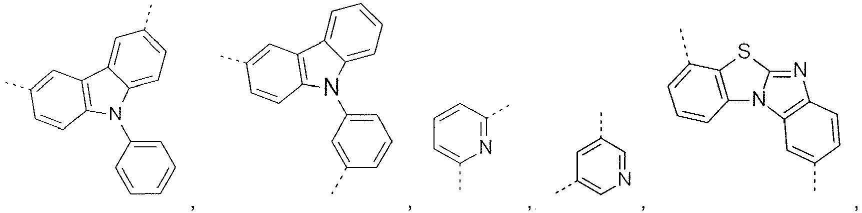 or

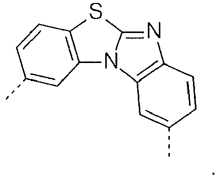

R¹⁶ may be a C₆-C₂₄aryl group, which can optionally be substituted by G, or a C₂-C₃₀heteroaryl group, which can optionally be substituted by G.

The C₆-C₂₄aryl group, which optionally can be substituted by G, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, or triphenylenyl (especially triphenylen-2-yl), which may be unsubstituted or substituted.

The C₂-C₃₀heteroaryl group R¹⁶, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated π-electrons such as 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, benzimidazo[1,2-a]benzimidazo-5-yl, benzimidazo[1,2-a]benzimidazo-2-yl, benzimidazolo[2,1-b][1,3]benzothiazolyl, carbazolyl, 9-phenylcarbazolyl, azabenzimidazo[1,2-a]benzimidazolyl, or phenoxazinyl, which can be unsubstituted or substituted.

The C₆-C₂₄aryl and C₂-C₃₀heteroaryl groups may be substituted by G.

G is preferably C₁-C₁₈alkyl, such as methyl, ethyl, n-propyl, iso-propyl, n-butyl, isobutyl, sec-butyl, hexyl, octyl, or 2-ethyl-hexyl; -CF₃, a C₆-C₁₄aryl group, a C₆-C₁₄aryl group, which is substituted by F, or C₁-C₁₈alkyl; a C₂-C₁₀heteroaryl group, or a C₂-C₁₀heteroaryl group, which is substituted by F, or C₁-C₁₈alkyl.

Prefered C₂-C₃₀heteroaryl groups are pyridyl, triazinyl, pyrimidinyl, especially 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, benzimidazo[1,2-a]benzimidazo-5-yl 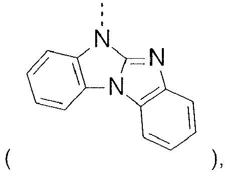 benzimidazo[1,2-a]benzimidazo-2-yl 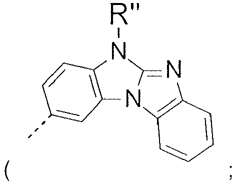 R" is C₆-C₁₀aryl, or C₆-C₁₀aryl, which is substituted by C₁-C₄alkyl; or C₂-C₁₄heteroaryl), benzimidazolo[2,1-b][1,3]benzothiazolyl 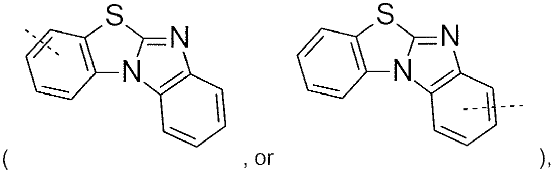 carbazolyl, dibenzofuranyl, dibenzothiophenyl, 4-azabenzimidazo[1,2-a]benzimidazo-6-yl 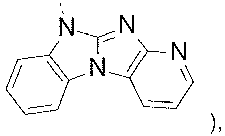 3-azabenzimidazo[1,2-a]benzimidazo-6-yl 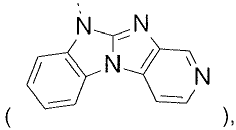 2-azabenzimidazo[1,2-a]benzimidazo-6-yl 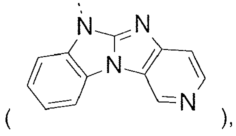 1-azabenzimidazo[1,2-a]benzimidazo-6-yl 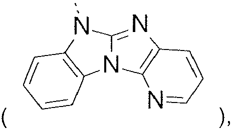 4-azabenzimidazo[1,2-a]benzimidazo-5-yl 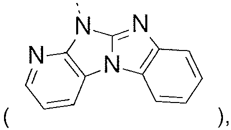 3-azabenzimidazo[1,2-a]benzimidazo-5-yl 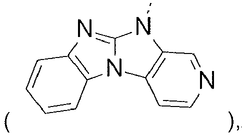 2-azabenzimidazo[1,2-a]benzimidazo-5-yl 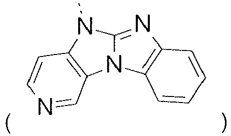 and 1-azabenzimidazo[1,2-a]benzimidazo-5-yl 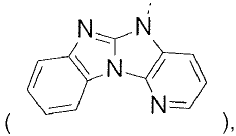 which can be unsubstituted or substituted especially by C₆-C₁₀aryl, or C₆-C₁₀aryl, which is substituted by C₁-C₄alkyl; or C₂-C₁₄heteroaryl.

Most preferred, R¹⁶ is a group of formula

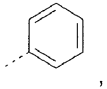

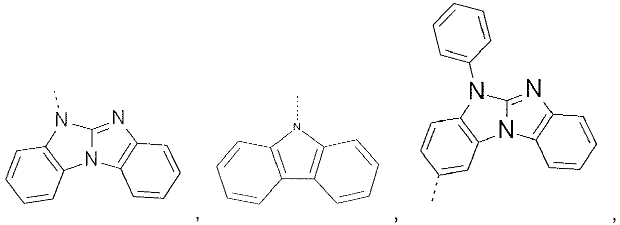

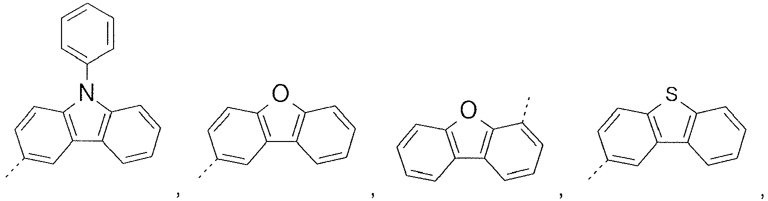

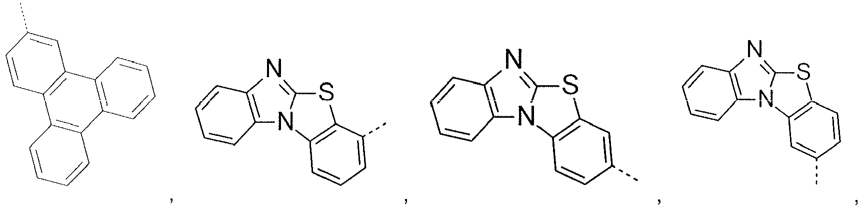

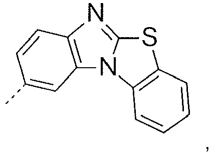

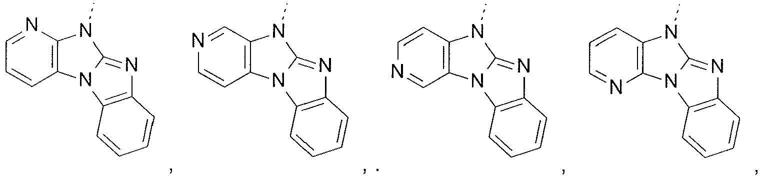

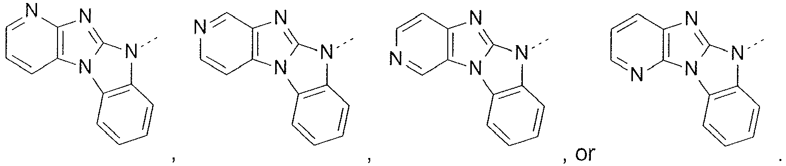

In a particularly preferred embodiment of the present invention X¹ is a group of formula-(A¹)ₒ-(A²)ₚ-(A³)_{q}-(A⁴)ᵣ-R¹⁶, o is 0, or 1, p is 0, or 1, q is 0, or 1, r is 0, or 1,

A¹, A², A³ and A⁴ are independently of each other a group of formula 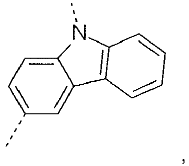 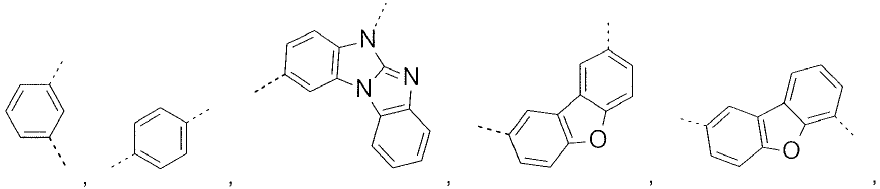 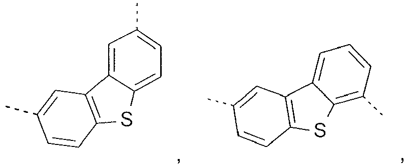 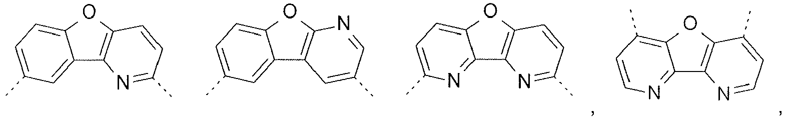 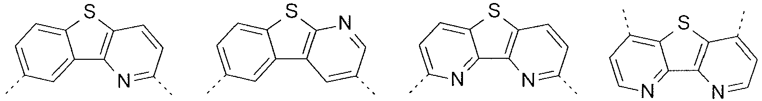 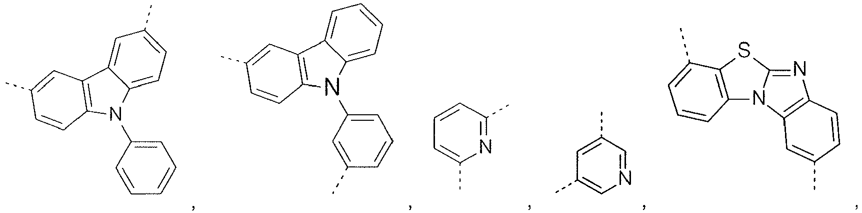 or 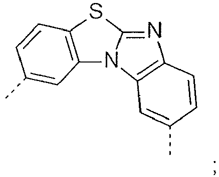 and R¹⁶ is a group of formula

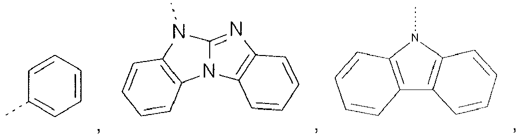

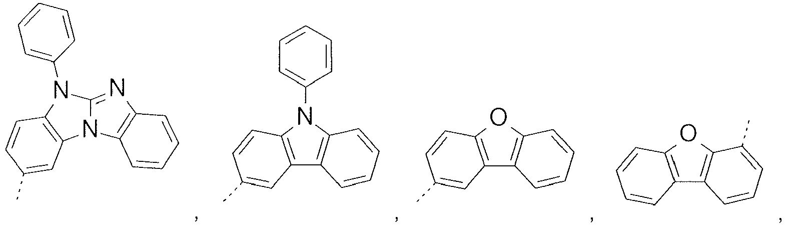

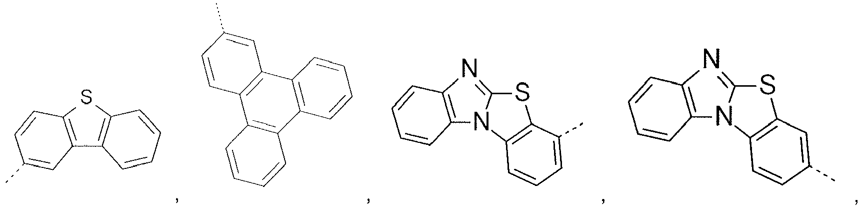

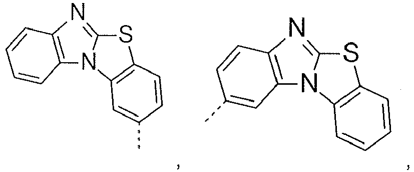

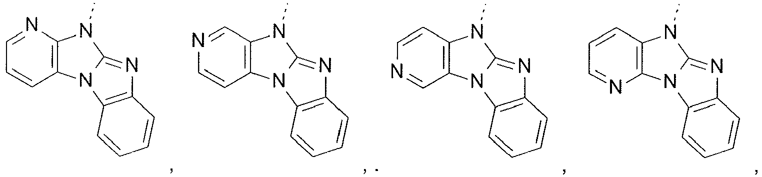

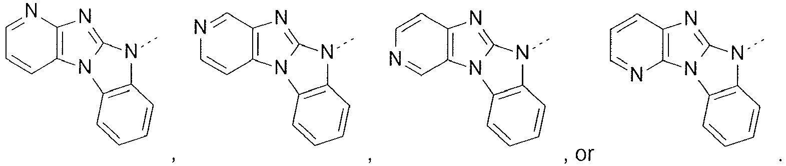

Most preferred, X¹ is a group of formula --(A¹)ₒ-(A²)ₚ-(A³)_{q}-(A⁴)ᵣ-R¹⁶, wherein --(A¹)ₒ-(A²)ₚ-(A³)_{q}-(A⁴)ᵣ- is a group of formula 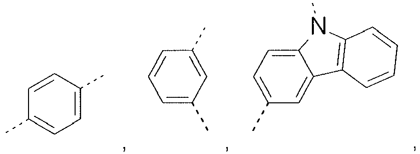 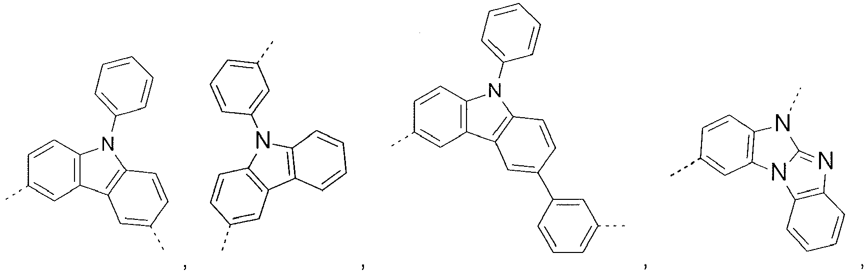 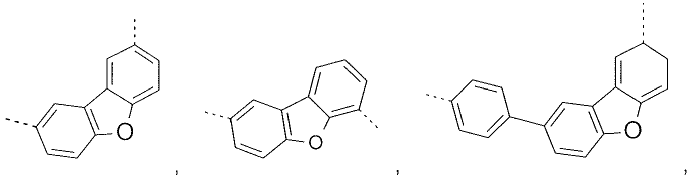 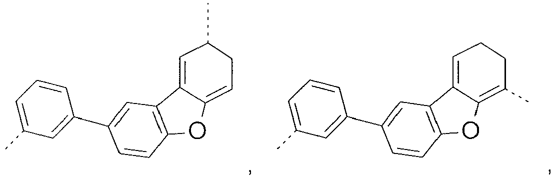 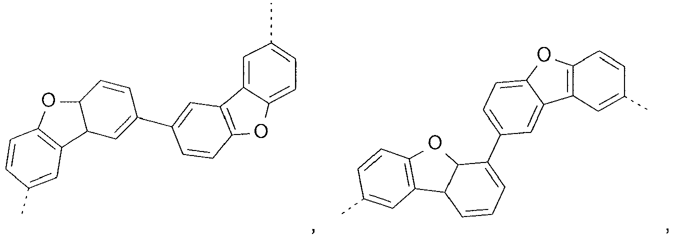 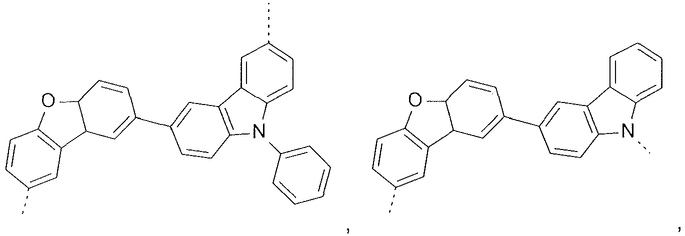 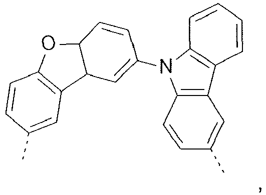 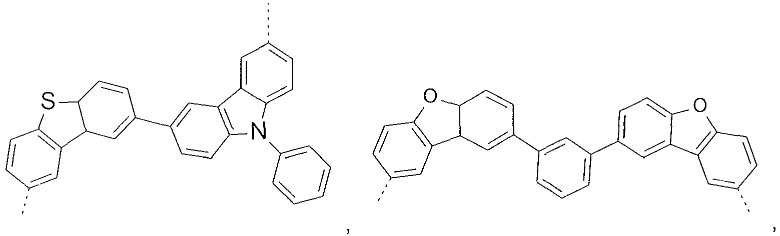 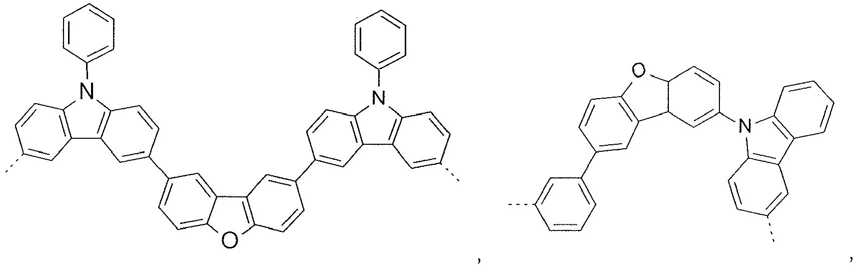 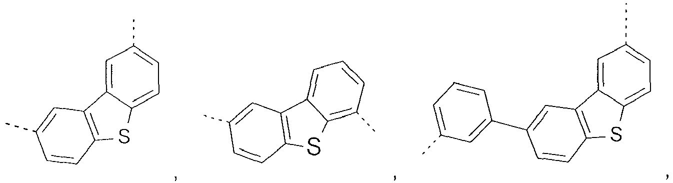 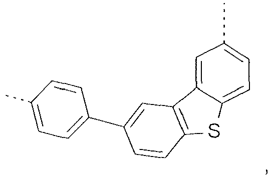 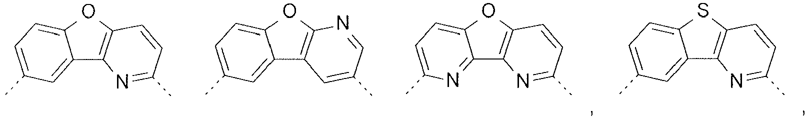 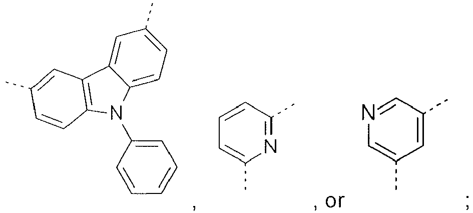 and R¹⁶ is a group of formula 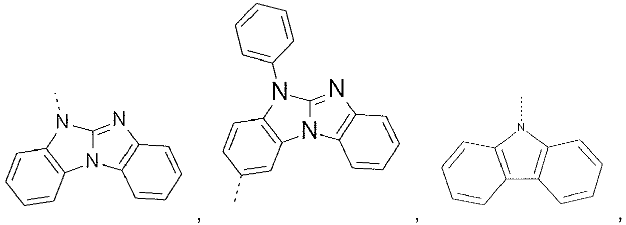 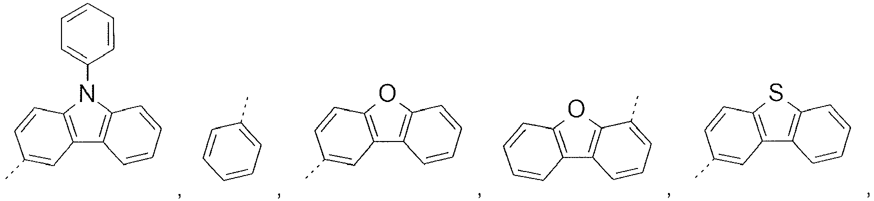 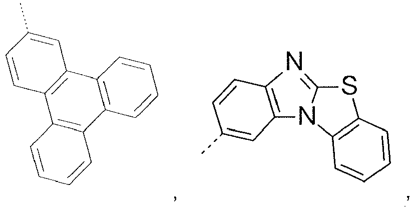 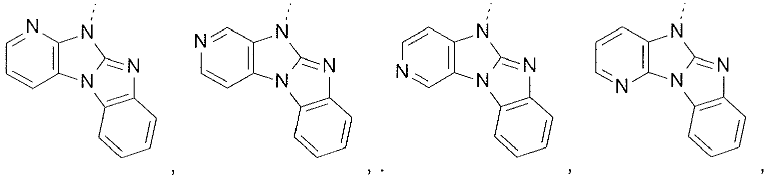 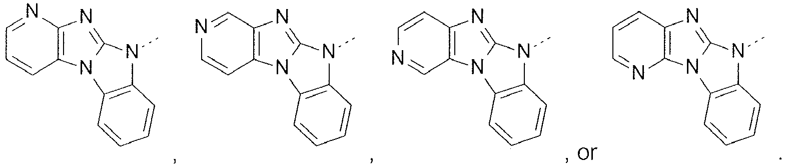 Compounds of the formula 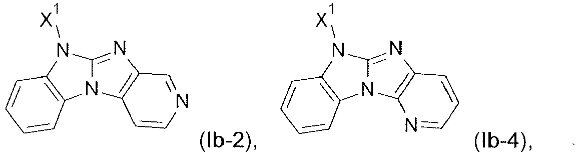 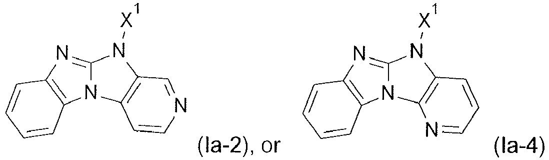 are most preferred, wherein X¹ is a group of formula --(A¹)ₒ-(A²)ₚ₋(A³)_{q}-(A⁴)ᵣ₋R¹⁶,
o is 0, or 1, p is 0, or 1, q is 0, or 1, r is 0, or 1,
A¹, A², A³ and A⁴ are independently of each other a group of formula 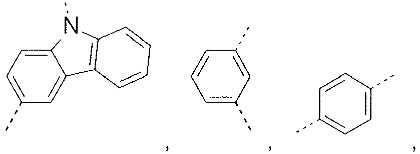 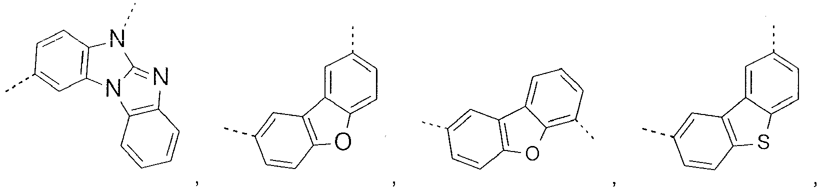 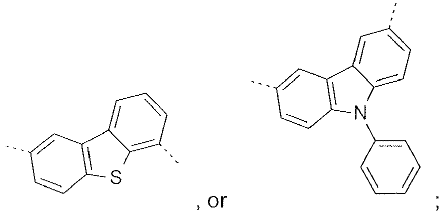 and R¹⁶ is a group of formula

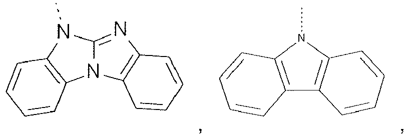

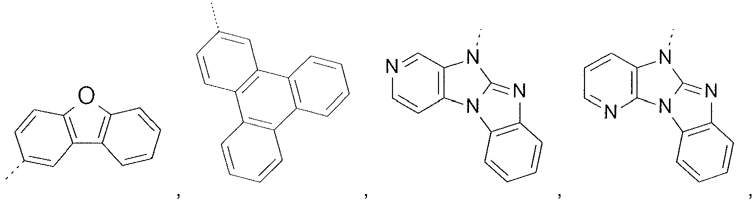

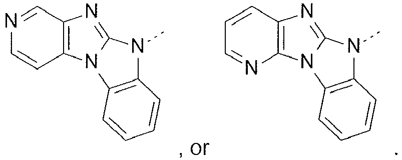

Examples of compounds of formulae (Ia) and (Ib) are shown in the table below. 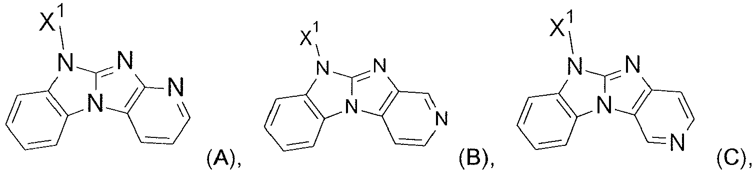 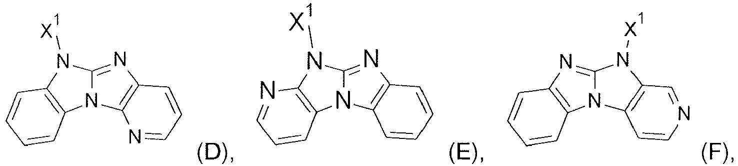 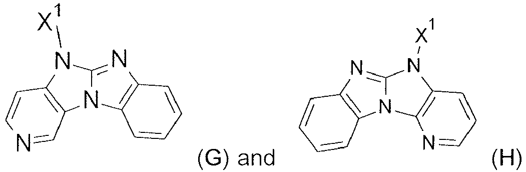 (X¹ is a group of formula --(A¹)ₒ-(A²)ₚ-(A3)_{q}-(A4)ᵣ₋R¹⁶):

| Compound | -(A¹)ₒ-(A²)ₚ-(A³)_{q}-(A⁴)ᵣ- | R¹⁶ |
|---|---|---|
| A-1, B-1, C-1, D-1, E-1, F-1, G-1, H-1 | 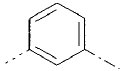 | 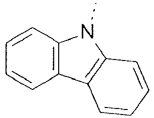 |
| A-2, B-2, C-2, D-2, E-2, F-2, G-2, H-2 | 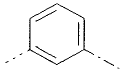 | 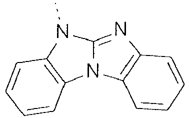 |
| A-3, B-3, C-3, D-3, E-3, F-3, G-3, H-3 | 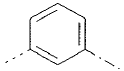 | 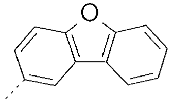 |
| A-4, B-4, C-4, D-4, E-4, F-4, G-4, H-4 | 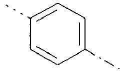 | 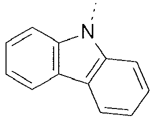 |
| A-5, B-5, C-5, D-5, E-5, F-5, G-5, H-5 | 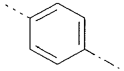 | 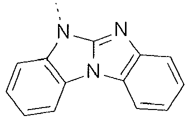 |
| A-6, B-6, C-6, D-6, E-6, F-6, G-6, H-6 | 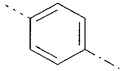 | 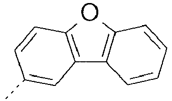 |
| A-7, B-7, C-7, D-7, E-7, F-7, G-7, H-7 | 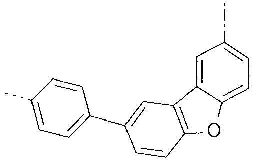 | 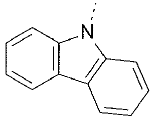 |
| A-8, B-8, C-8, D-8, E-8, F-8, G-8, H-8 | 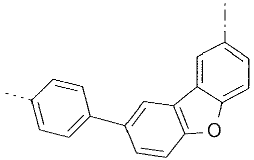 | 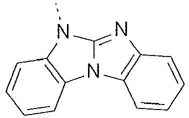 |
| A-9, B-9, C-9, D-9, E-9, F-9, G-9, H-9 | 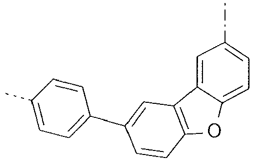 | 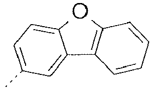 |
| A-10, B-10, C-10, D-10, E-10, F-10, G-10, H-10 | 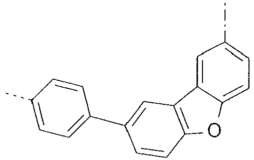 | 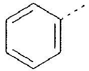 |
| A-11, B-11, C-11, D-11, E-11, F-11, G-11, H-11 | 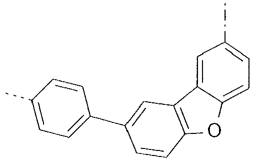 | 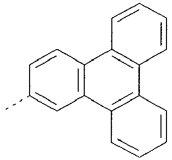 |
| A-12, B-12, C-12, D-12, E-12, F-12, G-12, H-12 | 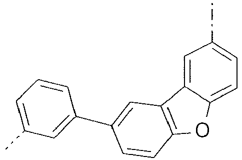 | 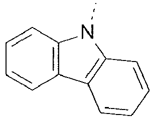 |
| A-13, B-13, C-13, D-13, E-13, F-13, G-13, H-13 | 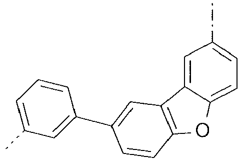 | 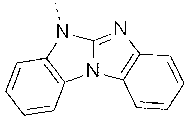 |
| A-14, B-14, C-14, D-14, E-14, F-14, G-14, H-14 | 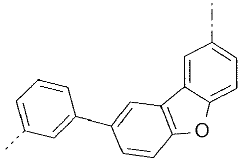 | 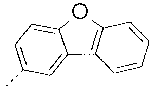 |
| A-15, B-15, C-15, D-15, E-15, F-15, G-15, H-15 | 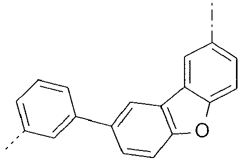 | 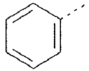 |
| A-16, B-16, C-16, D-16, E-16, F-16, G-16, H-16 | 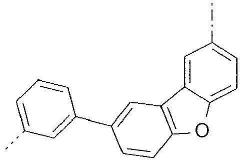 | 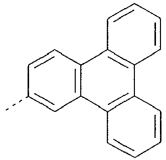 |
| A-17, B-17, C-17, D-17, E-17, F-17, G-17, H-17 | 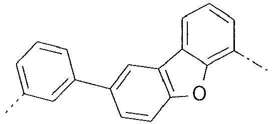 | 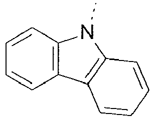 |
| A-18, B-18, C-18, D-18, E-18, F-18, G-18, H-18 | 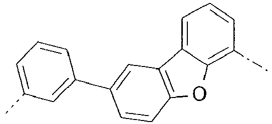 | 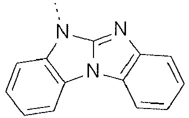 |
| A-19, B-19, C-19, D-19, E-19, F-19, G-19, H-19 | 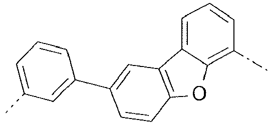 | 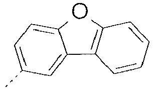 |
| A-20, B-20, C-20, D-20, E-20, F-20, G-20, H-20 | 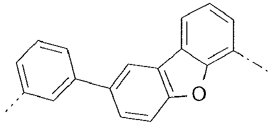 | 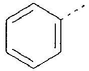 |
| A-21, B-21, C-21, D-21, E-21, F-21, G-21, H-21 | 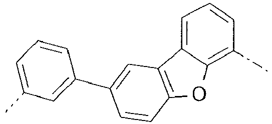 | 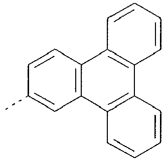 |
| A-22, B-22, C-22, D-22, E-22, F-22, G-22, H-22 | 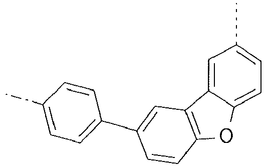 | 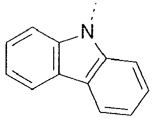 |
| A-23, B-23, C-23, D-23, E-23, F-23, G-23, H-23 | 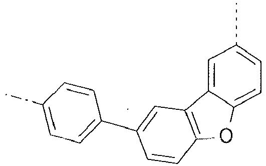 | 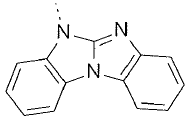 |
| A-24, B-24, C-24, D-24, E-24, F-24, G-24, H-24 | 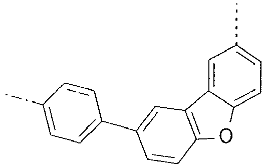 | 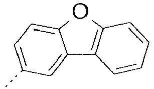 |
| A-25, B-25, C-25, D-25, E-25, F-25, G-25, H-25 | 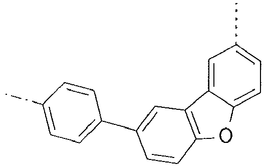 | 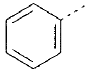 |
| A-26, B-26, C-26, D-26, E-26, F-26, G-26, H-26 | 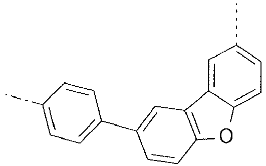 | 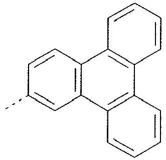 |
| A-27, B-27, C-27, D-27, E-27, F-27, G-27, H-27 | 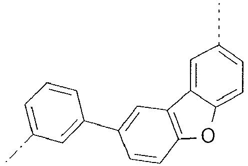 | 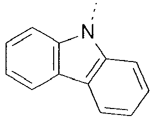 |
| A-28, B-28, C-28, D-28, E-28, F-28, G-28, H-28 | 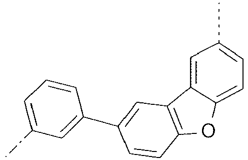 | 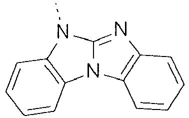 |
| A-29, B-29, C-29, D-29, E-29, F-29, G-29, H-29 | 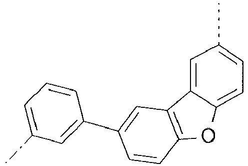 | 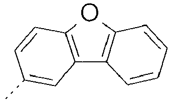 |
| A-30, B-30, C-30, D-30, E-30, F-30, G-30, H-30 | 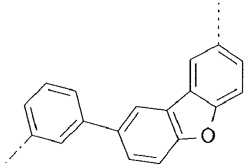 | 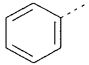 |
| A-31, B-31, C-31, D-31, E-31, F-31, G-31, H-31 | 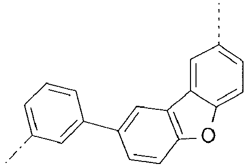 | 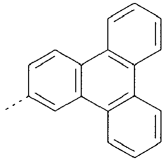 |
| A-32, B-32, C-32, D-32, E-32, F-32, G-32, H-32 | 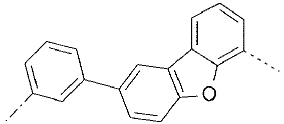 | 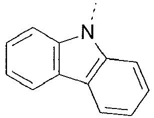 |
| A-33, B-33, C-33, D-33, E-33, F-33, G-33, H-33 | 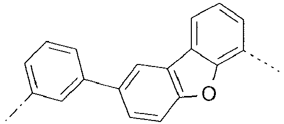 | 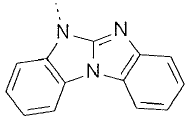 |
| A-34, B-34, C-34, D-34, E-34, F-34, G-34, H-34 | 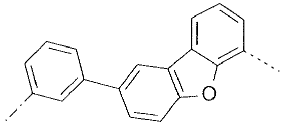 | 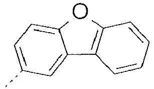 |
| A-35, B-35, C-35, D-35, E-35, F-35, G-35, H-35 | 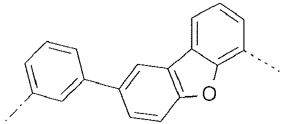 | 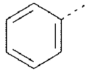 |
| A-36, B-36, C-36, D-36, E-36, F-36, G-36, H-36 | 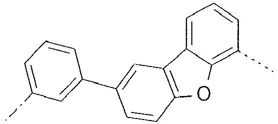 | 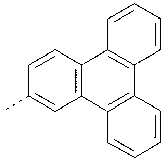 |
| A-37, B-37, C-37, D-37, E-37, F-37, G-37, H-37 | 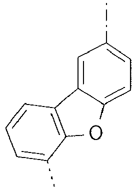 | 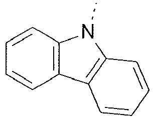 |
| A-38, B-38, C-38, D-38, E-38, F-38, G-38, H-38 | 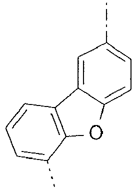 | 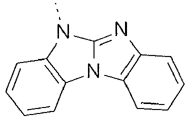 |
| A-39, B-39, C-39, D-39, E-39, F-39, G-39, H-39 | 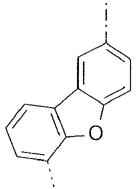 | 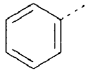 |
| A-40, B-40, C-40, D-40, E-40, F-40, G-40, H-40 | 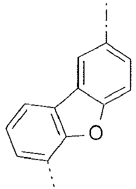 | 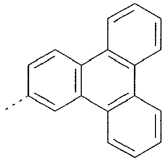 |
| A-41, B-41, C-41, D-41, E-41, F-41, G-41, H-41 | 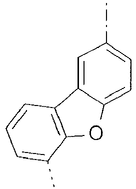 | 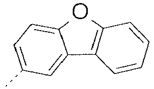 |
| A-42, B-42, C-42, D-42, E-42, F-42, G-42, H-42 | 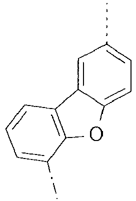 | 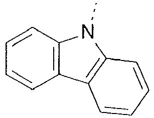 |
| A-43, B-43, C-43, D-43, E-43, F-43, G-43, H-43 | 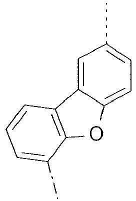 | 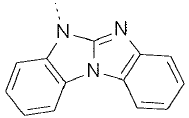 |
| A-44, B-44, C-44, D-44, E-44, F-44, G-44, H-44 | 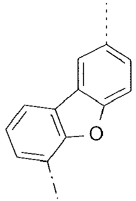 | 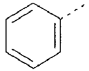 |
| A-45, B-45, C-45, D-45, E-45, F-45, G-45, H-45 | 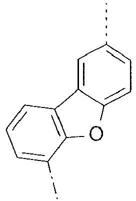 | 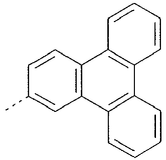 |
| A-46, B-46, C-46, D-46, E-46, F-46, G-46, H-46 | 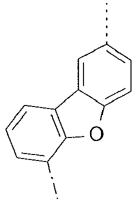 | 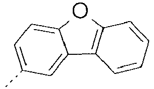 |
| A-47, B-47, C-47, D-47, E-47, F-47, G-47, H-47 | 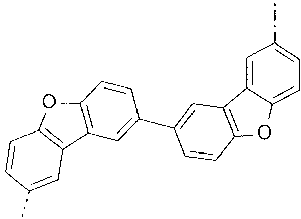 | 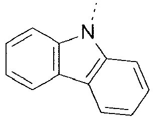 |
| A-48, B-48, C-48, D-48, E-48, F-48, G-48, H-48 | 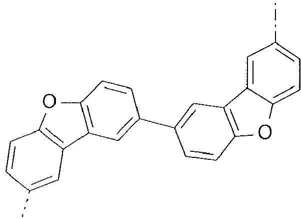 | 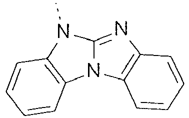 |
| A-49, B-49, C-49, D-49, E-49, F-49, G-49, H-49 | 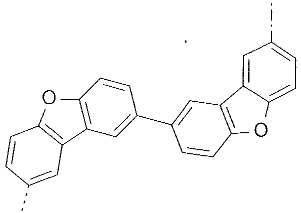 | 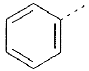 |
| A-50, B-50, C-50, D-50, E-50, F-50, G-50, H-50 | 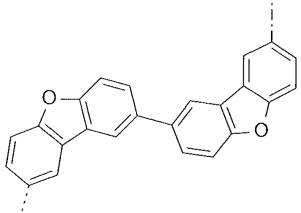 | 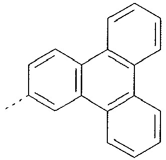 |
| A-51, B-51, C-51, D-51, E-51, F-51, G-51, H-51 | 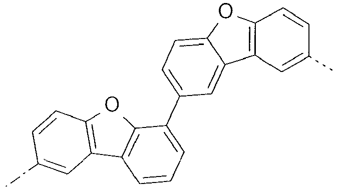 | 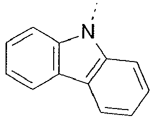 |
| A-52, B-52, C-52, D-52, E-52, F-52, G-52, H-52 | 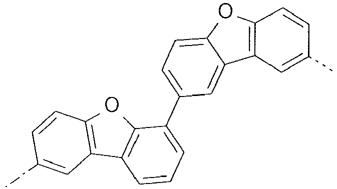 | 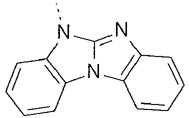 |
| A-53, B-53, C-53, D-53, E-53, F-53, G-53, H-53 | 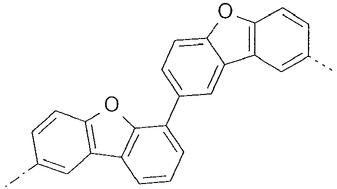 | 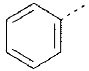 |
| A-54, B-54, C-54, D-54, E-54, F-54, G-54, H-54 | 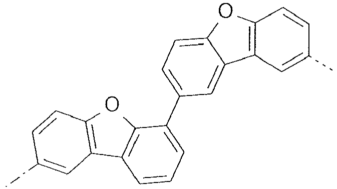 | 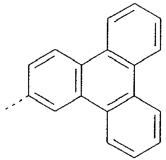 |
| A-55, B-55, C-55, D-55, E-55, F-55, G-55, H-55 | 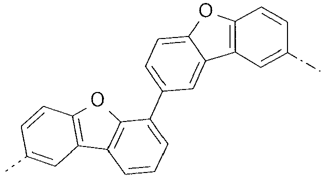 | 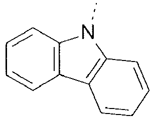 |
| A-56, B-56, C-56, D-56, E-56, F-56, G-56, H-56 | 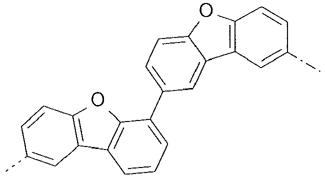 | 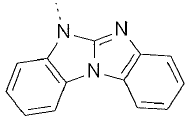 |
| A-57, B-57, C-57, D-57, E-57, F-57, G-57, H-57 | 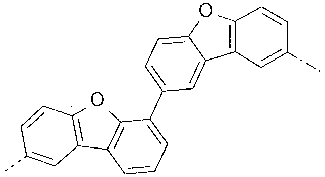 | 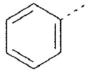 |
| A-58, B-58, C-58, D-58, E-58, F-58, G-58, H-58 | 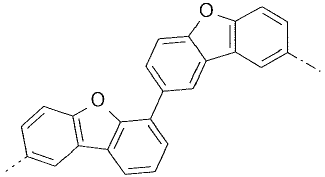 | 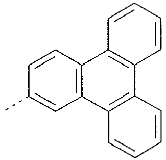 |
| A-59, B-59, C-59, D-59, E-59, F-59, G-59, H-59 | 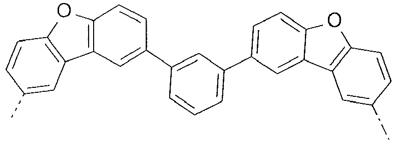 | 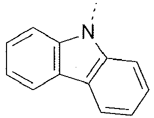 |
| A-60, B-60, C-60, D-60, E-60, F-60, G-60, H-60 | 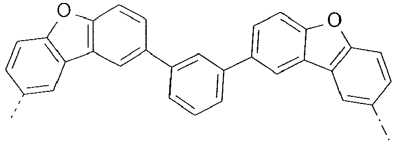 | 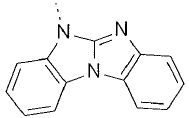 |
| A-61, B-61, C-61, D-61, E-61, F-61, G-61, H-61 | 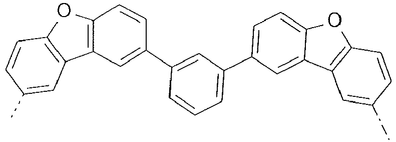 | 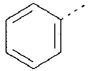 |
| A-62, B-62, C-62, D-62, E-62, F-62, G-62, H-62 | 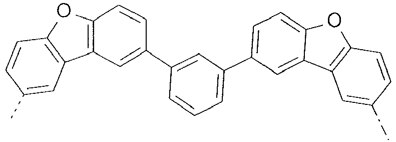 | 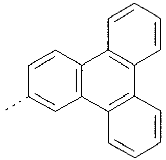 |
| A-63, B-63, C-63, D-63, E-63, F-63, G-63, H-63 | 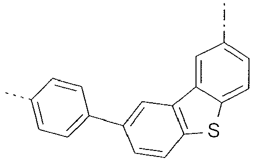 | 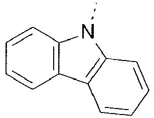 |
| A-64, B-64, C-64, D-64, E-64, F-64, G-64, H-64 | 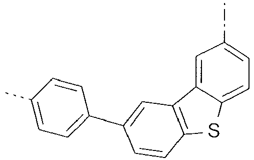 | 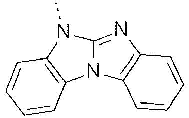 |
| A-65, B-65, C-65, D-65, E-65, F-65, G-65, H-65 | 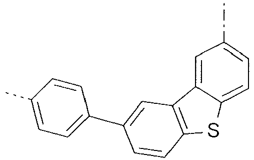 | 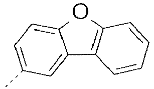 |
| A-66, B-66, C-66, D-66, E-66, F-66, G-66, H-66 | 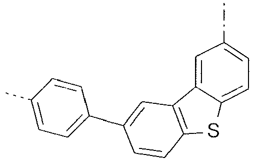 | 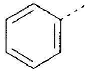 |
| A-67, B-67, C-67, D-67, E-67, F-67, G-67, H-67 | 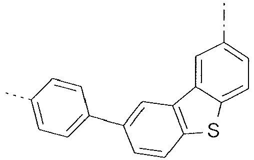 | 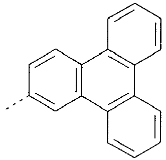 |
| A-68, B-68, C-68, D-68, E-68, F-68, G-68, H-68 | 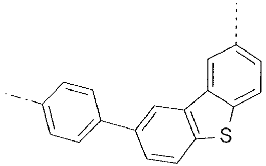 | 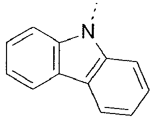 |
| A-69, B-69, C-69, D-69, E-69, F-69, G-69, H-69 | 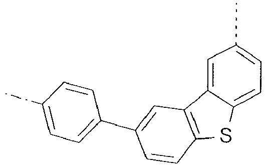 | 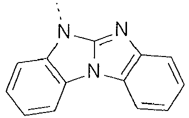 |
| A-70, B-70, C-70, D-70, E-70, F-70, G-70, H-70 | 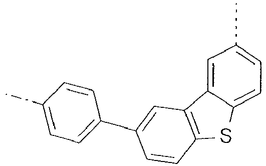 | 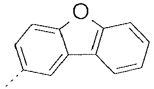 |
| A-71, B-71, C-71, D-71, E-71, F-71, G-71, H-71 | 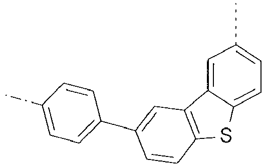 | 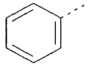 |
| A-72, B-72, C-72, D-72, E-72, F-72, G-72, H-72 | 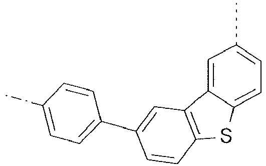 | 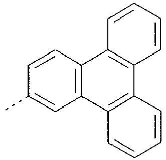 |
| A-73, B-73, C-73, D-73, E-73, F-73, G-73, H-73 | 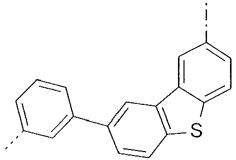 | 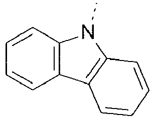 |
| A-74, B-74, C-74, D-74, E-74, F-74, G-74, H-74 | 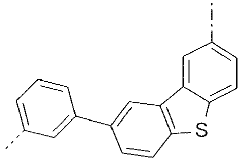 | 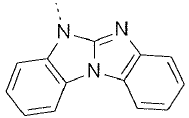 |
| A-75, B-75, C-75, D-75, E-75, F-75, G-75, H-75 | 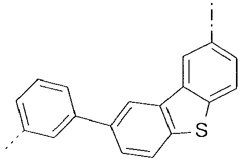 | 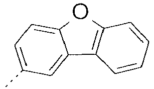 |
| A-76, B-76, C-76, D-76, E-76, F-76, G-76, H-76 | 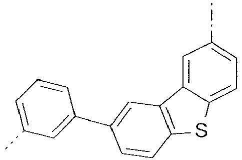 | 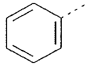 |
| A-77, B-77, C-77, D-77, E-77, F-77, G-77, H-77 | 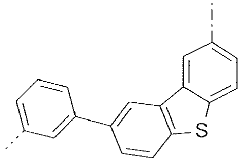 | 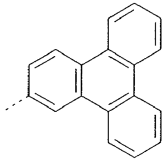 |
| A-78, B-78, C-78, D-78, E-78, F-78, G-78, H-78 | 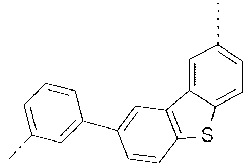 | 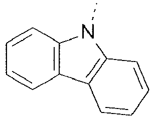 |
| A-79, B-79, C-79, D-79, E-79, F-79, G-79, H-79 | 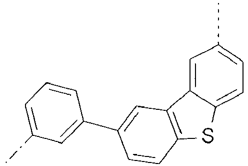 | 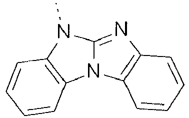 |
| A-80, B-80, C-80, D-80, E-80, F-80, G-80, H-80 | 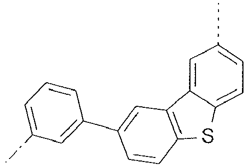 | 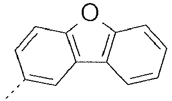 |
| A-81, B-81, C-81, D-81, E-81, F-81, G-81, H-81 | 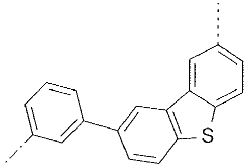 | 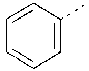 |
| A-82, B-82, C-82, D-82, E-82, F-82, G-82, H-82 | 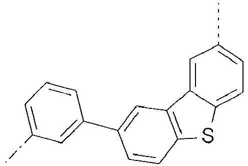 | 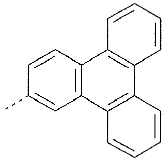 |
| A-83, B-83, C-83, D-83, E-83, F-83, G-83, H-83 | 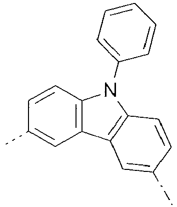 | 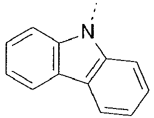 |
| A-84, B-84, C-84, D-84, E-84, F-84, G-84, H-84 | 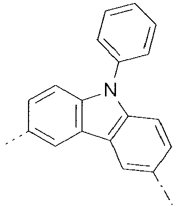 | 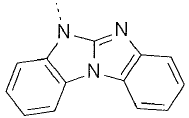 |
| A-85, B-85, C-85, D-85, E-85, F-85, G-85, H-85 | 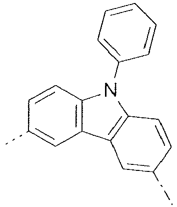 | 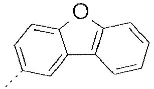 |
| A-86, B-86, C-86, D-86, E-86, F-86, G-86, H-86 | 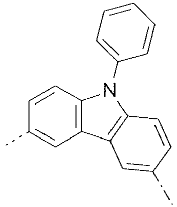 | 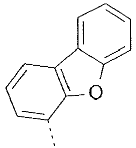 |
| A-87, B-87, C-87, D-87, E-87, F-87, G-87, H-87 | 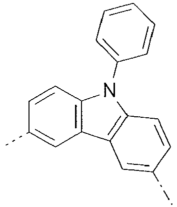 | 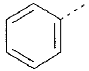 |
| A-88, B-88, C-88, D-88, E-88, F-88, G-88, H-88 | 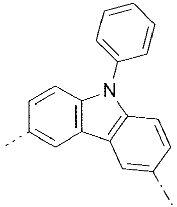 | 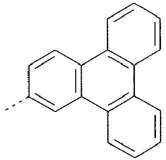 |
| A-89, B-89, C-89, D-89, E-89, F-89, G-89, H-89 | 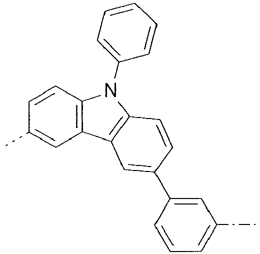 | 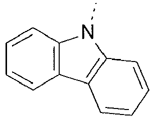 |
| A-90, B-90, C-90, D-90, E-90, F-90, G-90, H-90 | 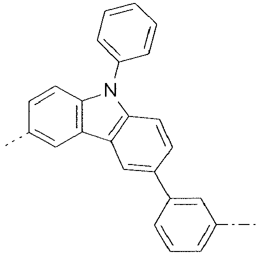 | 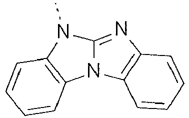 |
| A-91, B-91, C-91, D-91, E-91, F-91, G-91, H-91 | 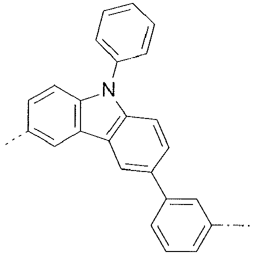 | 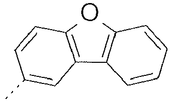 |
| A-92, B-92, C-92, D-92, E-92, F-92, G-92, H-92 | 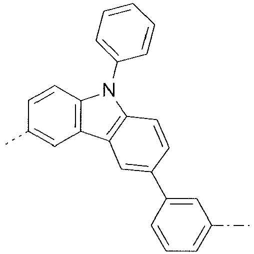 | 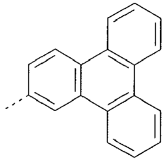 |
| A-93, B-93, C-93, D-93, E-93, F-93, G-93, H-93 | 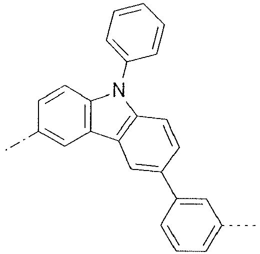 | 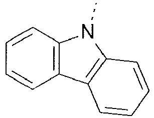 |
| A-94, B-94, C-94, D-94, E-94, F-94, G-94, H-94 | 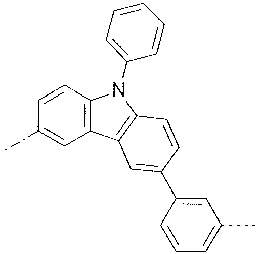 | 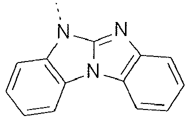 |
| A-95, B-95, C-95, D-95, E-95, F-95, G-95, H-95 | 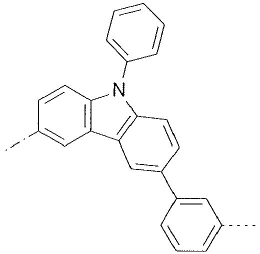 | 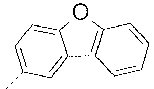 |
| A-96, B-96, C-96, D-96, E-96, F-96, G-96, H-96 | 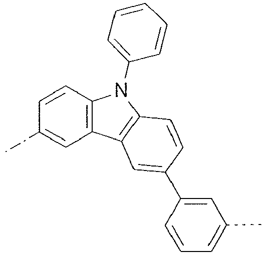 | 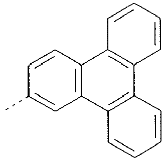 |
| A-97, B-97, C-97, D-97, E-97, F-97, G-97, H-97 | 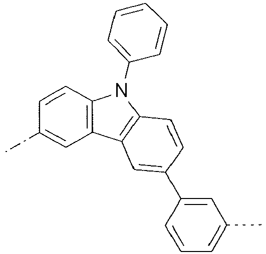 | 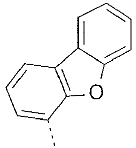 |
| A-98, B-98, C-98, D-98, E-98, F-98, G-98, H-98 | 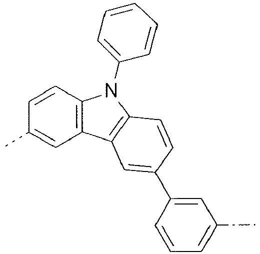 | 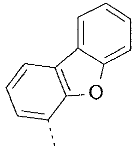 |
| A-99, B-99, C-99, D-99, E-99, F-99, G-99, H-99 | 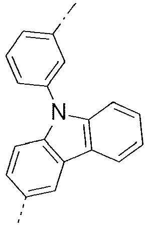 | 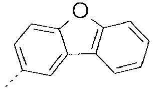 |
| A-100, B-100, C-100, D-100, E-100, F-100, G-100, H-100 | 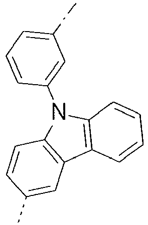 | 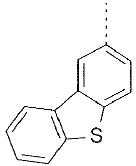 |
| A-101, B-101, C-101, D-101, E-101, F-101, G-101, H-101 | 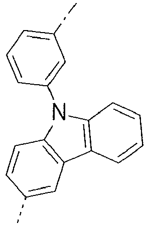 | 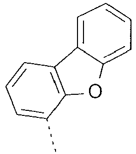 |
| A-102, B-102, C-102, D-102, E-102, F-102, G-102, H-102 | 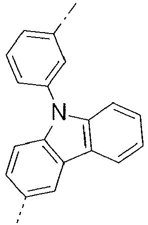 | 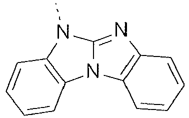 |
| A-103, B-103, C-103, D-103, E-103, F-103, G-103, H-103 | 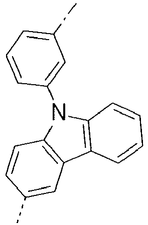 | 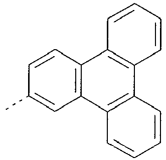 |
| A-104, B-104, C-104, D-104, E-104, F-104, G-104, H-104 | 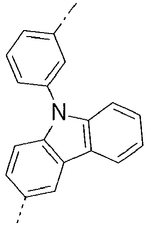 | 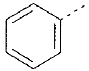 |
| A-105, B-105, C-105, D-105, E-105, F-105, G-105, H-105 | 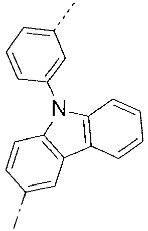 | 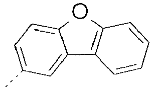 |
| A-106, B-106, C-106, D-106, E-106, F-106, G-106, H-106 | 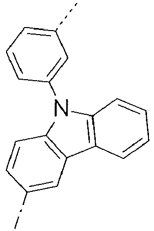 | 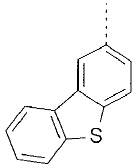 |
| A-107, B-107, C-107, D-107, E-107, F-107, G-107, H-107 | 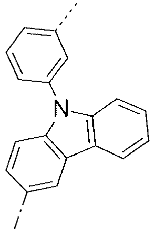 | 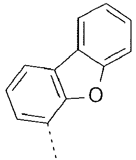 |
| A-108, B-108, C-108, D-108, E-108, F-108, G-108, H-108 | 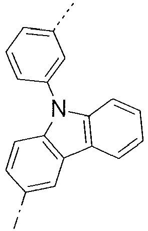 | 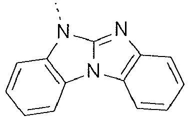 |
| A-109, B-109, C-109, D-109, E-109, F-109, G-109, H-109 | 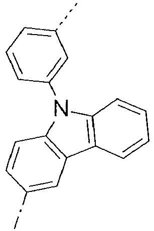 | 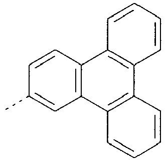 |
| A-110, B-110, C-110, D-110, E-110, F-110, G-110, H-110 | 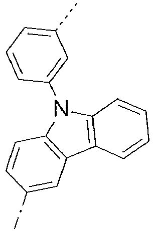 | 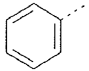 |
| A-111, B-111, C-111, D-111, E-111, F-111, G-111, H-111 | 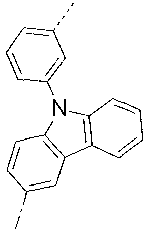 | 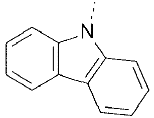 |
| A-112, B-112, C-112, D-112, E-112, F-112, G-112, H-112 | 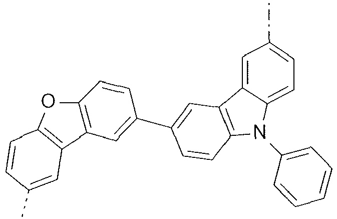 | 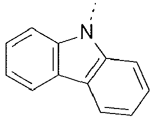 |
| A-113, B-113, C-113, D-113, E-113, F-113, G-113, H-113 | 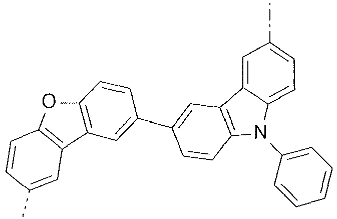 | 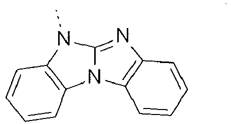 |
| A-114, B-114, C-114, D-114, E-114, F-114, G-114, H-114 | 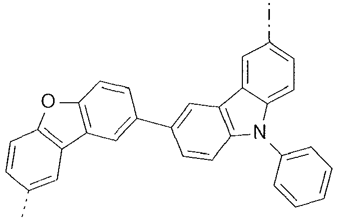 | 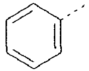 |
| A-115, B-115, C-115, D-115, E-115, F-115, G-115, H-115 | 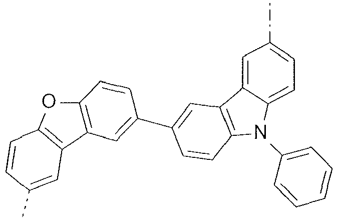 | 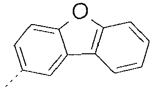 |
| A-116, B-116, C-116, D-116, E-116, F-116, G-116, H-116 | 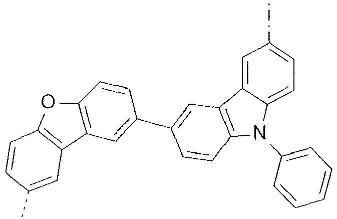 | 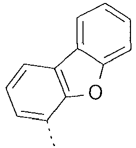 |
| A-117, B-117, C-117, D-117, E-117, F-117, G-117, H-117 | 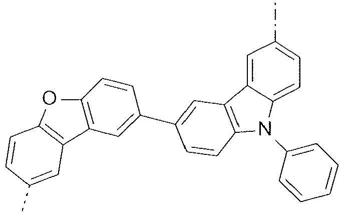 | 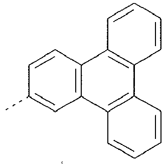 |
| A-118, B-118, C-118, D-118, E-118, F-118, G-118, H-118 | 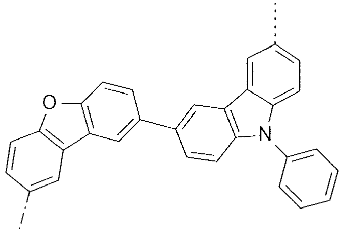 | 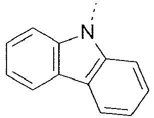 |
| A-119, B-119, C-119, D-119, E-119, F-119, G-119, H-119 | 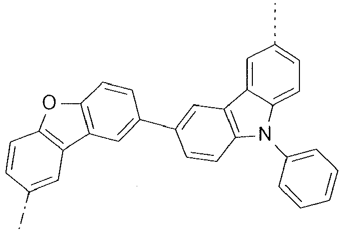 | 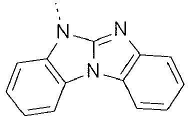 |
| A-120, B-120, C-120, D-120, E-120, F-120, G-120, H-120 | 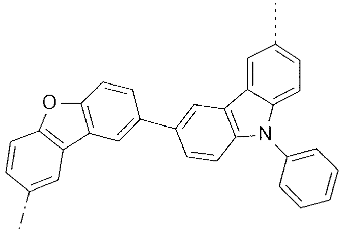 | 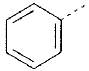 |
| A-121, B-121, C-121, D-121, E-121, F-121, G-121, H-121 | 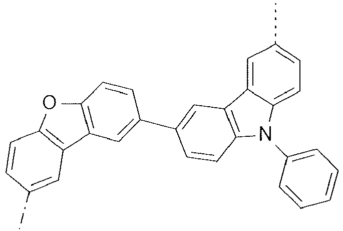 | 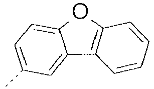 |
| A-122, B-122, C-122, D-122, E-122, F-122, G-122, H-122 | 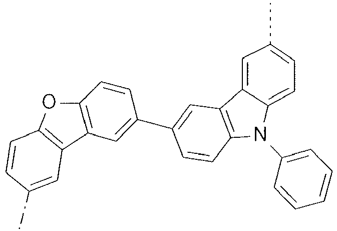 | 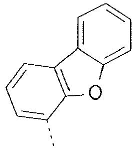 |
| A-123, B-123, C-123, D-123, E-123, F-123, G-123, H-123 | 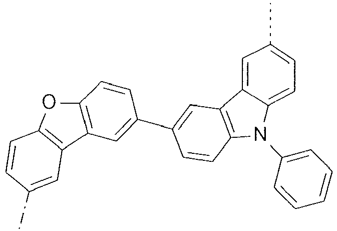 | 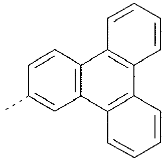 |
| A-124, B-124, C-124, D-124, E-124, F-124, G-124, H-124 | 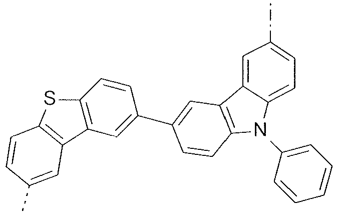 | 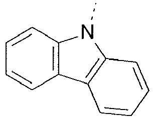 |
| A-125, B-125, C-125, D-125, E-125, F-125, G-125, H-125 | 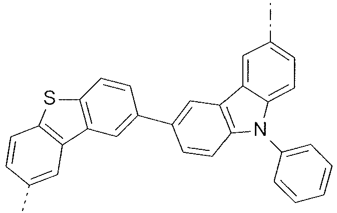 | 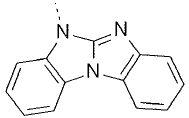 |
| A-126, B-126, C-126, D-126, E-126, F-126, G-126, H-126 | 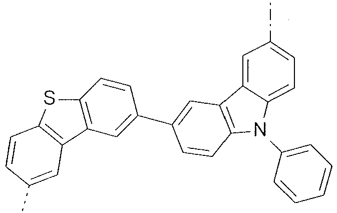 | 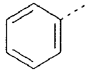 |
| A-127, B-127, C-127, D-127, E-127, F-127, G-127, H-127 | 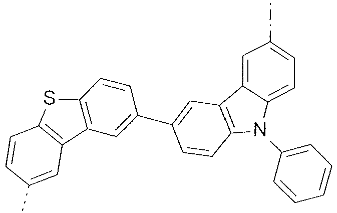 | 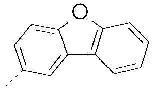 |
| A-128, B-128, C-128, D-128, E-128, F-128, G-128, H-128 | 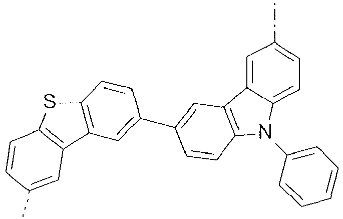 | 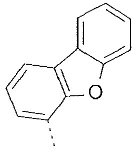 |
| A-129, B-129, C-129, D-129, E-129, F-129, G-129, H-129 | 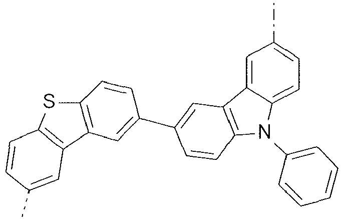 | 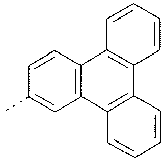 |
| A-130, B-130, C-130, D-130, E-130, F-130, G-130, H-130 | 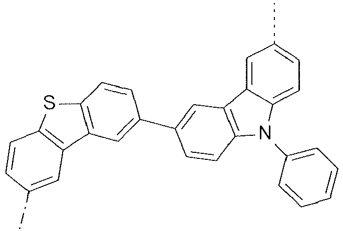 | 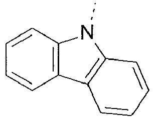 |
| A-131, B-131, C-131, D-131, E-131, F-131, G-131, H-131 | 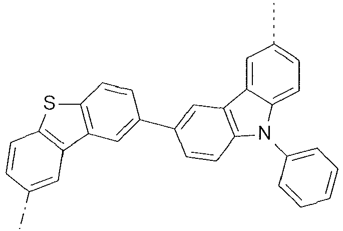 | 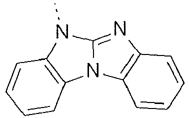 |
| A-132, B-132, C-132, D-132, E-132, F-132, G-132, H-132 | 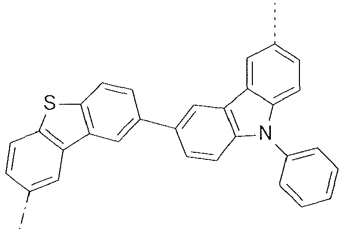 | 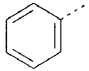 |
| A-133, B-133, C-133, D-133, E-133, F-133, G-133, H-133 | 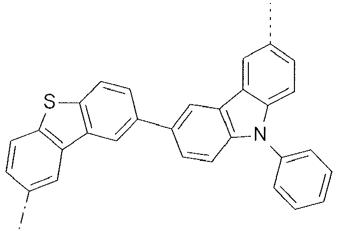 | 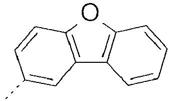 |
| A-134, B-134, C-134, D-134, E-134, F-134, G-134, H-134 | 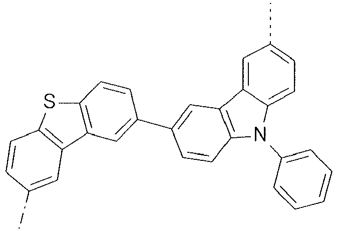 | 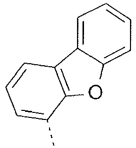 |
| A-135, B-135, C-135, D-135, E-135, F-135, G-135, H-135 | 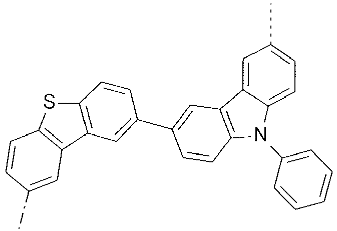 | 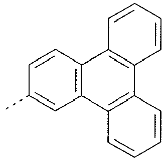 |
| A-136, B-136, C-136, D-136, E-136, F-136, G-136, H-136 | 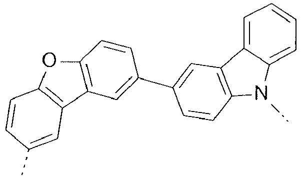 | 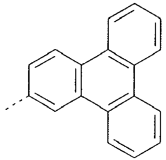 |
| A-137, B-137, C-137, D-137, E-137, F-137, G-137, H-137 | 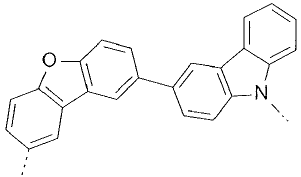 | 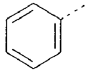 |
| A-138, B-138, C-138, D-138, E-138, F-138, G-138, H-138 | 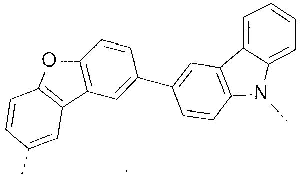 | 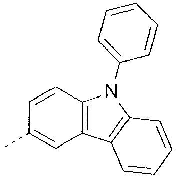 |
| A-139, B-139, C-139, D-139, E-139, F-139, G-139, H-139 | 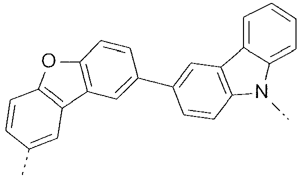 | 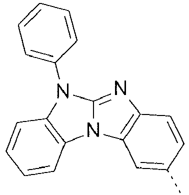 |
| A-140, B-140, C-140, D-140, E-140, F-140, G-140, H-140 | 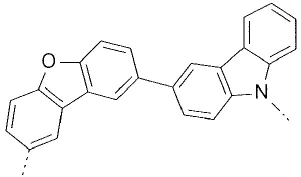 | 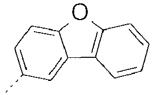 |
| A-141, B-141, C-141, D-141, E-141, F-141, G-141, H-141 | 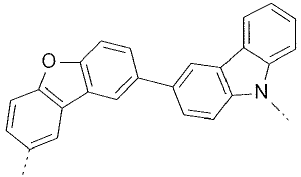 | 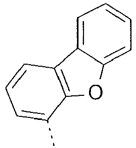 |
| A-142, B-142, C-142, D-142, E-142, F-142, G-142, H-142 | 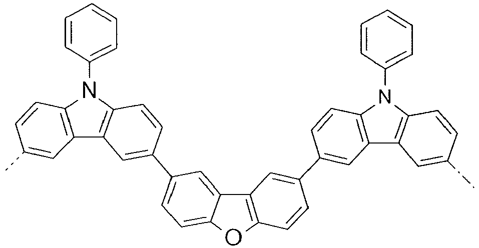 | 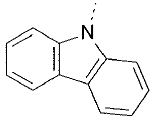 |
| A-143, B-143, C-143, D-143, E-143, F-143, G-143, H-143 | 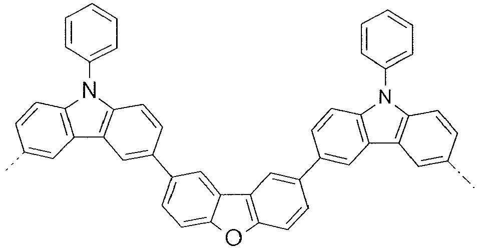 | 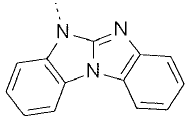 |
| A-144, B-144, C-144, D-144, E-144, F-144, G-144, H-144 | 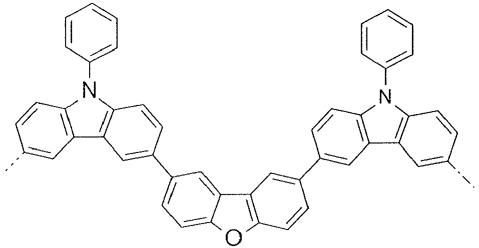 | 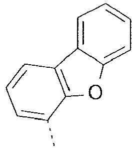 |
| A-145, B-145, C-145, D-145, E-145, F-145, G-145, H-145 | 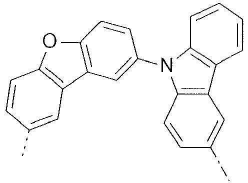 | 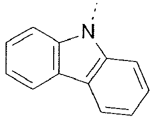 |
| A-146, B-146, C-146, D-146, E-146, F-146, G-146, H-146 | 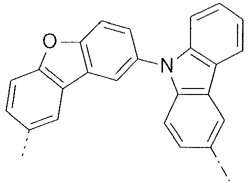 | 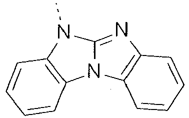 |
| A-147, B-147, C-147, D-147, E-147, F-147, G-147, H-147 | 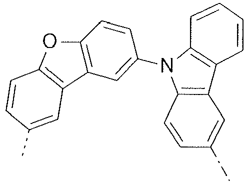 | 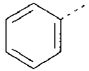 |
| A-148, B-148, C-148, D-148, E-148, F-148, G-148, H-148 | 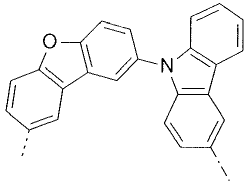 | 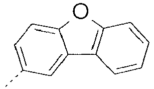 |
| A-149, B-149, C-149, D-149, E-149, F-149, G-149, H-149 | 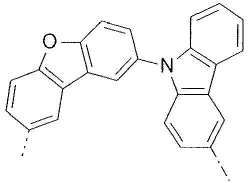 | 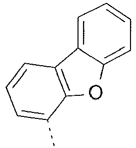 |
| A-150, B-150, C-150, D-150, E-150, F-150, G-150, H-150 | 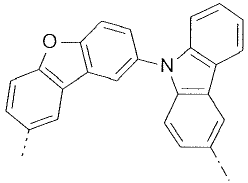 | 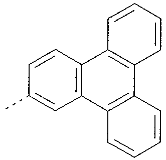 |
| A-151, B-151, C-151, D-151, E-151, F-151, G-151, H-151 | 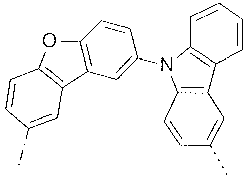 | 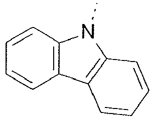 |
| A-152, B-152, C-152, D-152, E-152, F-152, G-152, H-152 | 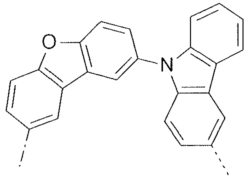 | 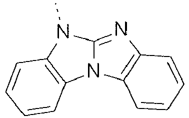 |
| A-153, B-153, C-153, D-153, E-153, F-153, G-153, H-153 | 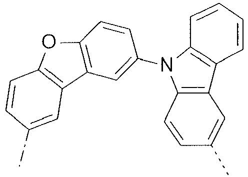 | 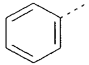 |
| A-154, B-154, C-154, D-154, E-154, F-154, G-154, H-154 | 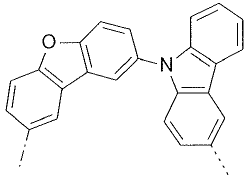 | 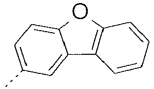 |
| A-155, B-155, C-155, D-155, E-155, F-155, G-155, H-155 | 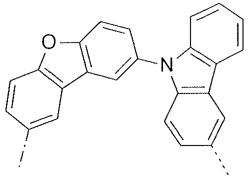 | 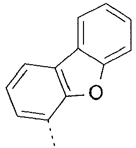 |
| A-156, B-156, C-156, D-156, E-156, F-156, G-156, H-156 | 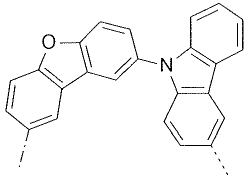 | 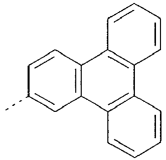 |
| A-157, B-157, C-157, D-157, E-157, F-157, G-157, H-157 | 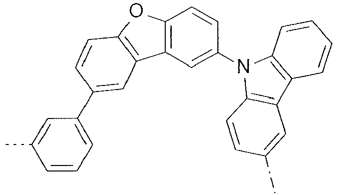 | 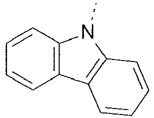 |
| A-158, B-158, C-158, D-158, E-158, F-158, G-158, H-158 | 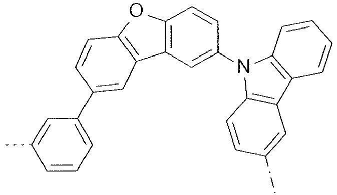 | 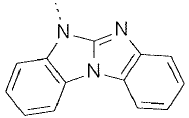 |
| A-159, B-159, C-159, D-159, E-159, F-159, G-159, H-159 | 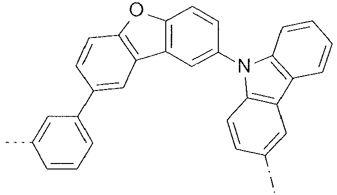 | 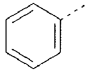 |
| A-160, B-160, C-160, D-160, E-160, F-160, G-160, H-160 | 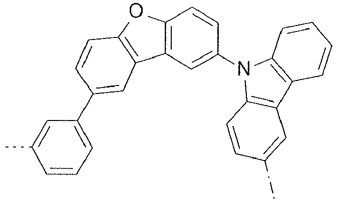 | 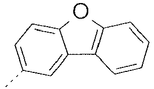 |
| A-161, B-161, C-161, D-161, E-161, F-161, G-161, H-161 | 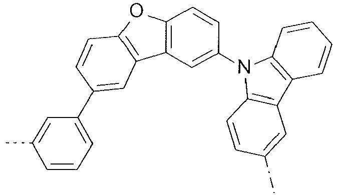 | 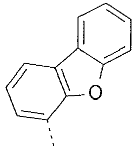 |
| A-162, B-162, C-162, D-162, E-162, F-162, G-162, H-162 | 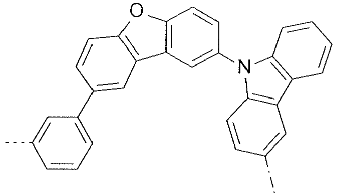 | 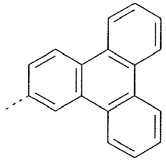 |
| A-163, B-163, C-163, D-163, E-163, F-163, G-163, H-163 | 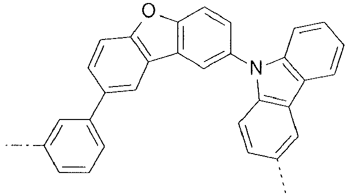 | 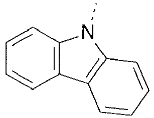 |
| A-164, B-164, C-164, D-164, E-164, F-164, G-164, H-164 | 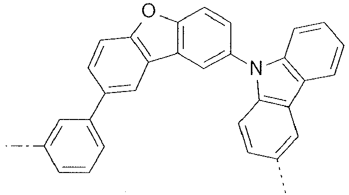 | 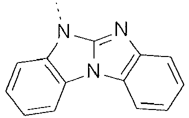 |
| A-165, B-165, C-165, D-165, E-165, F-165, G-165, H-165 | 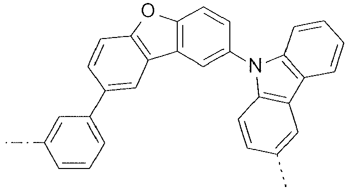 | 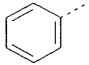 |
| A-166, B-166, C-166, D-166, E-166, F-166, G-166, H-166 | 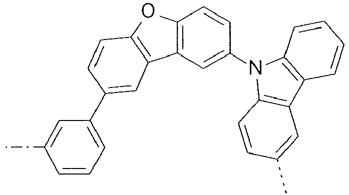 | 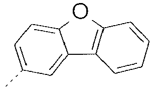 |
| A-167, B-167, C-167, D-167, E-167, F-167, G-167, H-167 | 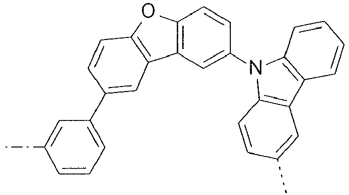 | 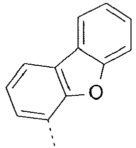 |
| A-168, B-168, C-168, D-168, E-168, F-168, G-168, H-168 | 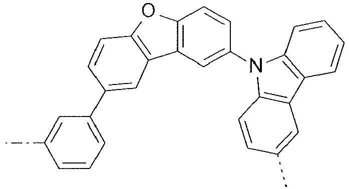 | 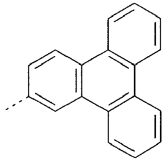 |
| A-169, B-169, C-169, D-169, E-169, F-169, G-169, H-169 | 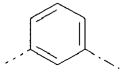 | 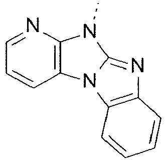 |
| A-170, B-170, C-170, D-170, E-170, F-170, G-170, H-170 | 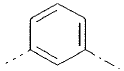 | 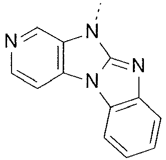 |
| A-171, B-171, C-171, D-171, E-171, F-171, G-171, H-171 | 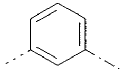 | 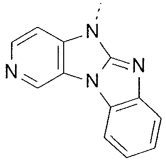 |
| A-172, B-172, C-172, D-172, E-172, F-172, G-172, H-172 | 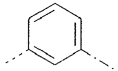 | 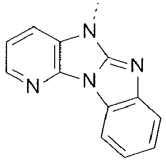 |
| A-173, B-173, C-173, D-173, E-173, F-173, G-173, H-173 | 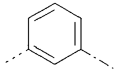 | 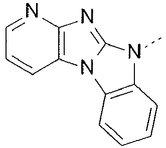 |
| A-174, B-174, C-174, D-174, E-174, F-174, G-174, H-174 | 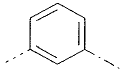 | 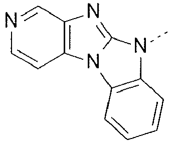 |
| A-175, B-175, C-175, D-175, E-175, F-175, G-175, H-175 | 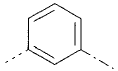 | 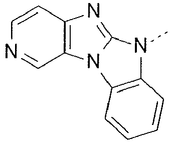 |
| A-176, B-176, C-176, D-176, E-176, F-176, G-176, H-176 | 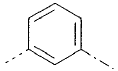 | 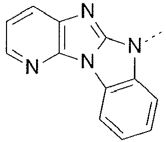 |
| A-177, B-177, C-177, D-177, E-177, F-177, G-177, H-177 | 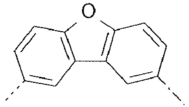 | 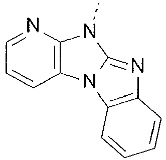 |
| A-178, B-178, C-178, D-178, E-178, F-178, G-178, H-178 | 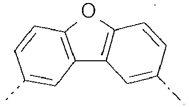 | 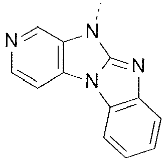 |
| A-179, B-179, C-179, D-179, E-179, F-179, G-179, H-179 | 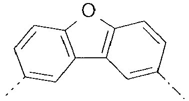 | 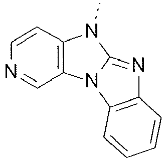 |
| A-180, B-180, C-180, D-180, E-180, F-180, G-180, H-180 | 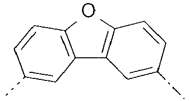 | 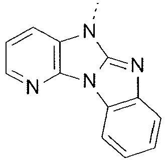 |
| A-181, B-181, C-181, D-181, E-181, F-181, G-181, H-181 | 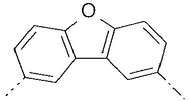 | 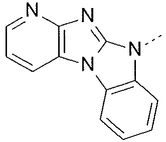 |
| A-182, B-182, C-182, D-182, E-182, F-182, G-182, H-182 | 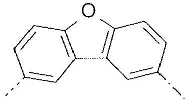 | 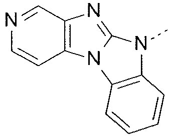 |
| A-183, B-183, C-183, D-183, E-183, F-183, G-183, H-183 | 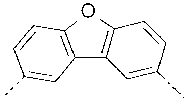 | 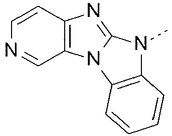 |
| A-184, B-184, C-184, D-184, E-184, F-184, G-184, H-184 | 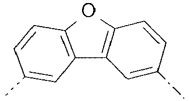 | 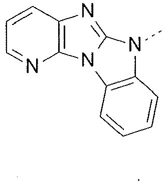 |
| A-185, B-185, C-185, D-185, E-185, F-185, G-185, H-185 | 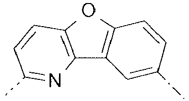 | 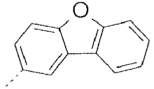 |
| A-186, B-186, C-186, D-186, E-186, F-186, G-186, H-186 | 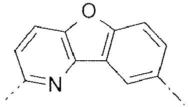 | 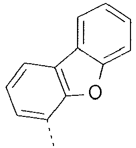 |
| A-187, B-187, C-187, D-187, E-187, F-187, G-187, H-187 | 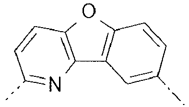 | 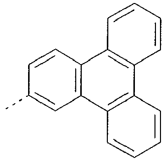 |
| A-188, B-188, C-188, D-188, E-188, F-188, G-188, H-188 | 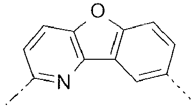 | 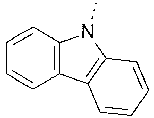 |
| A-189, B-189, C-189, D-189, E-189, F-189, G-189, H-189 | 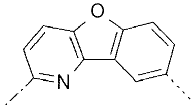 | 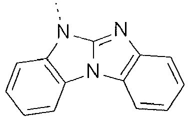 |
| A-190, B-190, C-190, D-190, E-190, F-190, G-190, H-190 | 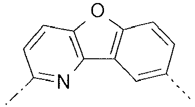 | 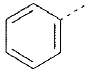 |
| A-191, B-191, C-191, D-191, E-191, F-191, G-191, H-191 | 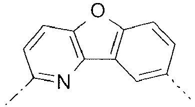 | 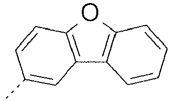 |
| A-192, B-192, C-192, D-192, E-192, F-192, G-192, H-192 | 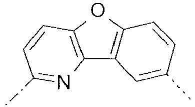 | 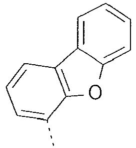 |
| A-193, B-193, C-193, D-193, E-193, F-193, G-193, H-193 | 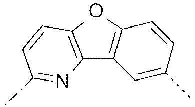 | 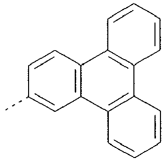 |
| A-194, B-194, C-194, D-194, E-194, F-194, G-194, H-194 | 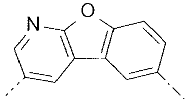 | 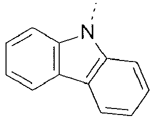 |
| A-195, B-195, C-195, D-195, E-195, F-195, G-195, H-195 | 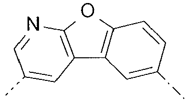 | 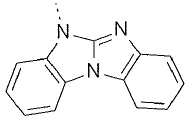 |
| A-196, B-196, C-196, D-196, E-196, F-196, G-196, H-196 | 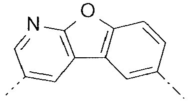 | 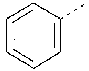 |
| A-197, B-197, C-197, D-197, E-197, F-197, G-197, H-197 | 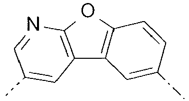 | 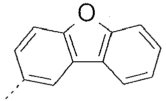 |
| A-198, B-198, C-198, D-198, E-198, F-198, G-198, H-198 | 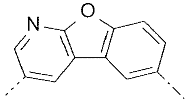 | 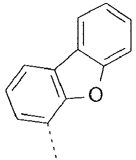 |
| A-199, B-199, C-199, D-199, E-199, F-199, G-199, H-199 | 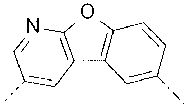 | 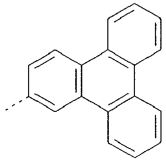 |
| A-200, B-200, C-200, D-200, E-200, F-200, G-200, H-200 | 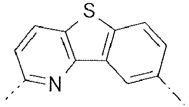 | 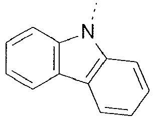 |
| A-201, B-201, C-201, D-201, E-201, F-201, G-201, H-201 | 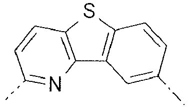 | 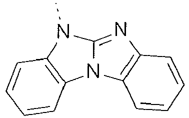 |
| A-202, B-202, C-202, D-202, E-202, F-202, G-202, H-202 | 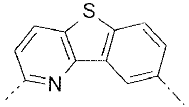 | 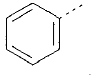 |
| A-203, B-203, C-203, D-203, E-203, F-203, G-203, H-203 | 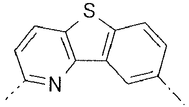 | 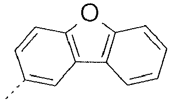 |
| A-204, B-204, C-204, D-204, E-204, F-204, G-204, H-204 | 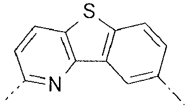 | 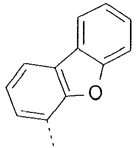 |
| A-205, B-205, C-205, D-205, E-205, F-205, G-205, H-205 | 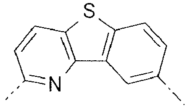 | 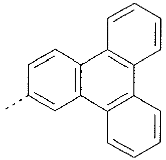 |
| A-206, B-206, C-206, D-206, E-206, F-206, G-206, H-206 | 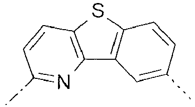 | 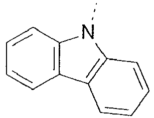 |
| A-207, B-207, C-207, D-207, E-207, F-207, G-207, H-207 | 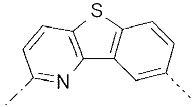 | 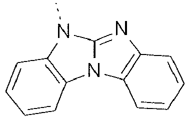 |
| A-208, B-208, C-208, D-208, E-208, F-208, G-208, H-208 | 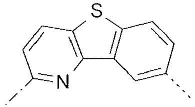 | 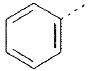 |
| A-209, B-209, C-209, D-209, E-209, F-209, G-209, H-209 | 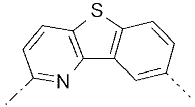 | 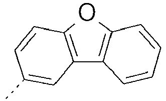 |
| A-210, B-210, C-210, D-210, E-210, F-210, G-210, H-210 | 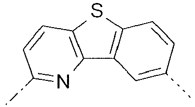 | 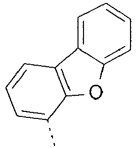 |
| A-211, B-211, C-211, D-211, E-211, F-211, G-211, H-211 | 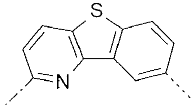 | 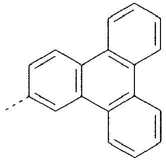 |
| A-212, B-212, C-212, D-212, E-212, F-212, G-212, H-212 | 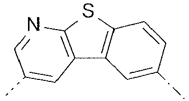 | 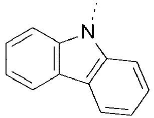 |
| A-213, B-213, C-213, D-213, E-213, F-213, G-213, H-213 | 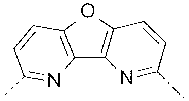 | 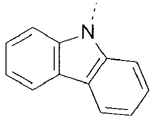 |
| A-214, B-214, C-214, D-214, E-214, F-214, G-214, H-214 | 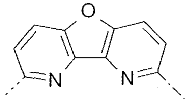 | 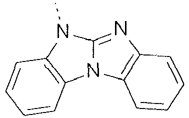 |
| A-215, B-215, C-215, D-215, E-215, F-215, G-215, H-215 | 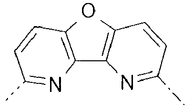 | 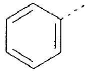 |
| A-216, B-216, C-216, D-216, E-216, F-216, G-216, H-216 | 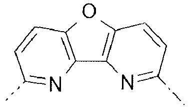 | 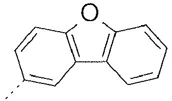 |
| A-217, B-217, C-217, D-217, E-217, F-217, G-217, H-217 | 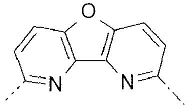 | 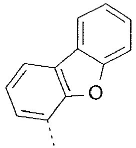 |
| A-218, B-218, C-218, D-218, E-218, F-218, G-218, H-218 | 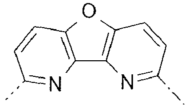 | 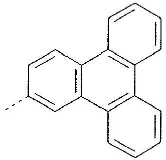 |
| A-219, B-219, C-219, D-219, E-219, F-219, G-219, H-219 | 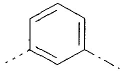 | 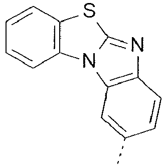 |
| A-220, B-220, C-220, D-220, E-220, F-220, G-220, H-220 | 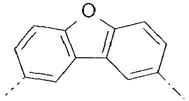 | 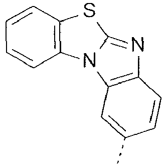 |
| A-221, B-221, C-221, D-221, E-221, F-221, G-221, H-221 | 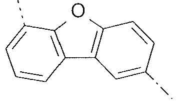 | 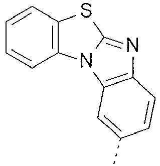 |
| A-222, B-222, C-222, D-222, E-222, F-222, G-222, H-222 | 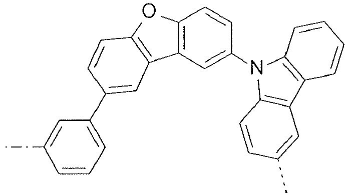 | 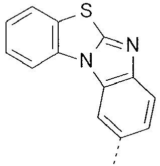 |
| A-223, B-223, C-223, D-223, E-223, F-223, G-223, H-223 | 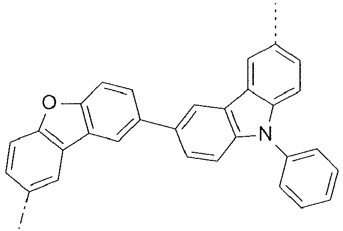 | 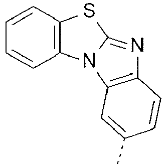 |
| A-224, B-224, C-224, D-224, E-224, F-224, G-224, H-224 | 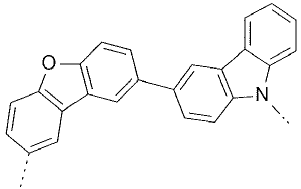 | 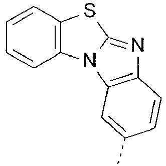 |

| | | |
|---|---|---|
| represents the bonding to R¹⁶. | | |

Compounds of the formula **B**, **D**, **F** and **H** ((**Ib-2**), (**Ib-4**), (**Ia-2**) or (**Ia-4**)) are preferred. Examples of preferred compounds of formulae Ia and Ib are compounds **B-1** to **B-184**, **D-1** to **D-184**, **F-1** to **F-184**, **H-1** to **H-184** shown above. Compounds **B-1** to **B-3**, **B-12**, **F-1**, **D-180**, **D-184** and **H-184** are particularly preferred.

Halogen is fluorine, chlorine, bromine and iodine.

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

The term "cycloalkyl group" is typically C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

C₆-C₂₄aryl (C₆-C₁₈aryl), which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted. Phenyl, 1-naphthyl and 2-naphthyl are examples of a C₆-C₁₀aryl group.

C₇-C₂₅aralkyl is typically benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

C₂-C₃₀heteroaryl represents a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

C₆-C₂₄arylen groups, which optionally can be substituted by G, are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted. Preferred C₆-C₂₄arylen groups are 1,3-phenylene, 3,3'-biphenylylene, 3,3'-m-terphenylene, 2- or 9-fluorenylene, phenanthrylene, which may be unsubstituted or substituted.

C₂-C₃₀heteroarylen groups, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated -electrons such as thienylene, benzothiophenylene, dibenzothiophenylene, thianthrenylene, furylene, furfurylene, 2H-pyranylene, benzofuranylene, isobenzofuranylene, dibenzofuranylene, phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene, benzimidazo[1,2-a]benzimidazo-2,5-ylene, or phenoxazinylene, which can be unsubstituted or substituted. Preferred C₂-C₃₀heteroarylen groups are pyridylene, triazinylene, pyrimidinylene, carbazolylene, dibenzofuranylene and benzimidazo[1,2-a]benzimidazo-2,5-ylene 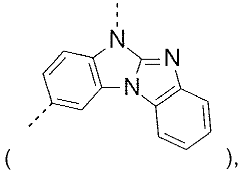 which can be unsubstituted or substituted, especially by C₆-C₁₀aryl, C₆-C₁₀aryl which is substituted by C₁-C₄alkyl; or C₂-C₁₄heteroaryl.

Possible substituents of the above-mentioned groups are C₁-C₈alkyl, a hydroxyl group, a mercapto group, C₁-C₈alkoxy, C₁-C₈alkylthio, halogen, halo-C₁-C₈alkyl, or a cyano group. The C₆-C₂₄aryl (C₆-C₁₈aryl) and C₂-C₃₀heteroaryl groups are preferably substituted by one, or more C₁-C₈alkyl groups.

If a substituent occurs more than one time in a group, it can be different in each occurrence.

Halo-C₁-C₈alkyl is an alkyl group where at least one of the hydrogen atoms is replaced by a halogen atom. Examples are -CF₃, -CF₂CF₃, -CF₂CF₂CF₃, -CF(CF₃)₂, -(CF₂)₃CF₃, and -C(CF₃)₃.

The wording "substituted by G" means that one, or more, especially one to three substituents G might be present.

As described above, the aforementioned groups may be substituted by E and/or, if desired, interrupted by D. Interruptions are of course possible only in the case of groups containing at least 2 carbon atoms connected to one another by single bonds; C₆-C₁₈aryl is not interrupted; interrupted arylalkyl contains the unit D in the alkyl moiety. C₁-C₁₈alkyl substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y}')-CH₂-O-R^{y}, where R^{y} is C₁-C₁₈alkyl, C₅-C₁₂cycloalkyl, phenyl, C₇-C₁₅phenylalkyl, and R^{y}' embraces the same definitions as R^{y} or is H;
C₁-C₈alkylene-COO-R^{z}, e.g. CH₂COOR_{z}, CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂.

An alkyl group substituted by E is, for example, an alkyl group where at least one of the hydrogen atoms is replaced by F. Examples are -CF₃, -CF₂CF₃,
-CF₂CF₂CF₃, -CF(CF₃)₂, -(CF₂)₃CF₃, and -C(CF₃)₃.

The synthesis of 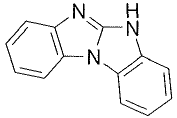 is described, for example, in Achour, Reddouane; Zniber, Rachid, Bulletin des Societes Chimiques Belges 96 (1987) 787-92.

Suitable base skeletons of the formula 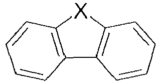 are either commercially available (especially in the cases when X is S, O, NH), or can be obtained by processes known to those skilled in the art. Reference is made to WO2010079051 and EP1885818.

The halogenation can be performed by methods known to those skilled in the art. Preference is given to brominating or iodinating in the 3 and 6 positions (dibromination) or in the 3 or 6 positions (monobromination) of the base skeleton of the formula 2,8 positions (dibenzofuran and dibenzothiophene) or 3,6 positions (carbazole).

Optionally substituted dibenzofurans, dibenzothiophenes and carbazoles can be dibrominated in the 2,8 positions (dibenzofuran and dibenzothiophene) or 3,6 positions (carbazole) with bromine or NBS in glacial acetic acid or in chloroform. For example, the bromination with Br₂ can be effected in glacial acetic acid or chloroform at low temperatures, e.g. 0°C. Suitable processes are described, for example, in M. Park, J.R. Buck, C.J. Rizzo, Tetrahedron, 54 (1998) 12707-12714 for X= NPh, and in W. Yang et al., J. Mater. Chem. 13 (2003) 1351 for X= S. In addition, 3,6-dibromocarbazole, 3,6-dibromo-9-phenylcarbazole, 2,8-dibromodibenzothiophene, 2,8-dibromodibenzofuran, 2-bromocarbazole, 3-bromodibenzothiophene, 3-bromodibenzofuran, 3-bromocarbazole, 2-bromodibenzothiophene and 2-bromodibenzofuran are commercially available.

Monobromination in the 4 position of dibenzofuran (and analogously for dibenzothiophene) is described, for example, in J. Am. Chem. Soc. 1984, 106, 7150. Dibenzofuran (dibenzothiophene) can be monobrominated in the 3 position by a sequence known to those skilled in the art, comprising a nitration, reduction and subsequent Sandmeyer reaction.

Monobromination in the 2 position of dibenzofuran or dibenzothiophene and monobromination in the 3 position of carbazole are effected analogously to the dibromination, with the exception that only one equivalent of bromine or NBS is added.

Alternatively, it is also possible to utilize iodinated dibenzofurans, dibenzothiophenes and carbazoles. The preparation is described, inter alia, in Tetrahedron. Lett. 47 (2006) 6957-6960, Eur. J. Inorg. Chem. 24 (2005) 4976-4984, J. Heterocyclic Chem. 39 (2002) 933-941, J. Am. Chem. Soc. 124 (2002) 11900-11907, J. Heterocyclic Chem, 38 (2001) 77-87.

For the nucleophilic substitution, Cl- or F-substituted dibenzofurans, dibenzothiophenes and carbazoles are required. The chlorination is described, inter alia, in J. Heterocyclic Chemistry, 34 (1997) 891-900, Org. Lett., 6 (2004) 3501-3504; J. Chem. Soc. [Section] C: Organic, 16 (1971) 2775-7, Tetrahedron Lett. 25 (1984) 5363-6, J. Org. Chem. 69 (2004) 8177-8182. The fluorination is described in J. Org. Chem. 63 (1998) 878-880 and J. Chem. Soc., Perkin Trans. 2, 5 (2002) 953-957.

The introduction of the group 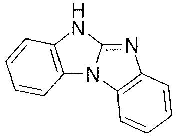 is performed in the presence of a base. Suitable bases are known to those skilled in the art and are preferably selected from the group consisting of alkali metal and alkaline earth metal hydroxides such as NaOH, KOH, Ca(OH)₂, alkali metal hydrides such as NaH, KH, alkali metal amides such as NaNH₂, alkali metal or alkaline earth metal carbonates such as K₂CO₃ or Cs₂CO₃, and alkali metal alkoxides such as NaOMe, NaOEt. In addition, mixtures of the aforementioned bases are suitable. Particular preference is given to NaOH, KOH, NaH or K₂CO₃.

Heteroarylation can be affected, for example, by copper-catalyzed coupling of 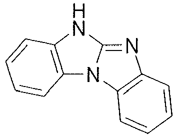 to a halogenated compound of the formula 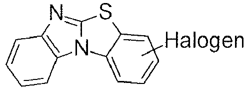 (Ullmann reaction).

The N-arylation is, for example, disclosed in H. Gilman and D. A. Shirley, J. Am. Chem. Soc. 66 (1944) 888; D. Li et al., Dyes and Pigments 49 (2001) 181 - 186 and Eur. J. Org. Chem. (2007) 2147-2151. The reaction can be performed in solvent or in a melt. Suitable solvents are, for example, (polar) aprotic solvents such as dimethyl sulfoxide, dimethylformamide, N-methyl-2-pyrrolidone (NMP), tridecane or alcohols.

The synthesis of 9-(8-bromodibenzofuran-2-yl)carbazole, 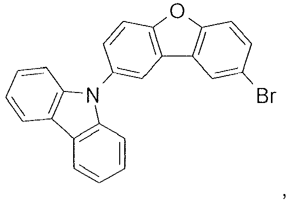 is described in WO2010079051. The synthesis of 2-bromo-8-iodo-dibenzofurane, 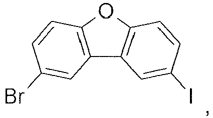 is described in EP1885818.

Diboronic acid or diboronate group containing dibenzofurans, dibenzothiophenes and carbazoles can be readily prepared by an increasing number of routes. An overview of the synthetic routes is, for example, given in Angew. Chem. Int. Ed. 48 (2009) 9240 - 9261.

By one common route diboronic acid or diboronate group containing dibenzofurans, dibenzothiophenes, and carbazoles can be obtained by reacting halogenated dibenzofurans, dibenzothiophenes and carbazoles with (Y¹O)₂B-B(OY¹)₂, 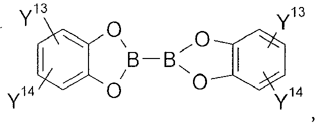 or 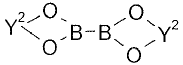 in the presence of a catalyst, such as, for example, [1,1'-bis(diphenylphosphino)ferrocene]dichloropalladium(II), complex (Pd(Cl)₂(dppf)), and a base, such as, for example, potassium acetate, in a solvent, such as, for example, dimethyl formamide, dimethyl sulfoxide, dioxane and/or toluene (cf. Prasad Appukkuttan et al., Synlett 8 (2003) 1204), wherein Y¹ is independently in each occurrence a C₁-C₁₈alkylgroup and Y² is independently in each occurrence a C₂-C₁₀alkylene group, such as -CY³Y⁴-CY⁵Y⁶-, or -CY⁷Y⁸-CY⁹Y¹⁰-CY¹¹Y¹²- wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-,-C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup.

Diboronic acid or diboronate group containing dibenzofurans, dibenzothiophenes and carbazoles can also be prepared by reacting halogenated dibenzofurans, dibenzothiophenes and carbazoles with alkyl lithium reagents, such as, for example, n-butyl lithium, or t-buthyl lithium, followed by reaction with boronic esters, such as, for example, B(isopropoxy)₃, B(methoxy)₃, or 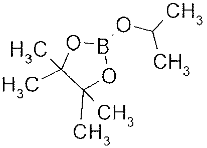 (cf. Synthesis (2000) 442-446).

Diboronic acid or diboronate group containing dibenzofurans, dibenzothiophenes and carbazoles can also be prepared by reacting dibenzofurans, dibenzothiophenes and carbazoles with lithium amides, such as, for example, lithium diisopropylamide (LDA) followed by reaction with boronic esters such as, for example, B(isopropoxy)₃, B(methoxy)₃, or 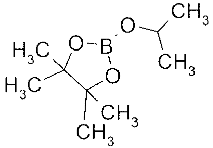 (J. Org. Chem. 73 (2008) 2176-2181).

Diboronic acid or diboronate group containing dibenzofurans, dibenzothiophenes and carbazoles, such as, for example, 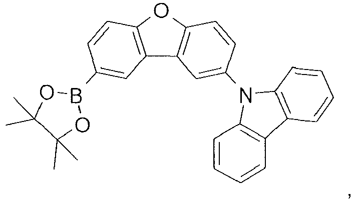 can be reacted with equimolar amounts of halogenated dibenzofurans, dibenzothiophenes, carbazoles and 4H-imidazo[1,2-a]imidazoles, such as, for example, 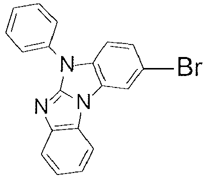 and 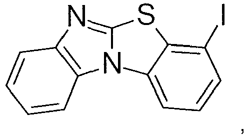 in a solvent and in the presence of a catalyst. The catalyst may be one of the µl-halo(triisopropylphosphine)(η³-allyl)palladium(II) type (see for example WO99/47474).

Preferably, the Suzuki reaction is carried out in the presence of an organic solvent, such as an aromatic hydrocarbon or a usual polar organic solvent, such as benzene, toluene, xylene, tetrahydrofurane, or dioxane, or mixtures thereof, most preferred toluene. Usually, the amount of the solvent is chosen in the range of from 1 to 10 I per mol of boronic acid derivative. Also preferred, the reaction is carried out under an inert atmosphere such as nitrogen, or argon. Further, it is preferred to carry out the reaction in the presence of an aqueous base, such as an alkali metal hydroxide or carbonate such as NaOH, KOH, Na₂CO₃, K₂CO₃, Cs₂CO₃ and the like, preferably an aqueous K₂CO₃ solution is chosen. Usually, the molar ratio of the base to boronic acid or boronic ester derivative is chosen in the range of from 0.5:1 to 50:1, very especially 1:1. Generally, the reaction temperature is chosen in the range of from 40 to 180°C, preferably under reflux conditions. Preferred, the reaction time is chosen in the range of from 1 to 80 hours, more preferably from 20 to 72 hours. In a preferred embodiment a usual catalyst for coupling reactions or for polycondensation reactions is used, preferably Pd-based, which is described in WO2007/101820. The palladium compound is added in a ratio of from 1:10000 to 1:50, preferably from 1:5000 to 1:200, based on the number of bonds to be closed. Preference is given, for example, to the use of palladium(II) salts such as PdAc₂ or Pd₂dba₃ and to the addition of ligands selected from the group consisting of 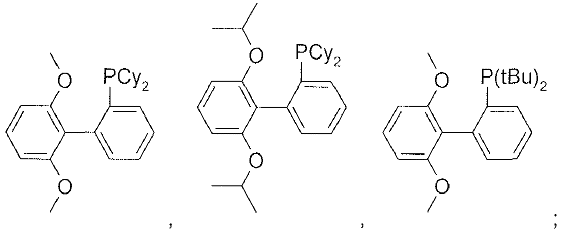 wherein Cy = 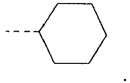 The ligand is added in a ratio of from 1:1 to 1:10, based on Pd. Also preferred, the catalyst is added as in solution or suspension. Preferably, an appropriate organic solvent such as the ones described above, preferably benzene, toluene, xylene, THF, dioxane, more preferably toluene, or mixtures thereof, is used. The amount of solvent usually is chosen in the range of from 1 to 10 l per mol of boronic acid derivative. Organic bases, such as, for example, tetraalkylammonium hydroxide, and phase transfer catalysts, such as, for example TBAB, can promote the activity of the boron (see, for example, Lead-beater & Marco; Angew. Chem. Int. Ed. Eng. 42 (2003) 1407 and references cited therein). Other variations of reaction conditions are given by T. I. Wallow and B. M. Novak in J. Org. Chem. 59 (1994) 5034-5037; and M. Remmers, M. Schulze, G. Wegner in Macromol. Rapid Commun. 17 (1996) 239-252 and G. A. Molander und B. Canturk, Angew. Chem. , 121 (2009) 9404 - 9425.

The synthesis of aza- and diaza-dibenzofuran is known in the literature, or can be done in analogy to known procedures. Reference is made, for example, to JP2011084531, US2010/0187984, L. Kaczmarek, Polish Journal of Chemistry 59 (1985) 1141, JP2002284862, Y. Fort, Tetrahedron 50 (41),11893 (1994) and J. Liu, J. Org. Chem. 73, 2951 (2008).

The synthesis of benzimidazolo[2,1-b][1,3]benzothiazole is, for example, described by Z. Wu et al., Eur. J. Org. Chem. (2011) 5242-5245:

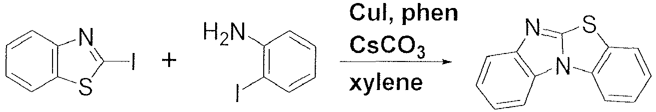

The halogenation can be performed by methods known to those skilled in the art.

4-lodobenzimidazolo[2,1-b][1,3]benzothiazole can be obtained by reacting enzimidazolo[2,1-b][1,3]benzothiazole with butyl lithium and l₂ in tetrahydrofurane. Heteroarylation can be effected, for example, by copper-catalyzed coupling of 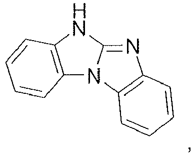 or 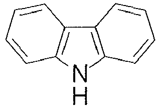 to 4-iodobenzimidazolo[2,1-b][1,3]benzothiazole (Ullmann reaction).

4-Chlorobenzimidazolo[2,1-b][1,3]benzothiazole can be prepared as described in Organic & Biomolecular Chemisty 10 (2012) 7944:

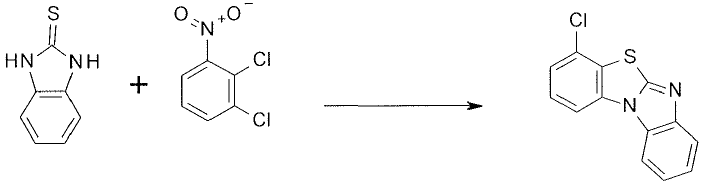

2-lodobenzimidazolo[2,1-b][1,3]benzothiazole can be obtained by reacting benzimidazolo[2,1-b][1,3]benzothiazole in CH₃COOH and CF₃COOH in the presence of N-iodosuccinimide (NIS).

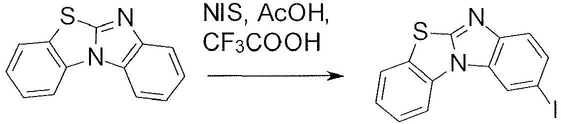

It has been found that the compounds of the formulae (Ia) and (Ib) are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs), the compounds of the formulae (Ia) and (Ib) being particularly suitable in OLEDs for use as matrix material in a light-emitting layer and/or as electron and/or exciton blocker material and/or as hole and/or exciton blocker material, especially in combination with a phosphorescence emitter. In the case of use of the inventive compounds of the formulae (Ia) and (Ib) in OLEDs, OLEDs which have good efficiencies and a long lifetime and which can be operated especially at a low use and operating voltage are obtained. The inventive compounds of the formulae (Ia) and (Ib) are suitable especially for use as matrix and/or charge/exciton blocker materials for blue and green emitters, for example light blue or deep blue emitters, these being especially phosphorescence emitters. Furthermore, the compounds of the formulae (Ia) and (Ib) can be used as conductor/complementary materials in organic electronics applications selected from switching elements and organic solar cells.

The compounds of the formulae (Ia) and (Ib) can be used as matrix material and/or charge/exciton blocker material and/or charge transport material (charge conductor material). The inventive compounds of the formulae (Ia) and (Ib) are preferably used as matrix materials in organic electronics applications, especially in OLEDs.

In the emission layer or one of the emission layers of an OLED, it is also possible to combine an emitter material with a matrix material of the compound of the formula (Ia) or (Ib) and a further matrix material which has, for example, a good hole transport property. This achieves a high quantum efficiency of this emission layer.

When a compound of the formula (Ia) or (Ib) is used as matrix (host) material in an emission layer and additionally as charge/exciton blocker material, owing to the chemical identity or similarity of the materials, an improved interface between the emission layer and the adjacent charge/exciton blocker material, which can lead to a decrease in the voltage with equal luminance and to an extension of the lifetime of the OLED. Moreover, the use of the same material for charge/exciton blocker material and for the matrix of an emission layer allows the production process of an OLED to be simplified, since the same source can be used for the vapor deposition process of the material of one of the compounds of the formula (Ia) or (Ib).

Suitable structures of organic electronic devices are known to those skilled in the art and are specified below.

The organic transistor generally includes a semiconductor layer formed from an organic layer with charge transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or charge transport capacity. In addition, it is possible for further layers known to those skilled in the art to be present in the organic solar cell. The layers with charge transport capacity may comprise the compounds of formula (Ia) or (Ib).

It is likewise possible that the compounds of the formula (Ia) or (Ib) are present both in the light-emitting layer (preferably as matrix material) and in the blocking layers (as charge/exciton blockers).

The present invention further provides an organic light-emitting diode comprising an anode (a) and a cathode (i) and a light-emitting layer (e) arranged between the anode (a) and the cathode (i), and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the at least one compound of the formula (Ia) or (Ib) is present in the light-emitting layer (e) and/or in at least one of the further layers. The at least one compound of the formula (Ia) or (Ib) is preferably present in the light-emitting layer and/or the charge/exciton blocking layers.

In a preferred embodiment of the present invention, at least one compound of the formula (Ia) or (Ib), especially a compound of the formula (**Ib-2**), (**Ib-4**), (**Ia-2**) or (**Ia-4**), is used as charge transport material. Examples of preferred compounds of formulae (Ia) and (Ib) are compounds **B-1** to **B-184**, **D-1** to **D-184**, **F-1** to **F-184**, **H-1** to **H-184** shown above. Compounds **B-1** to **B-3**, **B-12**, **F-1**, **D-180**, **D-184** and **H-184** are particularly preferred.

In another preferred embodiment of the present invention, at least one compound of the formula (Ia) or (Ib), especially a compound of the formula (**Ib-2**), (**Ib-4**), (**Ia-2**) or (**Ia-4**), is used as charge/exciton blocker material. Examples of preferred compounds of formulae (Ia) and (Ib) are compounds **B-1** to **B-184**, **D-1 to D-184**, **F-1** to **F-184**, **H-1** to **H-184** shown above. Compounds **B-1** to **B-3**, **B-12**, **F-1**, **D-180**, **D-184** and **H-184** are particularly preferred.

The present application further relates to a light-emitting layer comprising at least one compound of the formula (Ia) or (Ib).

### Structure of the inventive OLED

The inventive organic light-emitting diode (OLED) thus generally has the following structure:
an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the Light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron conduction layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA. Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

### Hole transport layer (c):

Either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (indolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 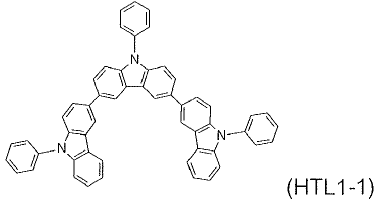 and 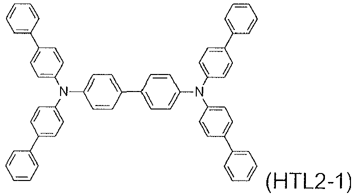 constitute the hole transport layer.

Customarily used hole-transporting molecules are selected from the group consisting of 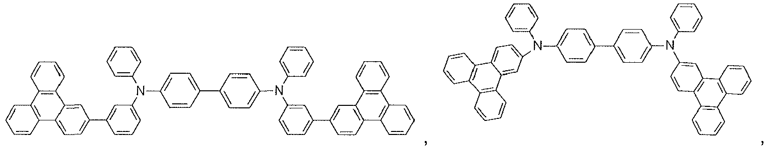 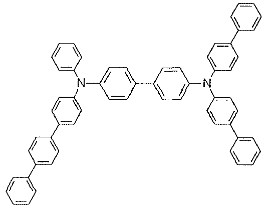 (4-phenyl-N-(4-phenylphenyl)-N-[4-[4-(N-[4-(4-phenylphenyl)phenyl]anilino)phenyl]phenyl]aniline), 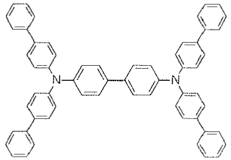 (4-phenyl-N-(4-phenylphenyl)-N-[4-[4-(4-phenyl-N-(4-phenylphenyl)anilino)phenyl]phenyl]aniline), 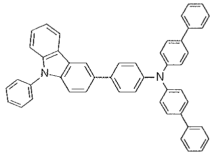 (4-phenyl-N-[4-(9-phenylcarbazol-3-yl)phenyl]-N-(4-phenylphenyl)aniline), 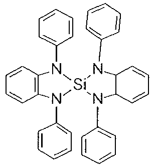 (1,1',3,3'-tetraphenylspiro[1,3,2-benzodiazasilole-2,2'-3a,7a-dihydro-1,3,2-benzodiazasilole]), 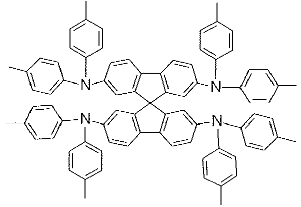 (N2,N2,N2',N2',N7,N7,N7',N7'-octakis(p-tolyl)-9,9'-spirobi[fluorene]-2,2',7,7'-tetramine),4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]-cyclohexane (TAPC), N,N'-bis(4-methylphenyl)-N,N'-bis(4-ethylphenyl)-[1,1'-(3,3'-dimethyl)-biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PE-DOT/PSS.

In a preferred embodiment it is possible to use metal carbene complexes as hole transport materials. Suitable carbene complexes are, for example, carbene complexes as described in WO2005/019373A2, WO2006/056418 A2, WO2005/113704, WO2007/115970, WO2007/115981, WO2008/000727 and PCT/EP2014/055520. One example of a suitable carbene complex is Ir(DPBIC)₃ with the formula: 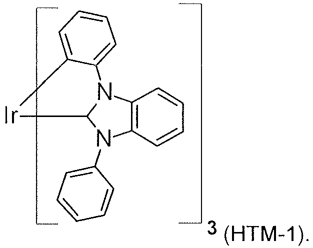 Another example of a suitable carbene complex is Ir(ABIC)₃ with the la:

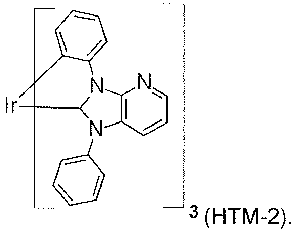

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6H-naphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254. Preferred mixtures comprise the aforementioned carbene complexes, such as, for example, the carbene complexes HTM-1 and HTM-2, and MoO₃ and/or ReO₃, especially MoO₃. In a particularly preferred embodiment the hole transport layer comprises from 0.1 to 10 wt % of MoO₃ and 90 to 99.9 wt % carbene complex, especially of the carbene complex **HTM-1** and **HTM-2**, wherein the total amount of the MoO₃ and the carbene complex is 100 wt %.

### Exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer. Suitable metal complexes for use as electron/exciton blocker material are, for example, carbene complexes as described in WO2005/019373A2, WO2006/056418A2, WO2005/113704, WO2007/115970, WO2007/115981, WO2008/000727 and PCT/EP2014/055520. Explicit reference is made here to the disclosure of the WO applications cited, and these disclosures shall be considered to be incorporated into the content of the present application. One example of a suitable carbene complex is compound **HTM-1** and **HTM-2.**

### Emitting layer (e)

The light-emitting layer (e) comprises at least one emitter material. In principle, it may be a fluorescence or phosphorescence emitter, suitable emitter materials being known to those skilled in the art. The at least one emitter material is preferably a phosphorescence emitter. The phosphorescence emitter compounds used with preference are based on metal complexes, and especially the complexes of the metals Ru, Rh, Ir, Pd and Pt, in particular the complexes of Ir, have gained significance. The compounds of the formulae (Ia) and (Ib) can be used as the matrix in the light-emitting layer.

Suitable metal complexes for use in the inventive OLEDs are described, for example, in documents WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldi-benzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetylacetonato)iridium(III), bis(2-phenylbenzothiazolato)(acetylacetonato)iridium(III), bis(2-(9,9-dihexylfluorenyl)-1-pyridine)(acetylacetonato)iridium(III) bis(2-benzo[b]thiophen-2-yl-pyridine)(acetylacetonato)iridium(III).

In addition, the following commercially available materials are suitable: tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenylphenanthroline)europium(III), tris(dibenzoylmethane)mono(4,7-di-methylphenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)-benzoylmethane)]mono(phenanthroline)europium(III) and tris[di[4-(2-(2-ethoxy-ethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Preferred phosphorescence emitters are carbene complexes. Suitable phosphorescent blue emitters are specified in the following publications: WO2006/056418A2, WO2005/113704, WO2007/115970, WO2007/115981, WO2008/000727, WO2009050281, WO2009050290, WO2011051404, US2011/057559 WO2011/073149, WO2012/121936A2, US2012/0305894A1, WO2012/170571, WO2012/170461, WO2012/170463, WO2006/121811, WO2007/095118, WO2008/156879, WO2008/156879, WO2010/068876, US2011/0057559, WO2011/106344, US2011/0233528, WO2012/048266, WO2012/172482, PCT/EP2014/064054 and PCT/EP2014/066272.

Preferably, the light emitting layer (e) comprises at least one carbine complex as phosphorescence emitter. Suitable carbine complexes are, for example, compounds of the formula 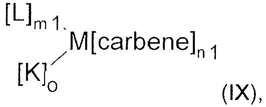 which are described in WO 2005/019373 A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
Carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof; phosphites; CO; pyridines; nitriles and conjugated dienes which form a π complex with M¹; n1 is the number of carbene ligands, where n1 is at least 1 and when n1 > 1 the carbene ligands in the complex of the formula I can be identical or different;
m1 is the number of ligands L, where m1 can be 0 or ≥ 1 and when m1 > 1 the ligands L can be identical or different;
o is the number of ligands K, where o can be 0 or ≥ 1 and when o > 1 the ligands K can be identical or different;
where the sum n1 + m1 + o is dependent on the oxidation state and coordination number of the metal atom and on the denticity of the ligands carbene, L and K and also on the charge on the ligands, carbene and L, with the proviso that n1 is at least 1.

More preferred are metal-carbene complexes of the general formula 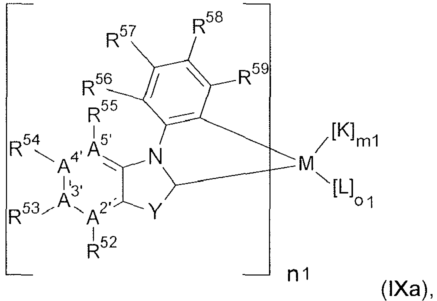 which are described in WO2011/073149, where M, n1, Y, A^{2'}, A^{3'}, A^{4'}, A^{5'}, R⁵¹, R⁵², R⁵³, R⁵⁴, R⁵⁵, R⁵⁶, R⁵⁷, R⁵⁸, R⁵⁹, K, L, m1 and o1 are each defined as follows:
M is Ir, or Pt,
n1 is an integer selected from 1, 2 and 3,
Y is NR⁵¹, O, S or C(R²⁵)₂,
A^{2'}, A^{3'}, A^{4'}, and A^{5'} are each independently N or C, where 2 A' = nitrogen atoms and at least one carbon atom is present between two nitrogen atoms in the ring,
R⁵¹ is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R⁵², R⁵³, R⁵⁴ and R⁵⁵ are each, if A^{2'}, A^{3'}, A^{4'} and/or A^{5'} is N, a free electron pair, or, if A^{2'} A^{3'}, A^{4'} and/or A^{5'} is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R⁵³ and R⁵⁴ together with A^{3'} and A^{4'} form an optionally substituted, unsaturated ring optionally interrupted by at least one further heteroatom and having a total of 5 to 18 carbon atoms and/or heteroatoms,
R⁵⁶, R⁵⁷, R⁵⁸ and R⁵⁹ are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R⁵⁶ and R⁵⁷, R⁵⁷ and R⁵⁸ or R⁵⁸ and R⁵⁹, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A^{5'} is C, R⁵⁵ and R⁵⁶ together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R²⁵ is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o1 is 0, 1 or 2, where, when o1 is 2, the L ligands may be the same or different.

The compound of formula IX is preferably a compound of the formula: 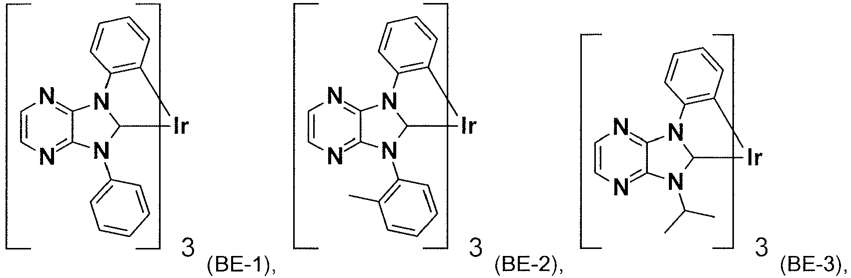 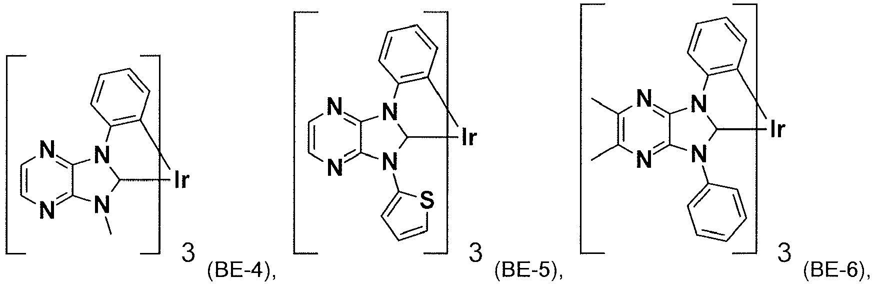 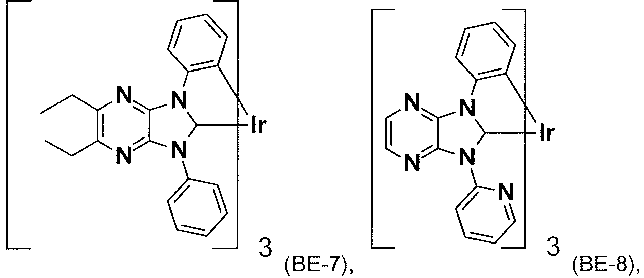 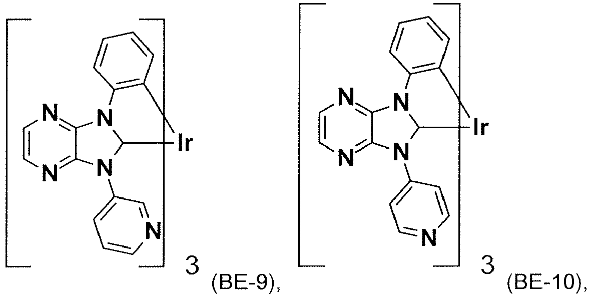 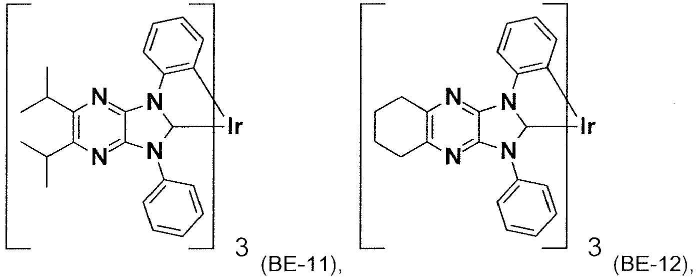 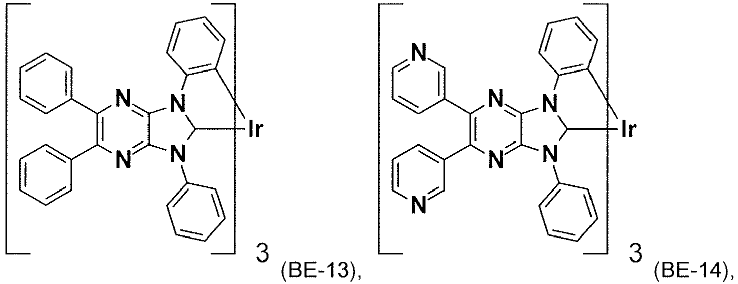 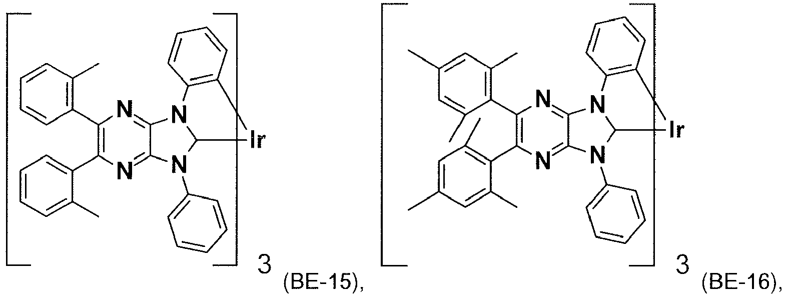 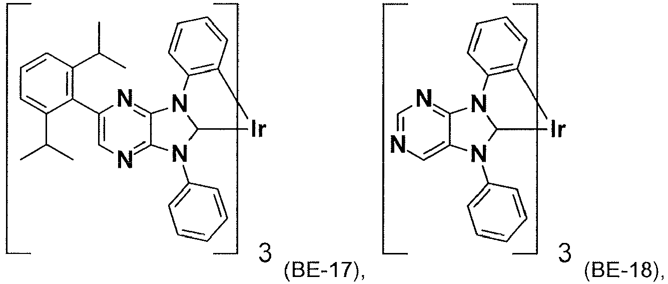 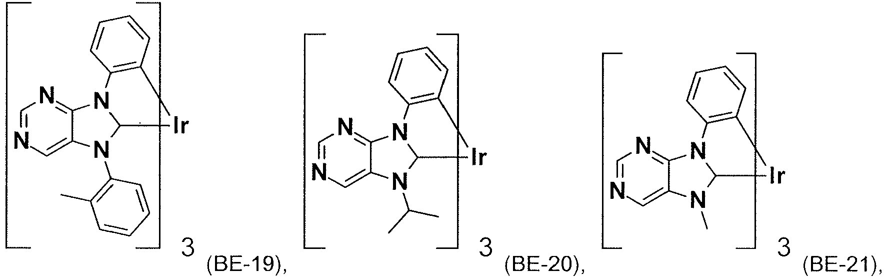 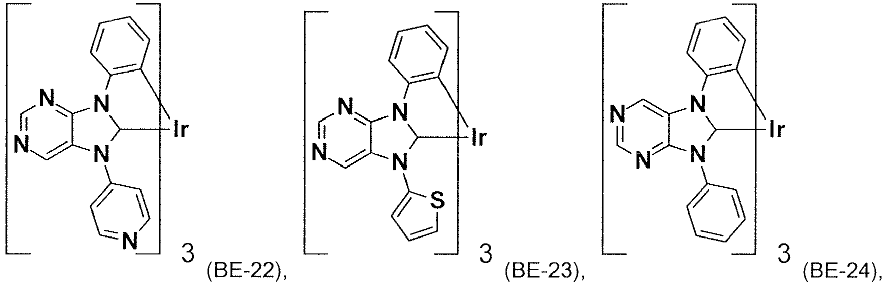 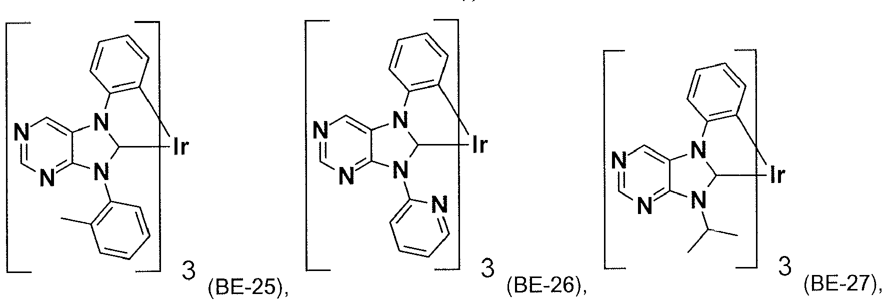 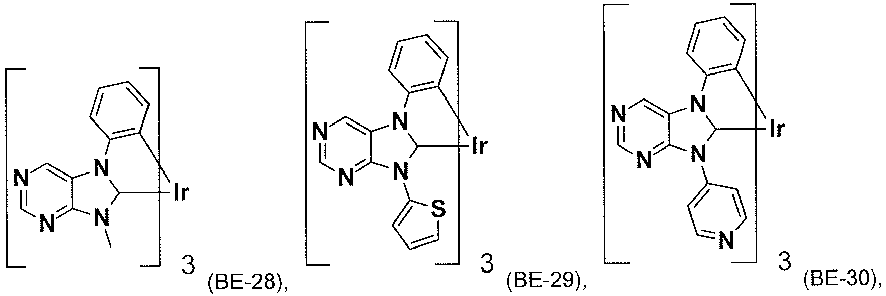 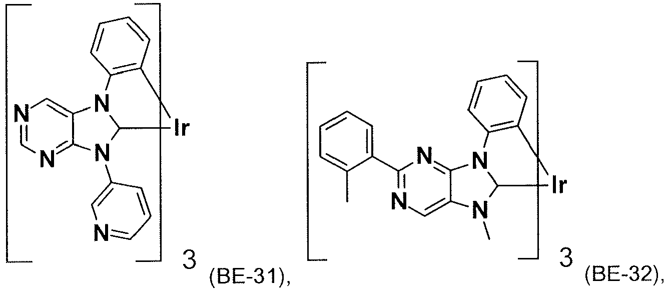 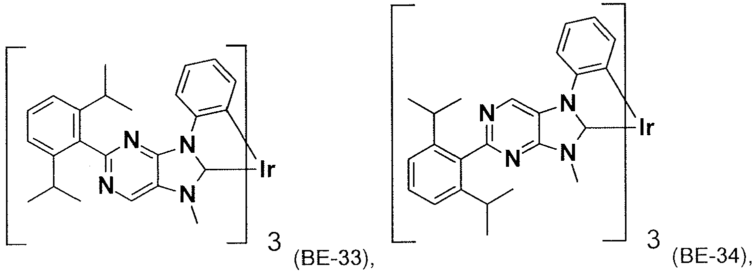 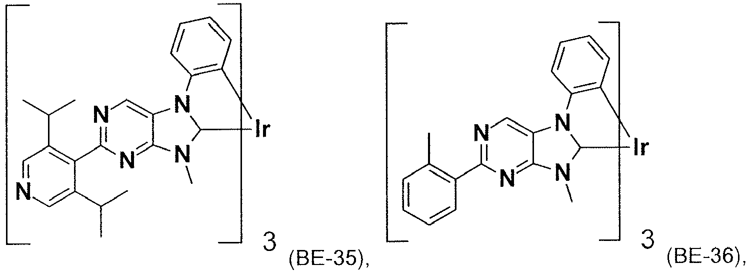 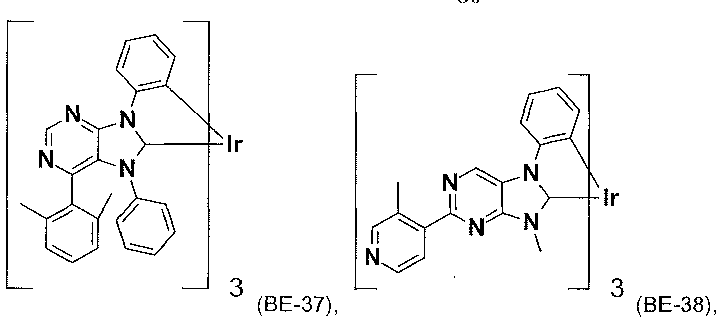 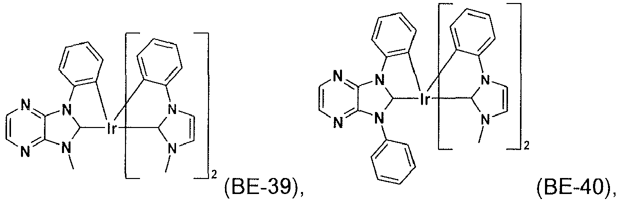 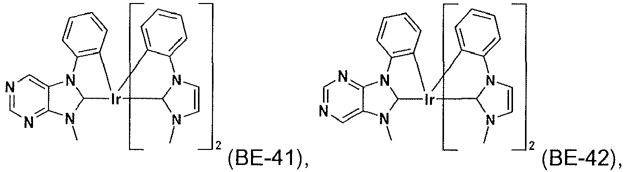 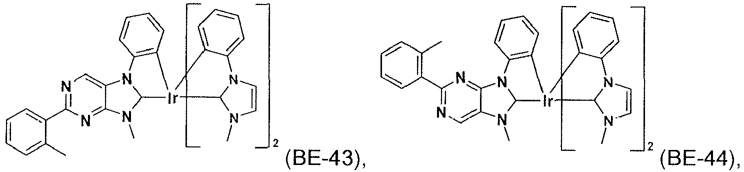 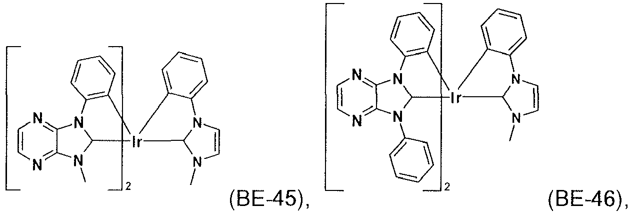 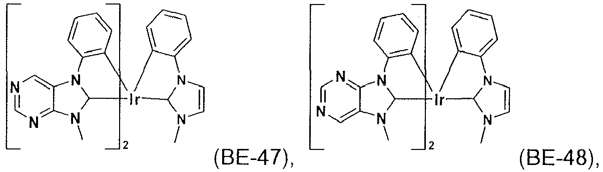 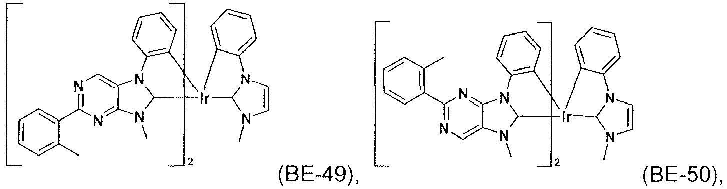 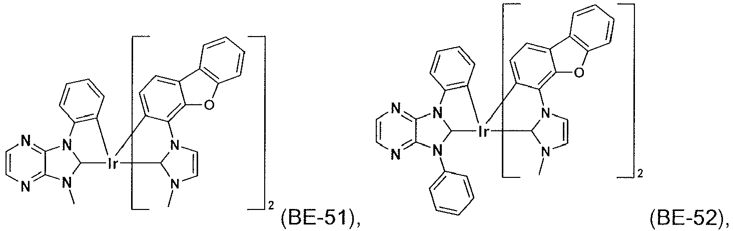 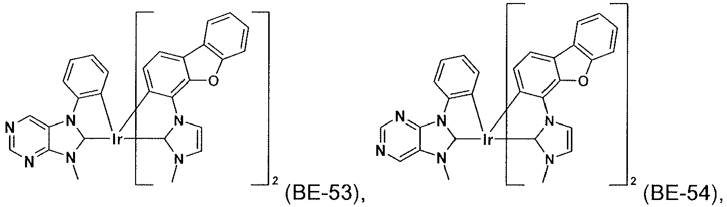 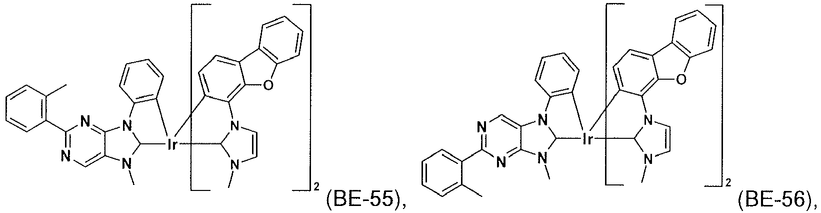 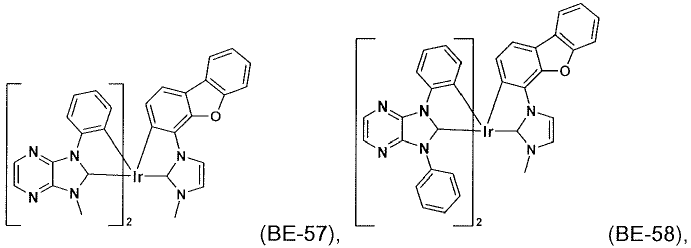 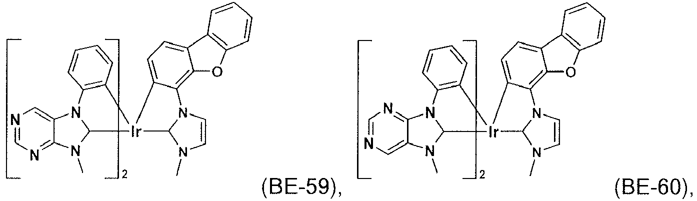 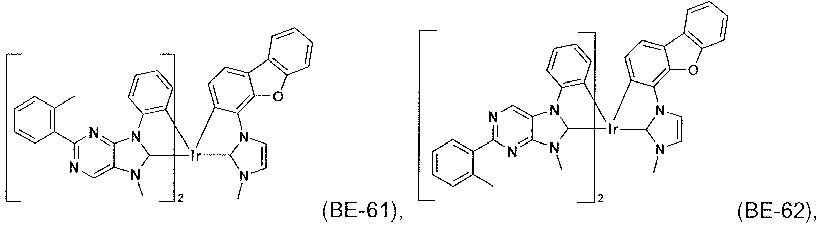 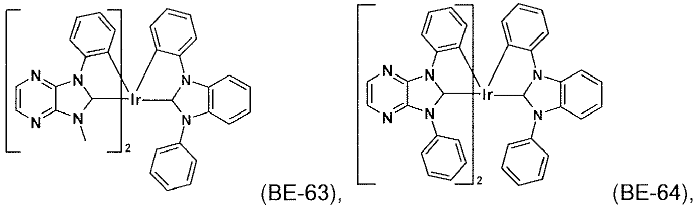 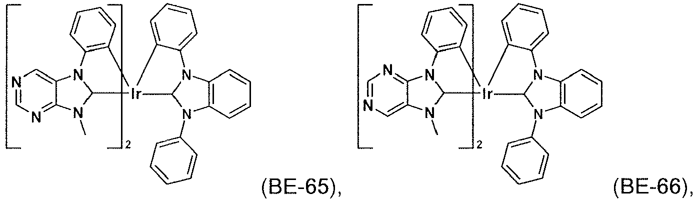 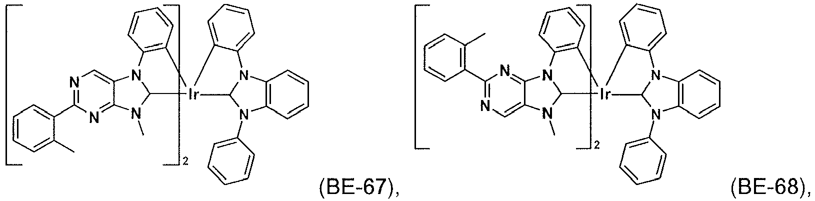 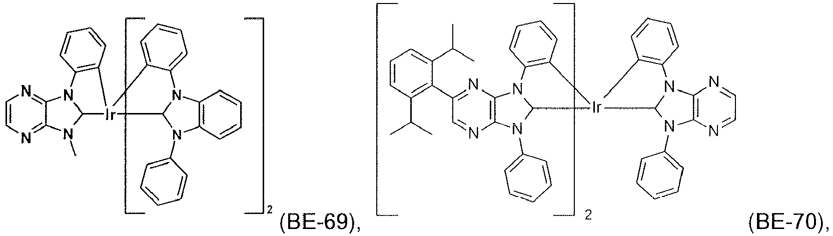 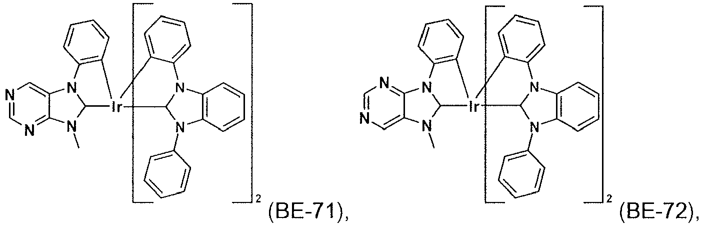 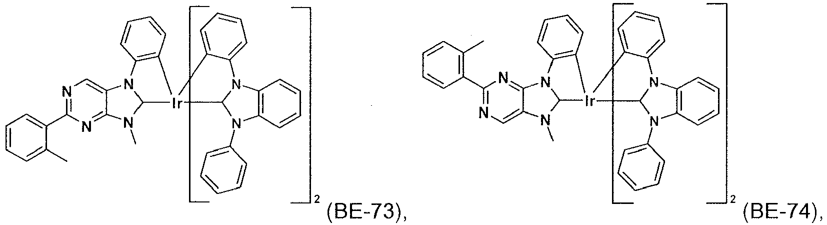 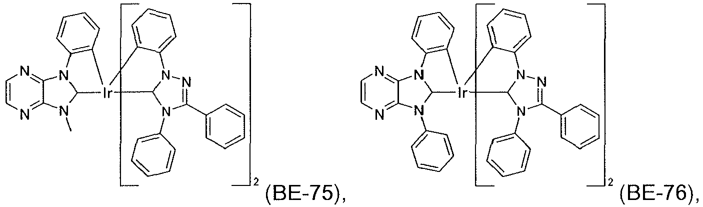 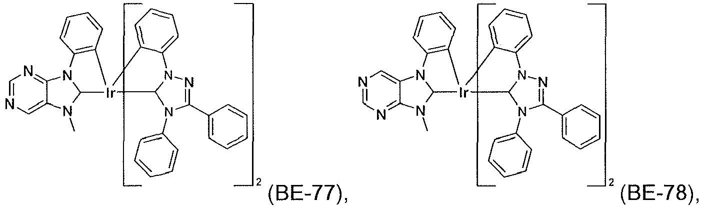 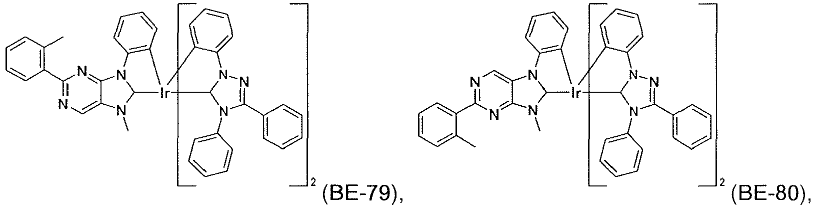 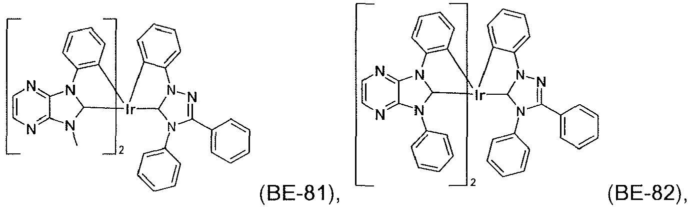 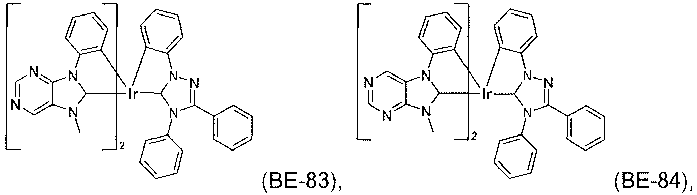 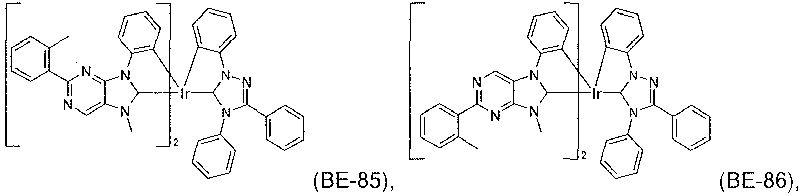 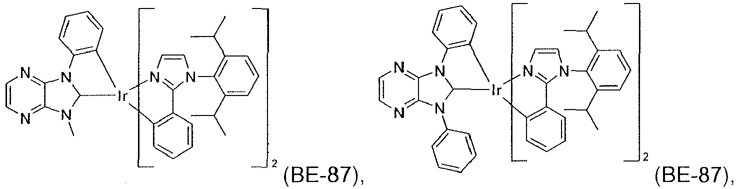 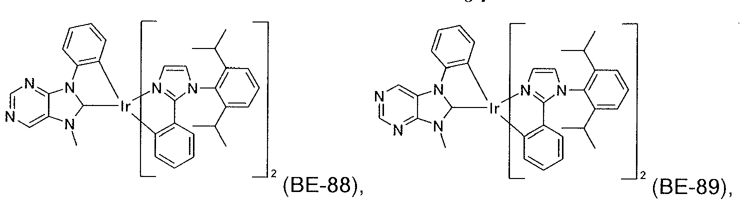 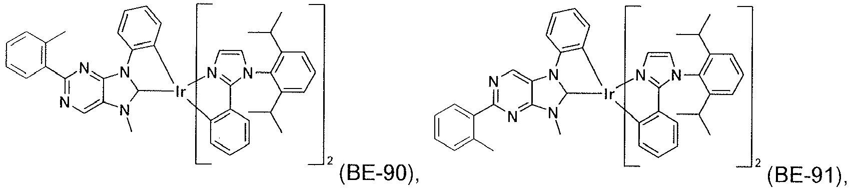 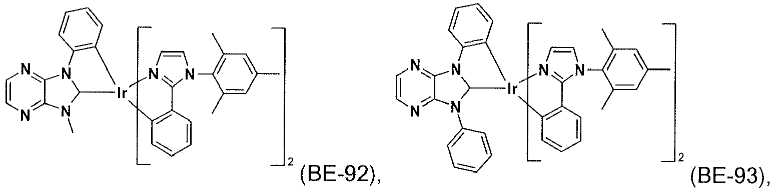 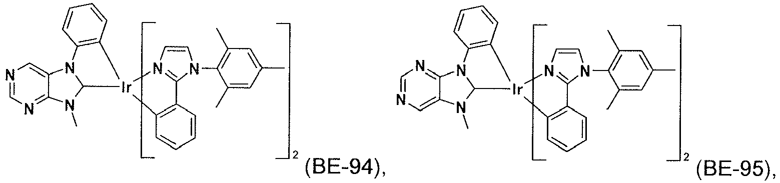 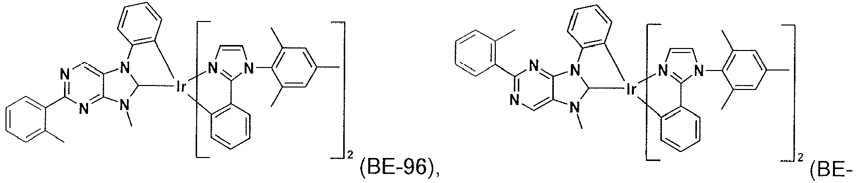 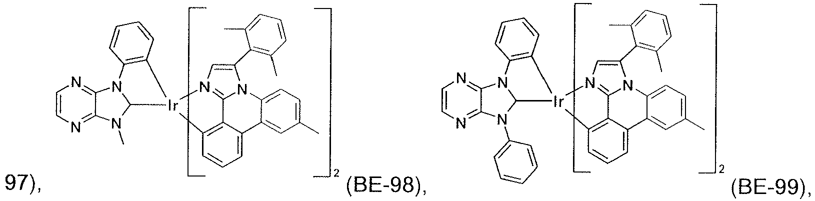 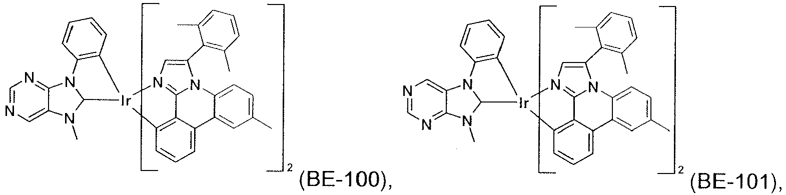 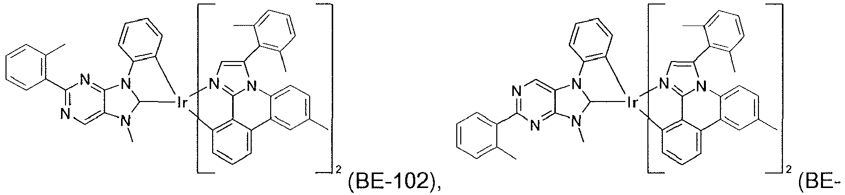 103).

Further suitable non-carbene emitter materials are mentioned below:

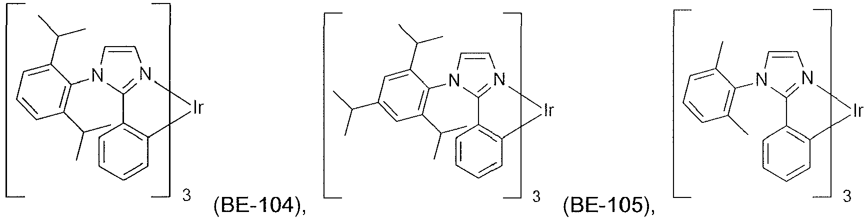

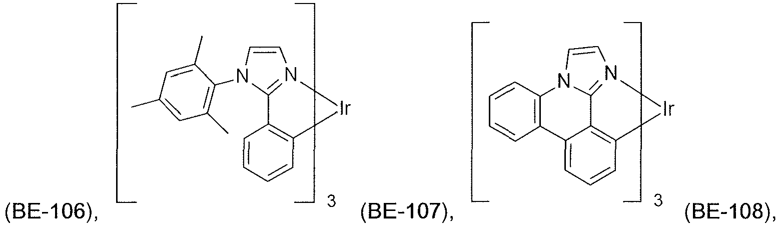

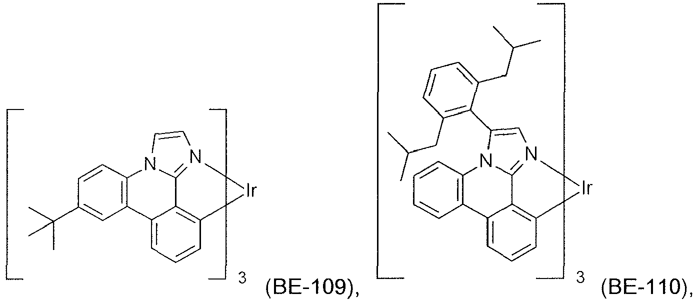

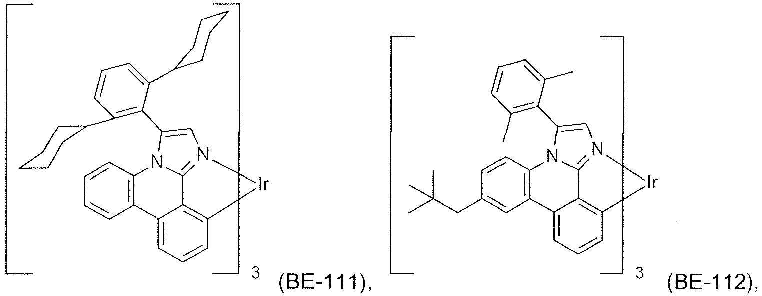

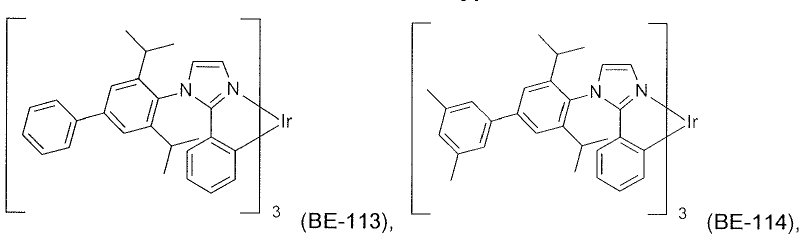

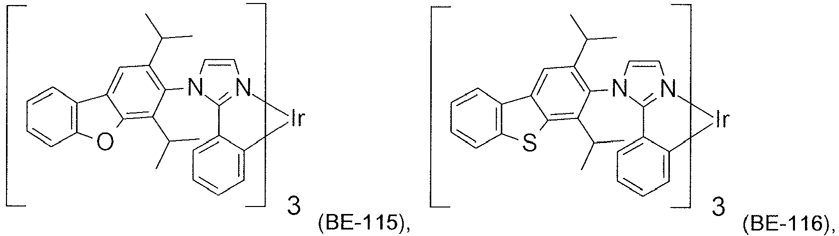

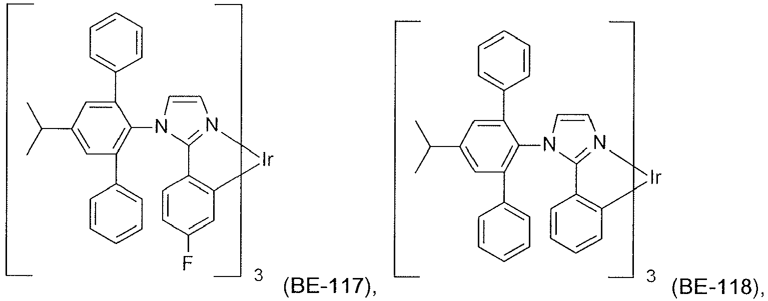

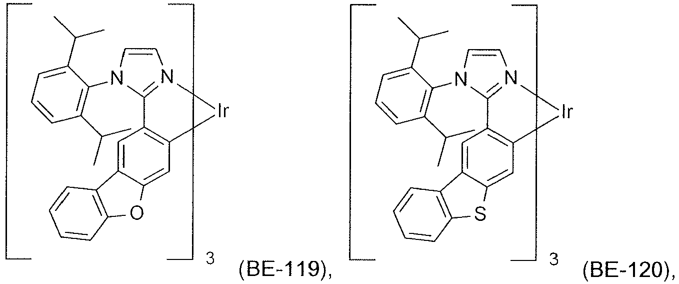

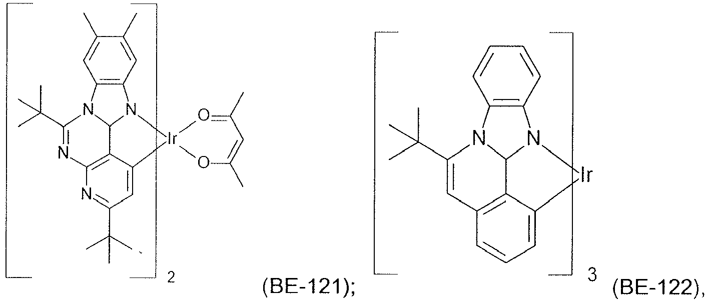

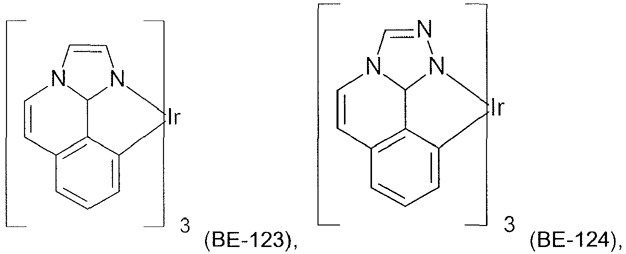

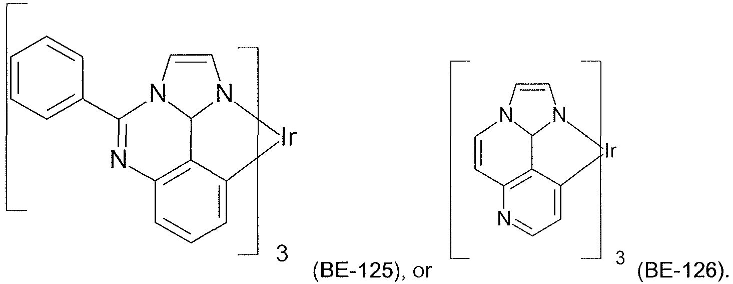

The compound of formula IX is more preferably a compound (**BE-1**), (**BE-2**), (**BE-7**), (**BE-12**), (**BE-16**), **(BE-64**), or (**BE-70**). The most preferred phosphorescent blue emitters are compounds (**BE-1**) and (**BE-12**).

The homoleptic metal-carbene complexes may be present in the form of facial or meridional isomers, preference being given to the facial isomers.

Suitable carbene complexes of formula (**IX**) and their preparation process are, for example, described in WO2011/073149.

The compounds of the present invention can also be used as host for phosphorescent green emitters. Suitable phosphorescent green emitters are, for example, specified in the following publications: WO2006014599, WO20080220265, WO2009073245, WO2010027583, WO2010028151, US20110227049, WO2011090535, WO2012/08881, WO20100056669, WO20100118029, WO20100244004, WO2011109042, WO2012166608, US20120292600, EP2551933A1; US6687266, US20070190359, US20070190359, US20060008670; WO2006098460, US20110210316, WO2012053627; US6921915, US20090039776; and JP2007123392.

Examples of suitable phosphorescent green emitters are shown below:

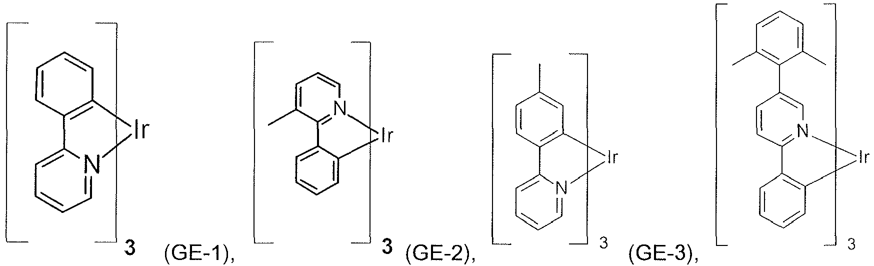

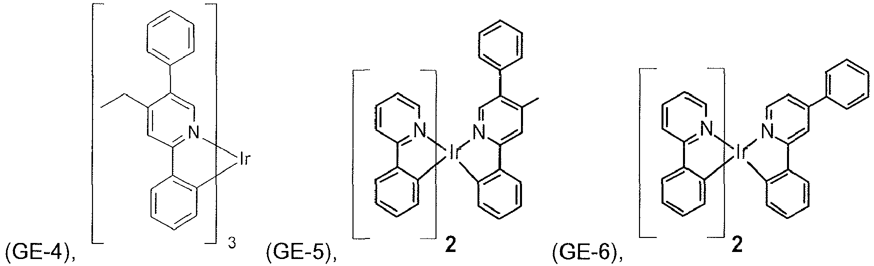

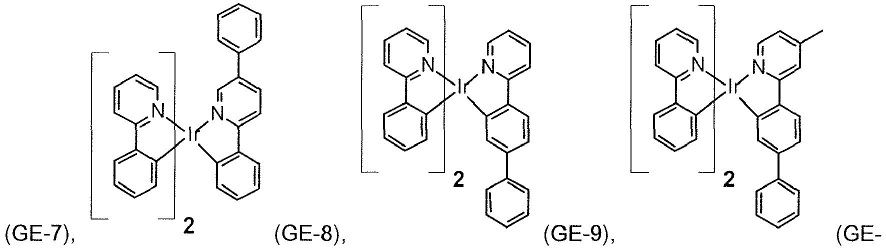

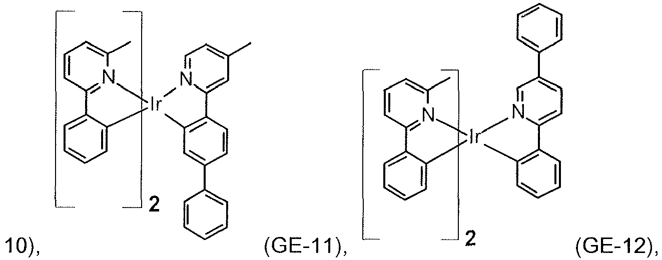

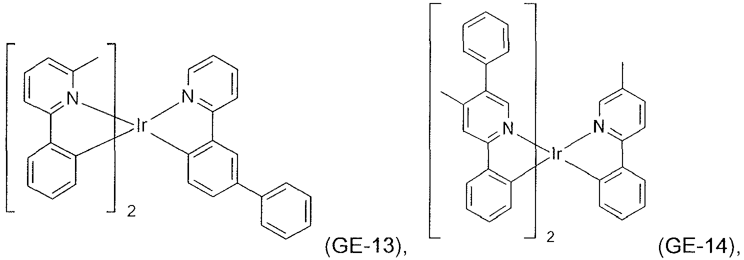

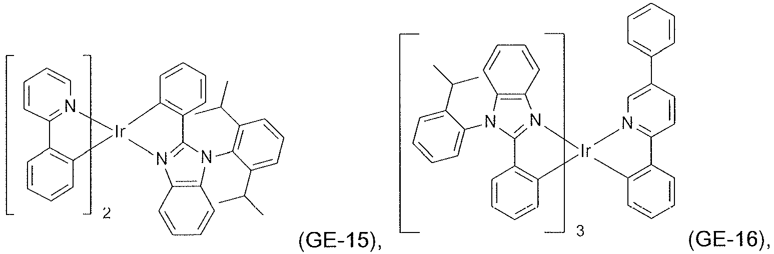

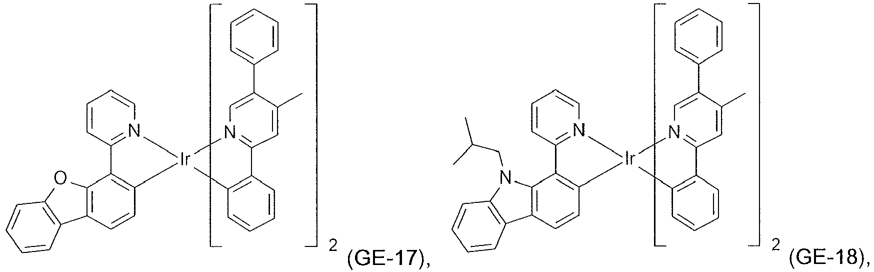

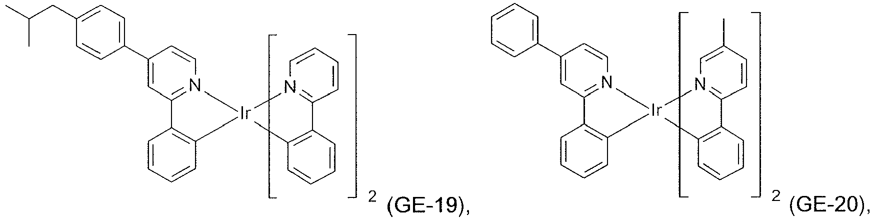

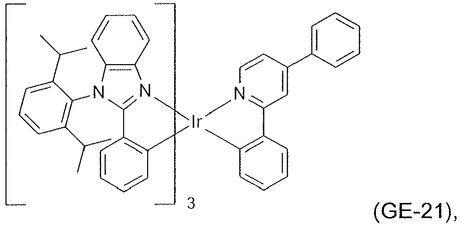

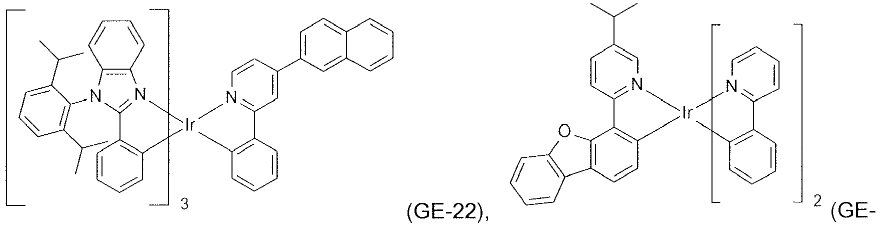

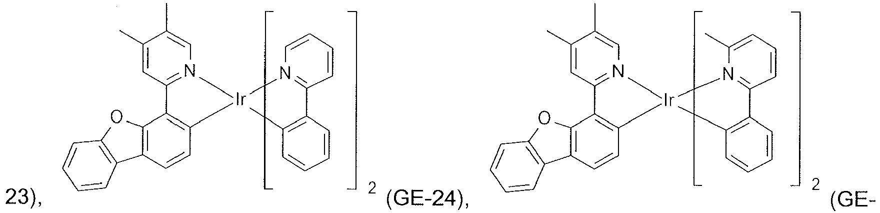

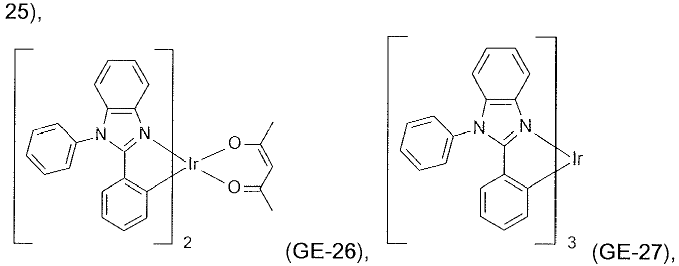

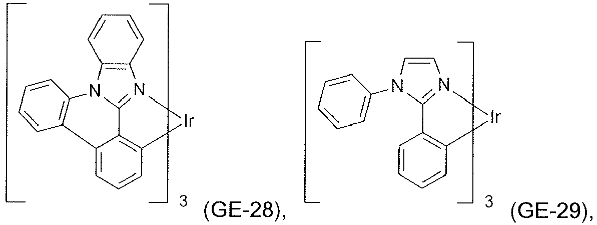

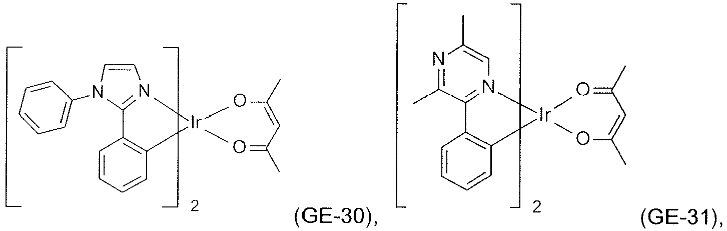

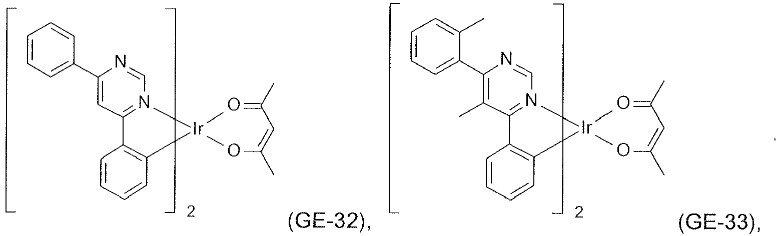

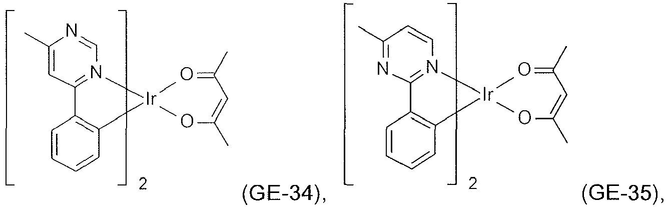

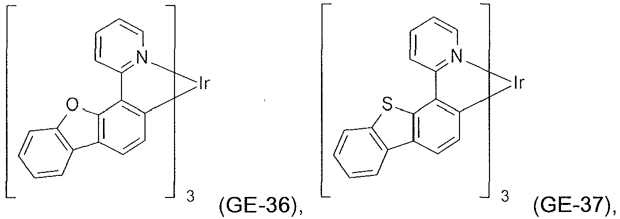

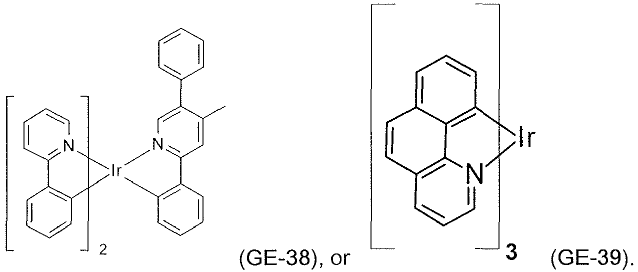

### Host (matrix) materials

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In another preferred embodiment of the present invention, at least one compound of the formula (Ia) or (Ib), especially a compound of the formula (**Ib-2**), (**Ib-4**), (**Ia-2**) or (**Ia-4**), is used as matrix material. Examples of preferred compounds of formulae la and Ib are compounds **B-1** to **B-184**, **D-1** to **D-184**, **F-1** to **F-184**, **H-1** to **H-184** shown above. Compounds **B-1** to **B-3**, **B-12**, **F-1**, **D-180**, **D-184** and **H-184** are particularly preferred.

In a preferred embodiment, the light-emitting layer is formed from 2 to 40% by weight, preferably 5 to 35% by weight, of at least one of the aforementioned emitter materials and 60 to 98% by weight, preferably 75 to 95% by weight, of at least one of the aforementioned matrix materials - in one embodiment at least one compound of the formula (Ia) or (Ib) - where the sum total of the emitter material and of the matrix material adds up to 100% by weight.

Suitable metal complexes for use together with the compounds of the formula (Ia) or (Ib) as matrix material in OLEDs are, for example, also carbene complexes as described in WO 2005/019373 A2, WO 2006/056418 A2, WO 2005/113704, WO 2007/115970, WO 2007/115981 and WO 2008/000727.

Further suitable host materials, which may be small molecules or (co)polymers of the small molecules mentioned, are specified in the following publications: WO2007108459 (H-1 to H-37), preferably H-20 to H-22 and H-32 to H-37, most preferably H-20, H-32, H-36, H-37, WO2008035571 A1 (Host 1 to Host 6), JP2010135467 (compounds 1 to 46 and Host-1 to Host-39 and Host-43), WO2009008100 compounds No.1 to No.67, preferably No.3, No.4, No.7 to No. 12, No.55, No.59, No. 63 to No.67, more preferably No. 4, No. 8 to No. 12, No. 55, No. 59, No.64, No.65, and No. 67, WO2009008099 compounds No. 1 to No. 110, WO2008140114 compounds 1-1 to 1-50, WO2008090912 compounds OC-7 to OC-36 and the polymers of Mo-42 to Mo-51, JP2008084913 H-1 to H-70, WO2007077810 compounds 1 to 44, preferably 1, 2, 4-6, 8, 19-22, 26, 28-30, 32, 36, 39-44, WO201001830 the polymers of monomers 1-1 to 1-9, preferably of 1-3, 1-7, and 1-9, WO2008029729 the (polymers of) compounds 1-1 to 1-36, WO20100443342 HS-1 to HS-101 and BH-1 to BH-17, preferably BH-1 to BH-17, JP2009182298 the (co)polymers based on the monomers 1 to 75, JP2009170764, JP2009135183 the (co)polymers based on the monomers 1-14, WO2009063757 preferably the (co)polymers based on the monomers 1-1 to 1-26, WO2008146838 the compounds a-1 to a-43 and 1-1 to 1-46, JP2008207520 the (co)polymers based on the monomers 1-1 to 1-26, JP2008066569 the (co)polymers based on the monomers 1-1 to 1-16, WO2008029652 the (co)polymers based on the monomers 1-1 to 1-52, WO2007114244 the (co)polymers based on the monomers 1-1 to 1-18, JP2010040830 the compounds HA-1 to HA-20, HB-1 to HB-16, HC-1 to HC-23 and the (co)polymers based on the monomers HD-1 to HD-12, JP2009021336, WO2010090077 the compounds 1 to 55, WO2010079678 the compounds H1 to H42, WO2010067746, WO2010044342 the compounds HS-1 to HS-101 and Poly-1 to Poly-4, JP2010114180 the compounds PH-1 to PH-36, US2009284138 the compounds 1 to 111 and H1 to H71, WO2008072596 the compounds 1 to 45, JP2010021336 the compounds H-1 to H-38, preferably H-1, WO2010004877 the compounds H-1 to H-60, JP2009267255 the compounds 1-1 to 1-105, WO2009104488 the compounds 1-1 to 1-38, WO2009086028, US2009153034, US2009134784, WO2009084413 the compounds 2-1 to 2-56, JP2009114369 the compounds 2-1 to 2-40, JP2009114370 the compounds 1 to 67, WO2009060742 the compounds 2-1 to 2-56, WO2009060757 the compounds 1-1 to 1-76, WO2009060780 the compounds 1-1 to 1-70, WO2009060779 the compounds 1-1 to 1-42, WO2008156105 the compounds 1 to 54, JP2009059767 the compounds 1 to 20, JP2008074939 the compounds 1 to 256, JP2008021687 the compounds 1 to 50, WO2007119816 the compounds 1 to 37, WO2010087222 the compounds H-1 to H-31, WO2010095564 the compounds HOST-1 to HOST-61, WO2007108362, WO2009003898, WO2009003919, WO2010040777, US2007224446, WO06128800, WO2012014621, WO2012105310, WO2012/130709 and WO2014/009317 and WO2014/044722 and WO2014/072320 (in particular page 25 to 29 of WO2014/072320).

The above-mentioned small molecules are more preferred than the above-mentioned (co)polymers of the small molecules.

Further suitable second host materials, are described in WO2011137072 (for example, 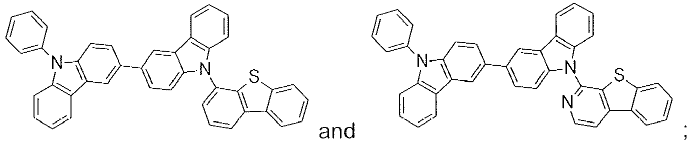 best results are achieved if said compounds are combined with 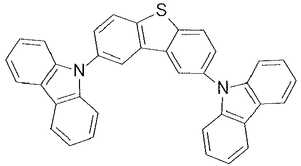 ); WO2012048266 (for example, 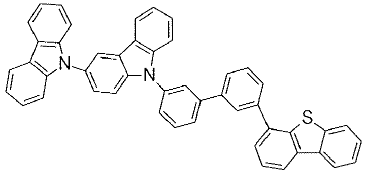 and 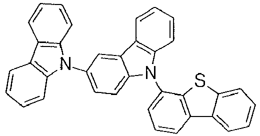 ); WO2012162325 (for example, 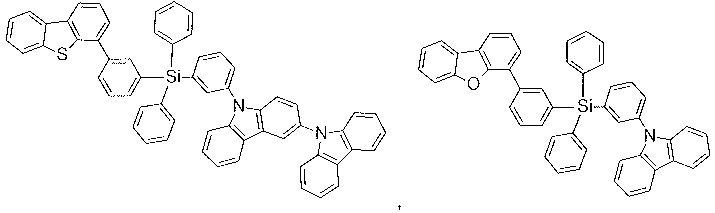 and 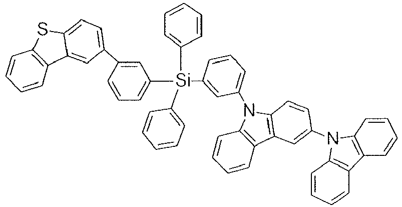 ); and EP2551932 (for example, 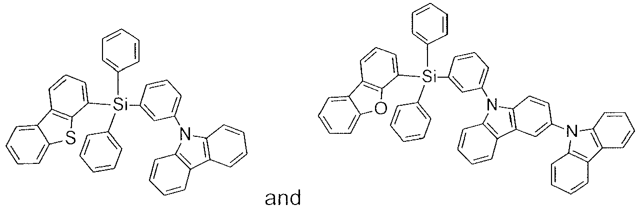 ).

In a particularly preferred embodiment, one or more compounds of the general formula (X) specified hereinafter are used as second host material. 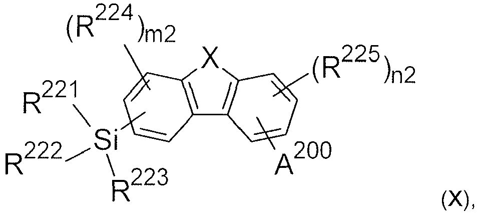 wherein
X is NR, S, O or PR;
R is aryl, heteroaryl, alkyl, cycloalkyl, or heterocycloalkyl;
A²⁰⁰ is -NR²⁰⁶R²⁰⁷, -P(O)R²⁰⁸R²⁰⁹, -PR²¹⁰R²¹¹, -S(O)₂R²¹², -S(O)R²¹³, -SR²¹⁴, or -OR²¹⁵;
R²²¹, R²²² and R²²³ are independently of each other aryl, heteroaryl, alkyl, cycloalkyl, or heterocycloalkyl, wherein at least on of the groups R²²¹, R²²², or R²²³ is aryl, or heteroaryl; R²²⁴ and R²²⁵ are independently of each other alkyl, cycloalkyl, heterocycloalkyl, aryl, heteroaryl, a group A²⁰⁰, or a group having donor, or acceptor characteristics;
n2 and m2 are independently of each other 0, 1, 2, or 3;
R²⁰⁶ and R²⁰⁷ form together with the nitrogen atom a cyclic residue having 3 to 10 ring atoms, which can be unsubstituted, or which can be substituted with one, or more substituents selected from alkyl, cycloalkyl, heterocycloalkyl, aryl, heteroaryl and a group having donor, or acceptor characteristics; and/or which can be annulated with one, or more further cyclic residues having 3 to 10 ring atoms, wherein the annulated residues can be unsubstituted, or can be substituted with one, or more substituents selected from alkyl, cycloalkyl, heterocycloalkyl, aryl, heteroaryl and a group having donor, or acceptor characteristics; and R²⁰⁸, R²⁰⁹, R²¹⁰, R²¹¹, R²¹², R²¹³, R²¹⁴ und R²¹⁵ are independently of each other aryl, heteroaryl, alkyl, cycloalkyl, or heterocycloalkyl. Compounds of formula X, such as, for example, 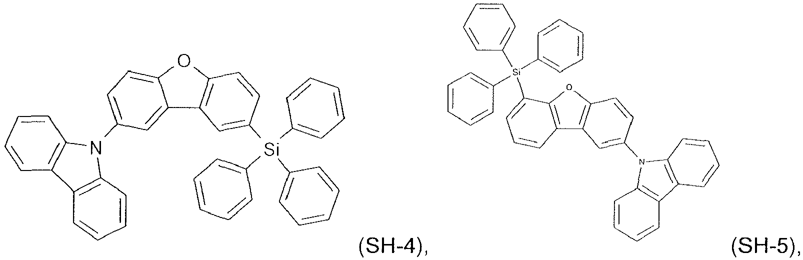 or 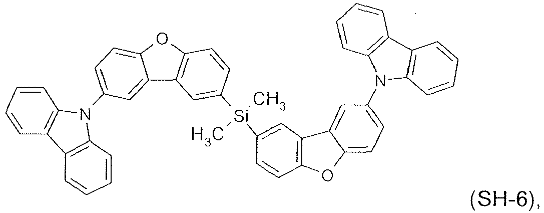 are described in WO2010079051 (in particular pages on 19 to 26 and in tables on pages 27 to 34, pages 35 to 37 and pages 42 to 43).

Additional host materials on basis of dibenzofurane are, for example, described in US2009066226, EP1885818B1, EP1970976, EP1998388 and EP2034538. Examples of particularly preferred host materials are shown below:

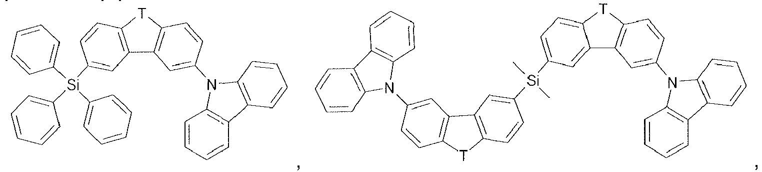

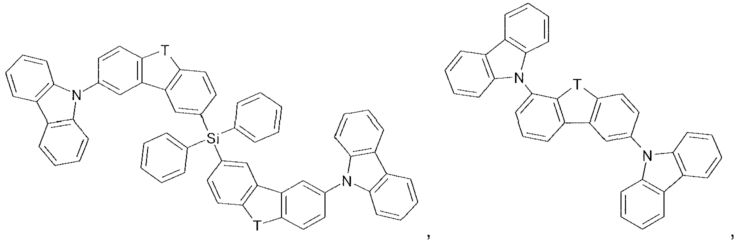

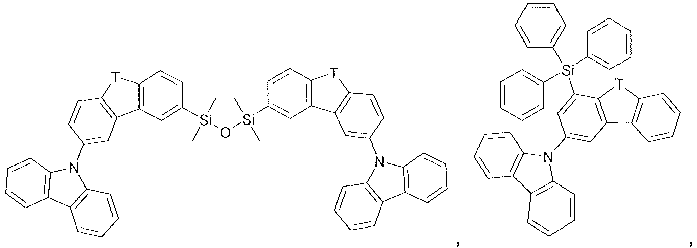

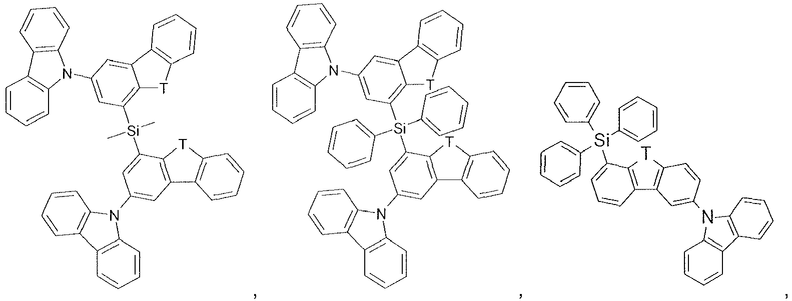

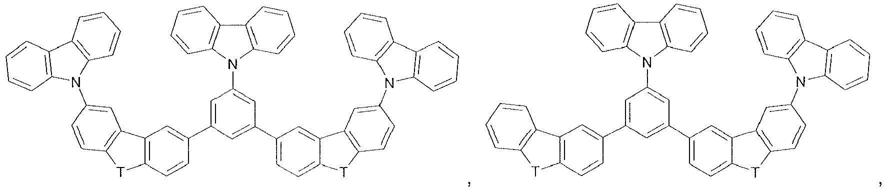

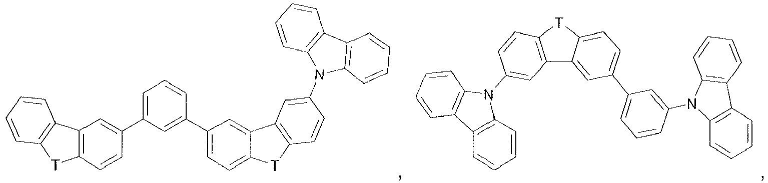

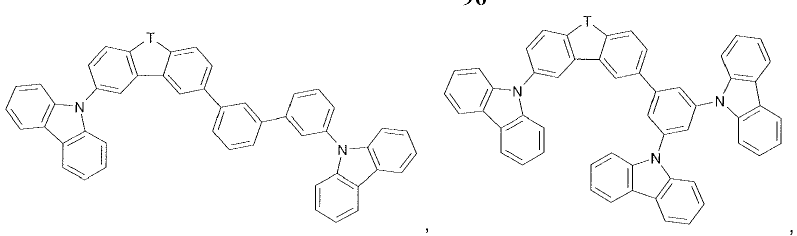

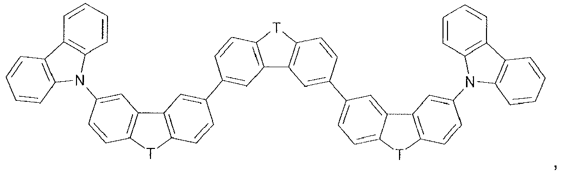

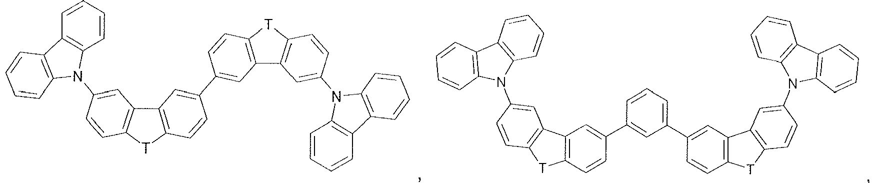

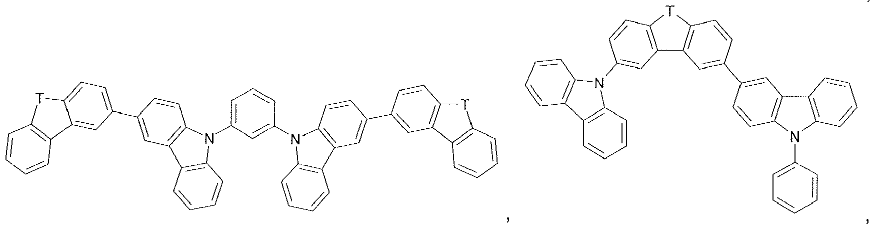

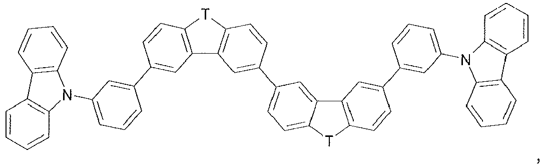

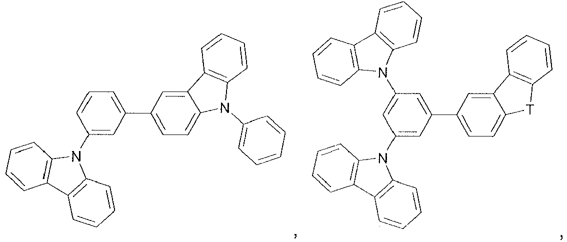

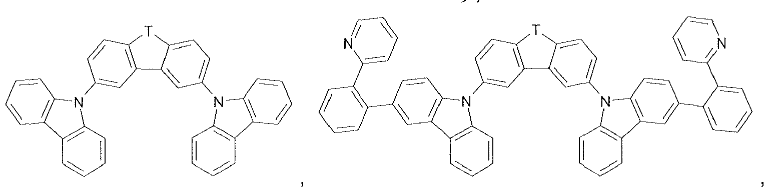

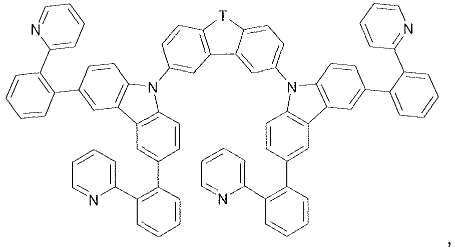

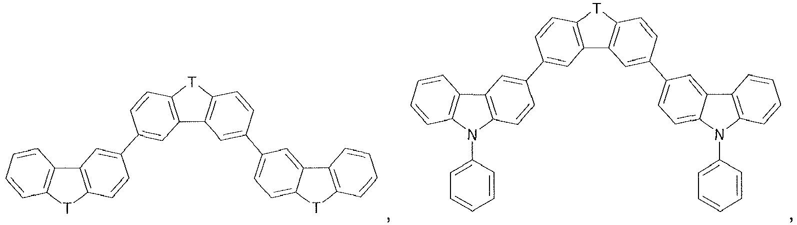

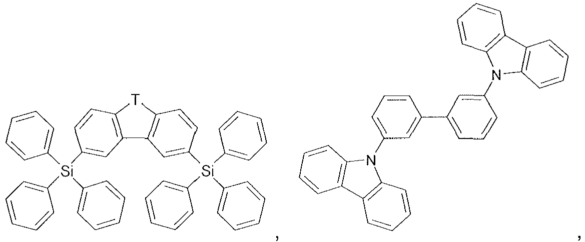

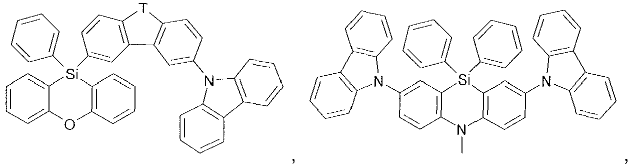

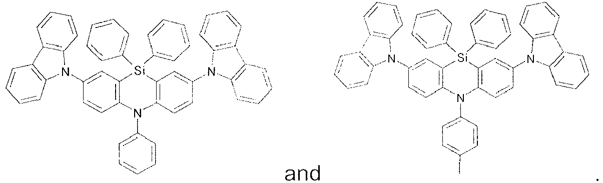

In the above-mentioned compounds T is O, or S, preferably O. If T occurs more than one time in a molecule, all groups T have the same meaning. Compounds 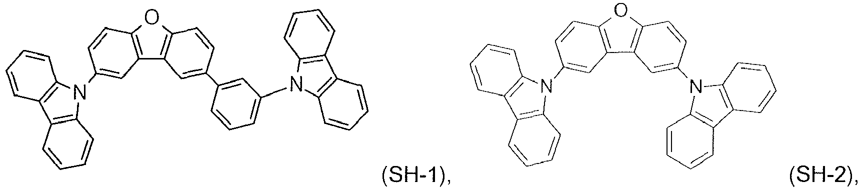 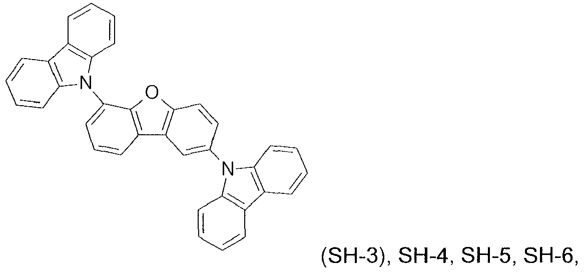 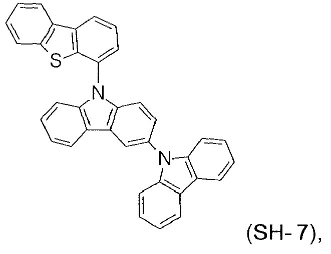 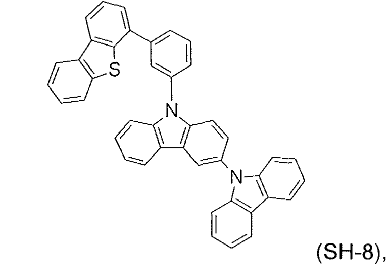 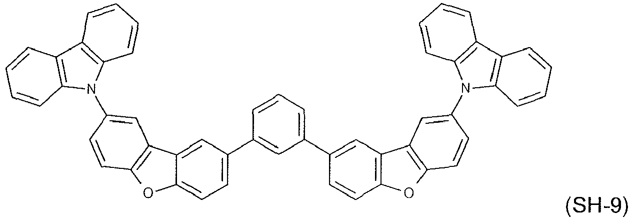 and 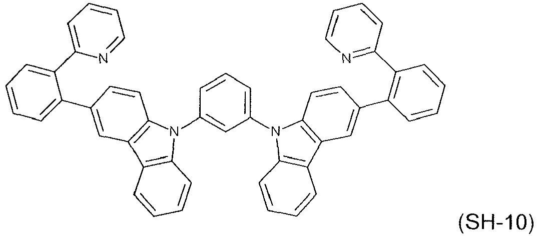 are most preferred.

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAlq), phenothiazine *S,S*-dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds (**SH-1**), (**SH-2**), (**SH-3**), **SH-4**, **SH-5**, **SH-6**, (**SH-7**), (**SH-8**), (**SH-9**) and (**SH-10**) may be used as hole/exciton blocking materials.

### Electron transport layer (g):

Electron transport layer may include a material capable of transporting electrons. Electron transport layer may be intrinsic (undoped), or doped. Doping may be used to enhance conductivity. Suitable electron-transporting materials for layer (g) of the inventive OLEDs comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula (**VIII**) below, preferably a compound of the formula (**VIIIaa**) below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below (**formula VII**). Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (**VII**) 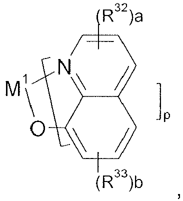 in which
R³² and R³³ are each independently F, C₁-C₈-alkyl, or C₆-C₁₄-aryl, which is optionally substituted by one or more C₁-C₈-alkyl groups, or
two R³² and/or R³³ substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula (**VII**) is 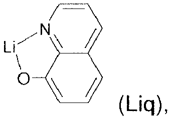 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (**VIII**), 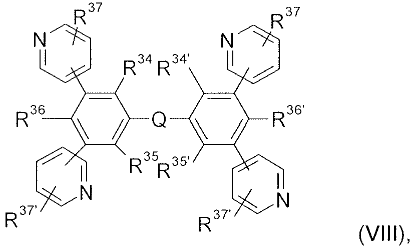 in which
R³⁴, R³⁵, R³⁶, R³⁷, R^{34'}, R^{35'}, R^{36'} and R^{37'} are each independently H, C₁-C₁₈-alkyl, C₁-C₁₈-alkyl which is substituted by E and/or interrupted by D, C₆-C₂₄-aryl, C₆-C₂₄-aryl which is substituted by G, C₂-C₂₀-heteroaryl or C₂-C₂₀-heteroaryl which is substituted by G,
Q is an arylene or heteroarylene group, each of which is optionally substituted by G;
D is -CO-; -COO-; -S-; -SO-; -SO₂-; -O-; -NR⁴⁰-; -SiR⁴⁵R⁴⁶-; -POR⁴⁷-; -CR³⁸=CR³⁹-; or -C≡C-;
E is -OR⁴⁴; -SR⁴⁴; -NR⁴⁰R⁴¹; -COR⁴³; -COOR⁴²; -CONR⁴⁰R⁴¹; -CN; or F;
G is E, C₁-C₁₈-alkyl, C₁-C₁₈-alkyl which is interrupted by D , C₁-C₁₈-perfluoroalkyl, C₁-C₁₈-alkoxy, or C₁-C₁₈-alkoxy which is substituted by E and/or interrupted by D,
in which
R³⁸ and R³⁹ are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R⁴⁰ and R⁴¹ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or R⁴⁰ and R⁴¹ together form a 6-membered ring;
R⁴² and R⁴³ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁴ is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁵ and R⁴⁶ are each independently C₁-C₁₈-alkyl, C₆-C₁₈-aryl or C₆-C₁₈-aryl which is substituted by C₁-C₁₈-alkyl,
R⁴⁷ is C₁-C₁₈-alkyl, C₆-C₁₈-aryl or C₆-C₁₈-aryl which is substituted by C₁-C₁₈-alkyl.

Preferred compounds of the formula (**VIII**) are compounds of the formula (**VIII**) 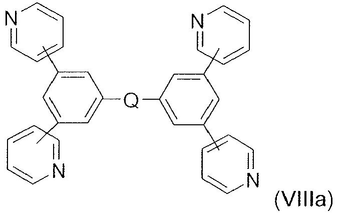 in which Q is: 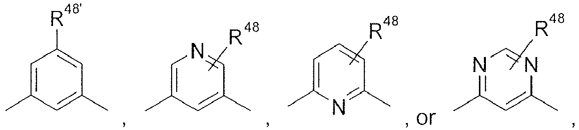 R48 is H or C₁-C₁₈-alkyl and R^{48'} is H, C₁-C₁₈-alkyl or

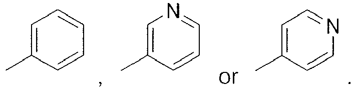

Particular preference is given to a compound of the formula

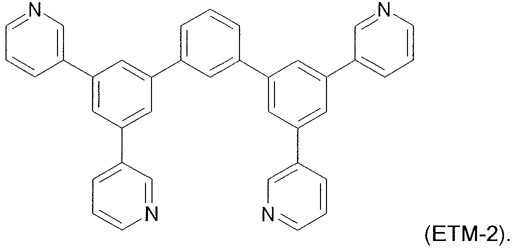

In a further, very particularly preferred embodiment, the electron-transport layer comprises a compound **Liq** and a compound **ETM-2.**

In a preferred embodiment, the electron-transport layer comprises the compound of the formula (**VII**) in an amount of 99 to 1% by weight, preferably 75 to 25% by weight, more preferably about 50% by weight, where the amount of the compounds of the formulae (**VII**) and the amount of the compounds of the formulae (**VIII**) adds up to a total of 100% by weight.

The preparation of the compounds of the formula (**VIII**) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1 to A-36** and **B-1** to **B-22** described in WO2011/157790 are preferred, wherein dibenzofuran compound 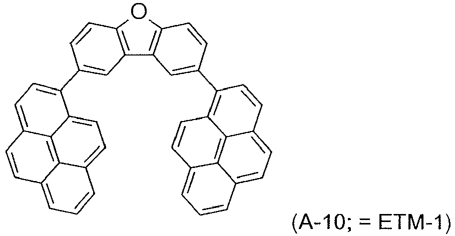 is most preferred.

In a preferred embodiment, the electron-transport layer comprises **Liq** in an amount of 99 to 1% by weight, preferably 75 to 25% by weight, more preferably about 50% by weight, where the amount of **Liq** and the amount of the dibenzofuran compound(s), especially **ETM-1**, adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 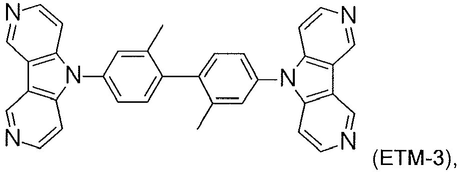 US2012/0261654 , such as, for example, a compound of formula 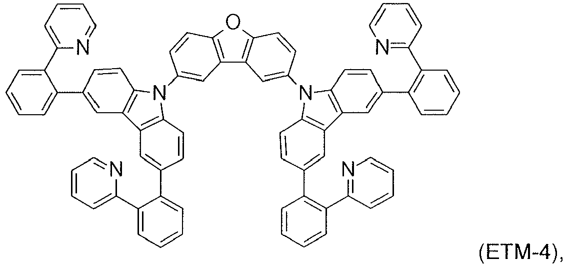 and WO2012/115034, such as for example, such as, for example, a compound of formula

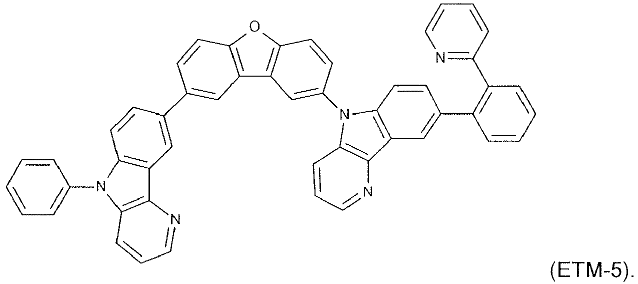

### Electron injection layer (h):

The electron injection layer may be any layer that improves the injection of electrons into an adjacent organic layer. Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds of the formula (Ia) or (Ib) in at least one layer of the OLED, preferably in the light-emitting layer (preferably as a matrix material), charge transport layer and/or in the charge/exciton blocking layer makes it possible to obtain OLEDs with high efficiency and with low use and operating voltage. Frequently, the OLEDs obtained by the use of the compounds of the formula (Ia) or (Ib) additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### Example 1

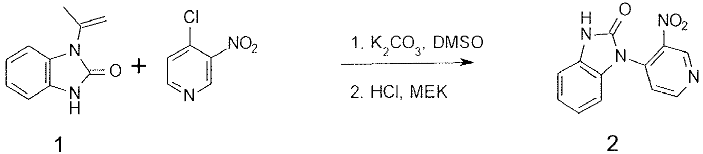

a) 15.56g of 1 (89 mmol) were combined with 4-chloro-3-nitropyridine (14.16 g, 89 mmol) and K₂CO₃ (12.34 g, 89 mmol) in DMSO (90 mL) and the resulting mixture was heated at 115 °C for 1.5 hours. The reaction was allowed to cool to room temperature and diluted with (200 mL). The mixture was then extracted with dichloromethane (3 x 200 mL) and the organic phases combined and back extracted with water (2 x 200 mL). The organic phase was then dried over anhydrous MgSO₄ and solvent evaporated to give the crude product as a brown waxy residue. ¹H NMR (400 MHz, DMSO d₆) 9.40 ppm (s, 1H), 9.13 ppm (1H, d, J = 5.3 Hz), 8.06 ppm (1H, d, J = 5.3 Hz), 7.24-7.37 ppm (4H, m), 5.56, 1H, br d, J = 1.18, Hz), 5.32 ppm (1H, br s), 2.18 ppm (3H, s). The crude reaction product was used without further purification. The product was suspended in Methyl ethyl ketone (MEK, 200 mL) and HCI (37 %, 15.5 mL) was added in one portion. The resulting reaction mixture was allowed to stir at 90 °C for 6 hours. The reaction was then cooled to room temperature nad the desired product 2 was filtered off and washed with MEK and dried. 20.67 g (90.3% over 2 steps) of 2 was collected. ¹H NMR (400 MHz, DMSO d₆) 11.53 ppm (1H, br s), 9.30 ppm (1H, s), 9.03 ppm (1H, d, J = 5.3 Hz), 7.97 ppm (1H, d, J = 5.3 ppm), 7.08 - 7.26 ppm (m). 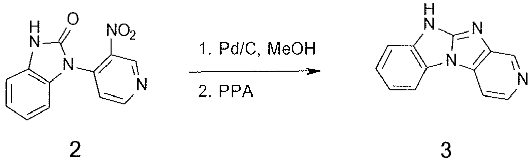
b) Compound 2 (20.67 g, 71 mmol) was dissolved in MeOH (150 mL) and hydrogenated over Pd/C (10%) under 3 bar H₂ at room temperature. After 6 hours, the reaction was complete and the catalyst was filtered and solvent evaporated to give the desired product as a colourless solid. ¹H NMR (400 MHz, DMSO d₆) 11.48 ppm (1H, s), 8.50 ppm, (1H, s), 8.14 ppm (1H, d, J = 5.32 Hz), 7.84 ppm (1H, d, J = 5.47 Hz), 6.98-7.15 ppm (3H, m), 6.78 ppm (1H, d, J = 7.77 Hz). 6.01 ppm (2H, br s). The crude material was used without further purification. It was taken up in polyphosphoric acid (PPA) and heated at 220 °C overnight. The reaction was cooled and poured into ice cold 5M NaOH. After complete dissolution of the PPA mixture, the pH of the aqueous solution was adjusted to 6 using 0.5M KHSO₄. The solid brown crude product was then filtered off and washed well with water. Purification was carried out by chromatography on silica using 5 - 15% MeOH in CHCl₃ as eluent. The product 3 isolated was then suspended in acetone and filtered to give 9 g (65 % yield) of the desired product as an off white solid. ¹H NMR (300 MHz, DMSO d₆) 8.82 ppm (1H, s), 8.38 ppm (1H, d, J = 5.35 Hz), 8.13-8.16 pp, (2H, m), 7.47-7.50 (1H, m), 7.26-7.39 (2H, m). 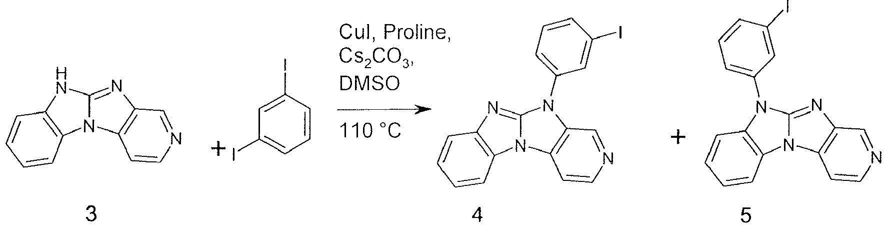
c) 6.3 g (30.0 mmol) of 3 was combined with 1,3-diiodobenzene (25 g, 76 mmol), Cul (0.58 g, 3 mmol), L-proline (0.70 g, 6 mmol) and Cs₂CO₃ (19.8 g, 60 mmol) in DMSO (200 mL). The reaction mixture was degassed with N₂ for 20 minutes. The mixture was then stirred at an oil bath temperature of 110 °C overnight. The reaction mixture was cooled to room temperature and diluted with water (300 mL). The insoluble precipitate was filtered over a pad of celite washing well with water. Excess diiodobenzene was removed by washing the filter cake with diethyl ether. The product was formed as a 2:1 mixture of regioisomers and was collected by washing through the filtercake with CHCl₃. This solution was then washed with 10 % aqueous ammonia (3 x 100 mL), dried over MgSO₄ and solvent evaporated. The crude material was then by chromatography on silica using ethyl acetate as eluent to remove the first isomer followed by 2 % MeOH in CHCl₃ to elute the second isomer. The minor isomer 4 was isolated first (2 g, 16.1 %) ¹H NMR (300 MHz, CDCl₃) 8.92 ppm (1H, s), 8.67 ppm (1H, d, J = 5.33 Hz), 8.25 ppm (1H, t, J = 1.85 Hz), 7.9-7.94 ppm (1H, m), 7.75-7.87 ppm (4H, m)7.34-7.47 ppm, (3H, m). 4.45 g (35.6 %) of the major isomer 5 eluted second. ¹H NMR (300 MHz, CDCl₃) 9.11 ppm (1H, d, J = 0.83 Hz) 8.55 ppm (1H, d, J = 5.47Hz), 8.19 ppm (1H, t, J = 1.83 Hz), 7.78-7.89 ppm (4H, m), 7.59-7.59 ppm (1H, m), 7.39-7.46 (3H, m). Products were confirmed by LCMS m/z = 411 (M+1). 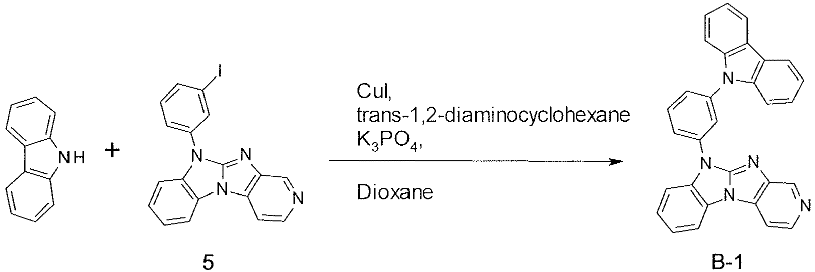
d) 0.9 g (2.2 mmol) of 5 was combined with carbazole (0.44 g, 2.6mmol) CuI (42 mg, 0.22 mmol), trans-1,2-diaminocyclohexane (0.12 mL, 1 mmol) and K₃PO₄ (0.93 g, 0.44 mmol) in dioxane (20 mL) and the resulting mixture was degassed under N₂ for 20 minutes. The reaction was then heated at an oil bath temperature of 110 °C overnight. The reaction was cooled to room temperature and the solvent evaporated. Crude material was dissolved in CHCl₃ (100 mL) and washed with water (100 mL) and 10% aqueous NH₃ (2 x 50 mL), dried over MgSO₄ and solvent evaporated. The crude material was treated with methanol and filtered. The product was then purified by chromatography on silica using 1% MeOH in CHCl₃ as eluent. The desired product B-1 was isolated (0.5 g, 49 % yield) in 99.2 % purity by HPLC. Reprecipitation from CHCl₃/MeOH increased the purity to 99.5 % by HPLC. ¹H NMR (300 MHz, CDCl₃) 9.12 1H, s) 8.56 ppm (1H, d, J = 5.42 Hz), 7.32-8.19 ppm (17H, m). Product confirmed by LCMS, m/z found 450 (M+1).

### Example 2

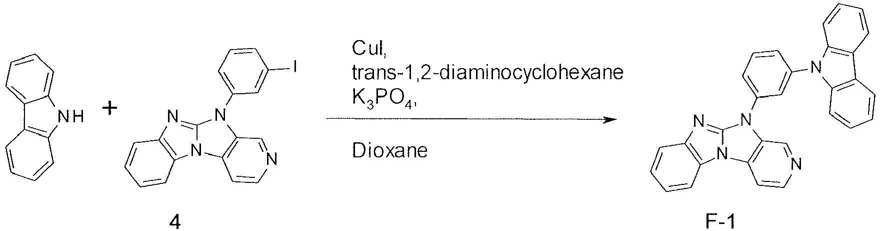

1.0 g (2.4 mmol) of 4 was combined with carbazole (0.49 g, 2.9 mmol) CuI (46 mg, 0.24 mmol), trans-1,2-diaminocyclohexane (55 · L, 0.48 mmol) and K₃PO₄ (1 g, 4.8 mmol) in dioxane (20 mL) and the resulting mixture was degassed under N₂ for 20 minutes. The reaction was then heated at an oil bath temperature of 110 °C overnight. The reaction was cooled to room temperature and the solvent evaporated. Crude material was dissolved in CHCl₃ (100 mL) and washed with water (100 mL) and 10% aqueous NH₃ (2 x 50 mL), dried over MgSO₄ and solvent evaporated. The product was purified by filtering through a plug of silica using EtOAc as eluant. Further purification was carried out by precipitation from CHCl₃ /toluene to give F-1 (0.9 g, 82 % yield) in 99.4 % purity by HPLC. ¹H NMR (400 MHz, CDCl₃) 9.4 ppm (1H, s), 8.58 ppm (1H, d, J = 5.26 Hz) 8.19 ppm (2H, m), 8.07 ppm (1H, m), 7.87 - 7.94 (2H, m) 7.80 (2H, m), 7.73 - 7.76 ppm (1H, m), 7.64ppm (1H, s) 7.62 ppm (1H, s), 7.44-7.51 ppm (3H, m), 7.38-7.41 ppm (3H, m). Product confirmed by LCMS, m/z found 450 (M+1).

### Example 3

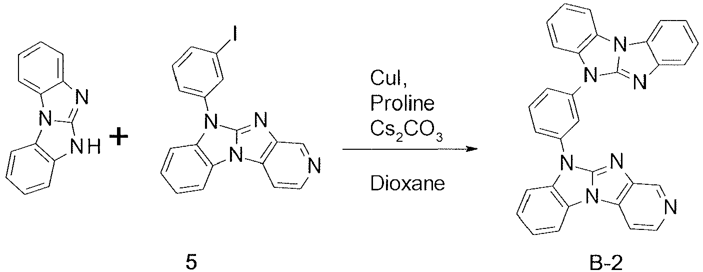

2.0 g (4.9 mmol) of 5 was combined with 6H-benzimidazolo[1,2-a]benzimidazole (1.21 g, 5.8 mmol) CuI (93 mg, 0.0.49 mmol), L-proline (0.11 g, 0.97 mmol) and Cs₂CO₃ (3.2 g, 9.7 mmol) in DMSO (20 mL) and the resulting mixture was degassed under N₂ for 20 minutes. The reaction was then heated at an oil bath temperature of 110 °C for 3 days. The reaction was cooled to room temperature and diluted with water. The crude material was filtered through a pad of celite washing well with water and then methanol. The product was collected by washing through with CHCl₃. Crude material was dissolved in CHCl₃ (100 mL) and washed with water (100 mL) and 10% aqueous NH₃ (2 x 50 mL), dried over MgSO₄ and solvent evaporated. The product was purified by filtering through a plug of silica using EtOAc as eluant. Further purification was carried out by precipitation from CHCl₃ /toluene to give **B-2** (0.9 g, 38 % yield) in 99.4 % purity by HPLC. ¹H NMR (400 MHz, CDCl₃) 9.13 ppm (1H, d, J = 0.73 Hz), 8.58 ppm, (1H, d, J = 5.39 Hz) 8.54 ppm (1H, t, 1.98 Hz), 7.99-8.07 ppm (2H, m), 7.87 - 7.94 ppm (5H, m),7.80-7.83 ppm (2H, m), 7.28-7.51 (7H, m). Product confirmed by LCMS m/z = 490 (M+1).

### Example 4

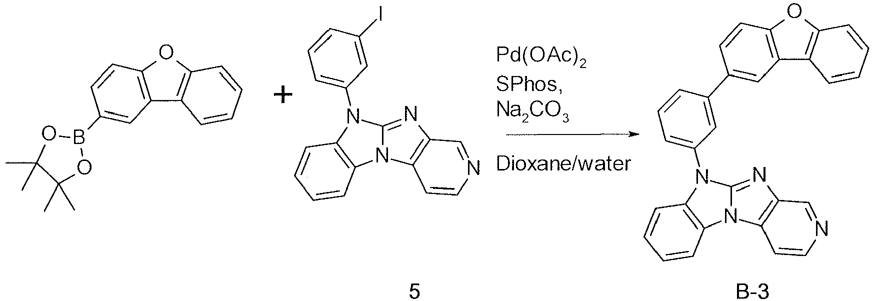

2 g (4.9 mmol) 5 was combined with 8-(4,4,5,5-tetramethyl-1,3,2-dioxaborolan-2-yl)dibenzofuran (1.72 g, 5.8 mmol) Pd(OAc)₂ (11 mg, 49 mmol), 2-dicyclohexylphosphino-2,6-dimethoxybiphenyl (SPhos, 80 mg, 0.19 mmol) and 2M aqueous Na₂CO₃ (4.9 mL, 9.7 mmol) in dioxane/water (50 mL, 4:1) and degassed under N₂ for 20 minutes. The reaction was then heated at an oil bath temperature of 110°C for 3 hours. The reaction was cooled to room temperature and the dioxane evaporated under reduced pressure. The aqueous residue was diluted with water and the mixture filtered through a pad of celite. The fil-tercake was washed with methanol and then acetone. The product B-3 was finally collected by washing through with CHCl₃. The crude material was purified by chromatography on silica using 5 - 15 % THF in DCM as eluent to give B-3 as a colourless solid (1.15 g, 52.4 % yield). ¹H NMR (300 MHz, CDCl₃) 9.13 ppm (1H, d, J = 0.89 Hz), 8.57 ppm (1H, d, J = 5.37 Hz), 8.23 ppm (1H, d, J = 1.43 Hz), 8.12 ppm (1H, t, J = 1.65 Hz), 8.01-8.04 (1H, m), 7.37-7.92 ppm (13H, m). Product confirmed by LCMS, m/z = 451 (M+1).

### Example 5

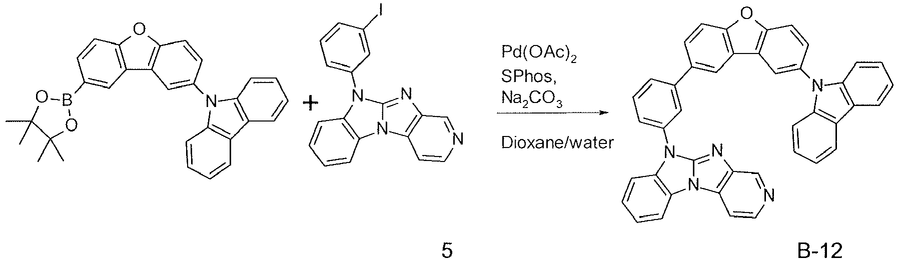

1.3 g (3.2 mmol) 5 was combined with 9-[8-(4,4,5,5-tetramethyl-1,3,2-dioxaborolan-2-yl)dibenzofuran-2-yl]carbazole (1.75 g, 3.8 mmol) Pd(OAc)₂ (7 mg, 32 mmol), 2-dicyclohexylphosphino-2,6-dimethoxybiphenyl (SPhos, 52 mg, 0.13 mmol) and 2M aqueous Na2CO₃ (3.2 mL, 6.3 mmol) in dioxane/water (100 mL, 4:1) and degassed under N₂ for 20 minutes. The reaction was then heated at an oil bath temperature of 110°C for 3 hours. The reaction was cooled to room temperature and the dioxane evaporated under reduced pressure. The aqueous residue was diluted with water and the product extracted with CHCl₃ (100 mL). The organic phase was washed with brine (50 mL) and dried over MgSO₄ and the solvent evaporated under reduced pressure. Product B-12 was purified by chromatography on silica using 5 - 15 % THF in DCM as eluant to give 1 g (51 %) of a colourless solid. ¹H NMR (400 MHz, CDCl₃) 9.09 ppm (1H, d, J = 0.71 Hz), 8.55 ppm (1H, d, J = 5.38 Hz), 8.18-8.22 ppm (4H, m), 8.12 ppm (1H, t, J = 1.77 Hz), 7.62-7.88 ppm (10H, m), 7.40-7.46 ppm (6H, m), 7.30-7.34 ppm (2H, m). Product confirmed by LCMS, m/z = 616 (M+1).

### Example 6

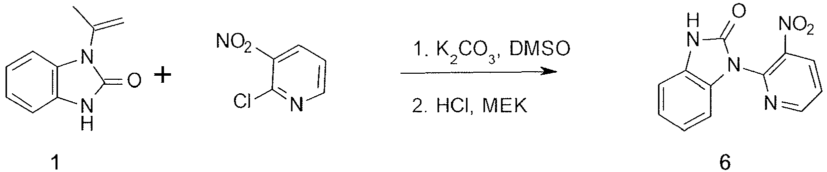

a) 10 g of 1 (57 mmol) were combined with 2-chloro-3-nitropyridine (8.19 g, 52 mmol) and K₂CO₃ (7.14 g, 52 mmol) in DMSO (100 mL) and the resulting mixture was heated at 110 °C for 4 hours. The reaction was allowed to cool to room temperature and diluted with water (200 mL). The mixture was then extracted with ethyl acetate (3 x 200 mL) and the organic phases combined and back extracted with water (2 x 200 mL). The organic phase was then dried over anhydrous MgSO₄ and solvent evaporated to give the crude product as a brown/red oil. ¹H NMR (300 MHz, DMSO d₆) 8.95 ppm (1H, dd, J₁ = 4.82 Hz, J₂ = 1.65 Hz ), 8.68 ppm (1H, dd, J₁ = 8.20 Hz, J₂ = 1.65 Hz), 7.81 ppm (1H, dd, J₁ = 8.20 Hz, J₂ = 4.82 Hz), 7.51 ppm (1H, m), 7.16-7.28 ppm (3H, m) 5.56, (1H, d, J = 1.34, Hz), 5.28 ppm (1H, s), 2.13 ppm (3H, s). The crude reaction product was used without further purification. The product was suspended in Methyl ethyl ketone (MEK, 100 mL) and HCI (37 %, 8 mL) was added in one portion. The resulting reaction mixture was allowed to stir at 90°C for 6 hours. The reaction was then cooled to room temperature and the desired product 7 was filtered off and dried. 9.3 g (61.5 % over 2 steps) of 7 was collected as a yellow powder. ¹H NMR (400 MHz, DMSO d₆) 11.43 ppm (1H, br s), 8.92 ppm (1H, dd, J₁ = 4.78 Hz, J₂ = 1.60 Hz), 8.64 ppm (1H, dd, J₁ = 8.21 Hz, J₂ = 4.82 Hz), 7.76 ppm (1H, dd, J₁ = 8.21 Hz, J₂ = 1.65 Hz), 7.45 ppm (1H, d, J = 7.82 Hz), 7.08 - 7.20 ppm (3H, m). 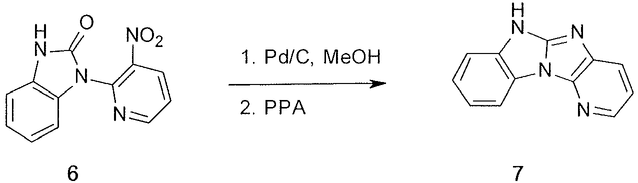
b) Compound 7 (9.7 g, 38 mmol) was dissolved in MeOH (150 mL) and hydrogenated over Pd/C (10%) under 5 bar H₂ at 50°C. After 6 hours, the reaction was complete and the cata-lyst was filtered and solvent evaporated to give the desired product (7 g) as a colourless solid. ¹H NMR (400 MHz, DMSO d₆) 11.04 ppm (1H, br s), 7.81 ppm, (1H, dd J₁ = 4.45 Hz, J₂ = 1.7 Hz), 7.30 ppm (1H, dd, J₁ = 8.13 Hz, J₂ = 1.68 Hz) 7.23 ppm (1H, dd, J₁ = 8.15 Hz, J₂ = 4.42 Hz), 7.06 ppm (2H, m), 6.95 ppm (1H, m), 6.74 ppm (1H, d, J = 7.70 Hz). 5.29 ppm (2H, br s). The crude material was used without further purification. It was taken up in polyphosphoric acid (PPA, 200 g) and heated at 220°C overnight. The reaction was cooled and poured into ice cold saturated K₂CO₃. The precipitate was filtered over a pad of celite and washed copiously with water. The precipitate was collected by washing through with CHCl₃/MeOH solution (4:1). The solvent was evaporated under reduced pressure and the crude product 7 was suspended in acetone and filtered to give 3.15 g (40 % yield) of the desired product as a brown solid which was used without further purification. ¹H NMR (300 MHz, DMSO d₆) 8.18 ppm (1H, dd, J₁ = 4.97 Hz, J₂ = 1.40 Hz), 7.98 ppm (1H, m), 7.85 ppm (1H, dd, J₁ = 7.98 Hz, J₂ = 1.40 Hz), 7.49 ppm (1H, m), 8.13-8.16 pp, (2H, m), 7.23-7.36 (3H, m). 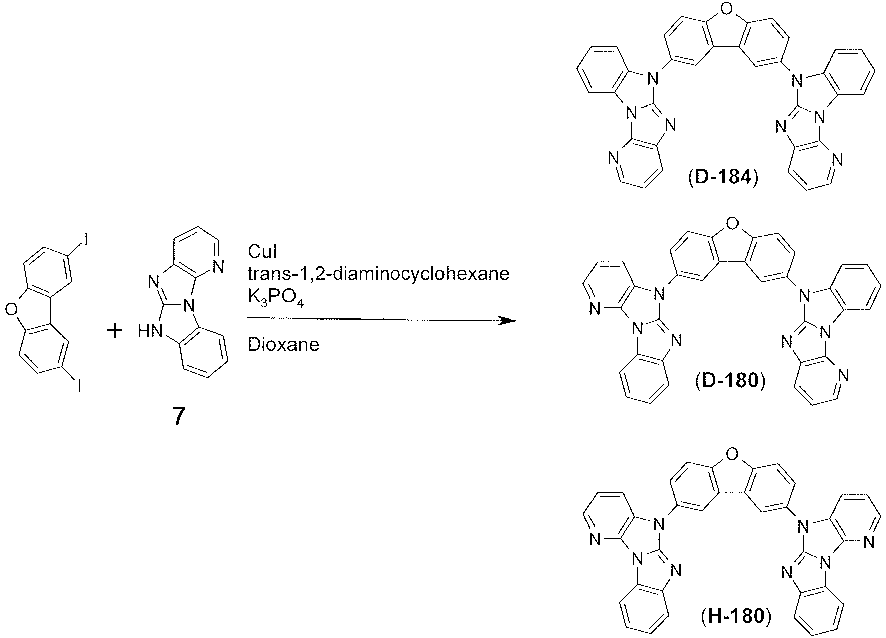
c) 0.38 g 7 (1.8 mmol) was combined with 2,8-diiodobenzofuran (0.38 g, 0.9 mmol), Cul (40 mg, 0.18 mmol), trans1,2-diaminocyclohexane (1.7 mL, 14.9 mmol) and K₃PO₄ (0.77 g, 3.6 mmol) in dioxane (15 mL) and the mixture was degassed under N₂ for 1 hour at room temperature. The reaction was then heated at an oil bath temperature of 110°C for 3 days. The reaction was analysed by HPLC-MS and found 40% conversion to the desired product as a statistical mixture of isomers. The reaction was worked up by filtering while hot. The filter cake was then further washed with dioxane followed by water and allowed to dry. The products D-180, D-184 and H-180 were confirmed by HPLC-MS m/z 581 (M+1).

### Comparative Application Example 1

The ITO substrate used as the anode is first cleaned with an isopropanol in an ultrasonic bath. To eliminate any possible organic residues, the substrate is exposed to a continuous ozone flow in an ozone oven for further 25 minutes. This treatment also improves the hole injection properties of the ITO. Then Plexcore® OC AJ20-1000 (commercially available from Plextronics Inc.) is spin-coated and dried to form a hole injection layer (-40 nm). Thereafter, the organic materials specified below are applied by vapor deposition to the Plexcore® coated substrate at a rate of approx. 0.5-5 nm/min at about 10⁻⁷-10⁻⁹ mbar. As a hole transport, compound 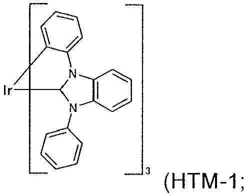 for preparation, see Ir complex (7) in the application WO2005/019373) is applied by vapor deposition in a thickness of 10 nm doped with MoOₓ (∼10%) to improve the conductivity.

As exciton and electron blocker, (HTM-1) is applied to the substrate with a thickness of 10 nm. Subsequently, a mixture of 10% by weight of emitter compound, 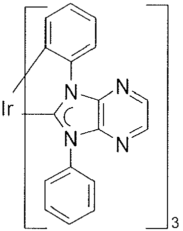 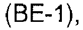 5% by weight of compound (HTM-1) and 85% by weight of host 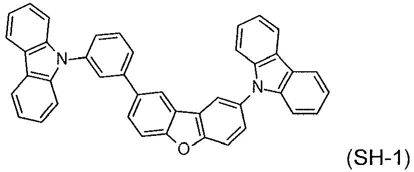 are applied by vapor deposition in a thickness of 40 nm. Subsequently, material (SH-1) is applied by vapour deposition with a thickness of 5 nm as blocker. Thereafter, a 20 nm thick electron transport layer is deposited consisting of 50% by weight of 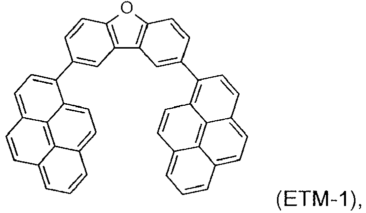 and of 50% of 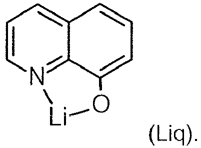 Finally a 2 nm KF layer serves as an electron injection layer and a 100 nm-thick Al electrode completes the device. All fabricated parts are sealed with a glass lid and a getter in an inert nitrogen atmosphere.

### Application Examples 1 and 2

### OLED characterization

To characterize the OLED, electroluminescence spectra are recorded at various currents and voltages. In addition, the current-voltage characteristic is measured in combination with the light output emitted. The light output is converted to photometric parameters by calibration with a photometer. The results are shown in Table 1. Data are given at luminance (L) = 1000 Cd/m² except otherwise stated.

Comparative Application Example 1 is repeated except that exciton and electron blocker, (HTM-1) is replaced by compounds 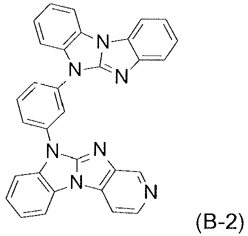 and 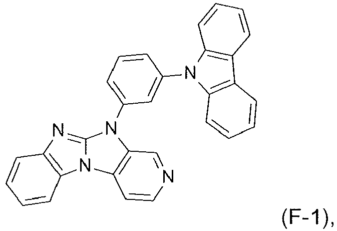 respectively. The device results are shown in Table 1. Data are given at luminance (L) = 1000 Cd/m² except otherwise stated.

**Table 1**

| Appl. Ex. | Exiton and electron blocker | U [V] | EQE [%] | CIEx | CIEy |
|---|---|---|---|---|---|
| 1 | (B-2) | 7.86 | 13.55 | 0.162 | 0.292 |
| 2 | (F-1) | 7.62 | 13.69 | 0.160 | 0.284 |
| Comp. Appl. Ex. 1 | (HTM-1) | 7.24 | 13.26 | 0.160 | 0.283 |

The results shown in Table 1 demonstrated that the EQE is improved when using compounds (B-2) and (F-1) as exiton and electron blocker instead of reference compound (HTM-1).

### Application Example 3

Comparative Application Example 1 is repeated except that host and blocker, (SH-1), are fully replaced by compound 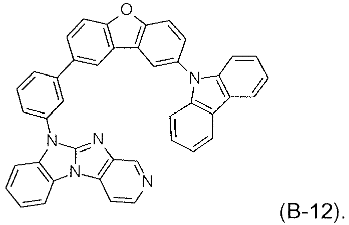 The device results are shown in Table 2. Data are given at luminance (L) = 1000 Cd/m² except otherwise stated.

**Table 2**

| Appl. Ex. | Host and blocker | U [V] | EQE [%] | CIEx | CIEy |
|---|---|---|---|---|---|
| 3 | (B-12) | 7.55 | 11.1 | 0.167 | 0.316 |
| Comp. Appl. Ex. 1 | (SH-1) | 7.85 | 12.9 | 0.162 | 0.297 |

The results shown in Table 3 demonstrated that the voltage is reduced when using compound (B-12) as host and blocker instead of reference compound (SH-1).

## Claims

1. A compound of the formula 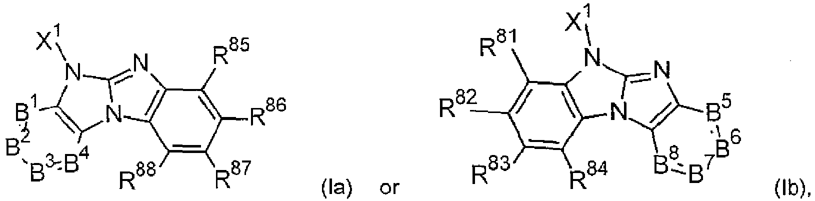 wherein
B¹ is N, or CR⁸¹,
B² is N, or CR⁸²,
B³ is N, or CR⁸³,
B⁴ is N, or CR⁸⁴,
B⁵ is N, or CR⁸⁵,
B⁶ is N, or CR⁸⁶,
B⁷ is N, or CR⁸⁷,
B⁸ is N, or CR⁸⁸,
R⁸¹, R⁸², R⁸³, R⁸⁴, R⁸⁵, R⁸⁶, R⁸⁷ and R⁸⁸ are H;
X¹ is a group of formula -(A¹)ₒ-(A²)ₚ-(A³)_{q}-(A⁴)ᵣ-R¹⁶,
o is 0, or 1, p is 0, or 1, q is 0, or 1, r is 0, or 1,
R¹⁶ is H, -NR¹⁰R¹¹, or -Si(R¹²)(R¹³)(R¹⁴), a C₆-C₂₄aryl group, which can optionally be substituted by G; or a C₂-C₃₀heteroaryl group, which can optionally be substituted by G;
R¹⁰ and R¹¹ are independently of each other a C₆-C₂₄aryl group, which can optionally be substituted by G; or a C₂-C₃₀heteroaryl group, which can optionally be substituted by G;
R¹², R¹³ and R¹⁴ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G;
A¹, A², A³ and A⁴ are independently of each other a C₆-C₂₄arylen group, which can optionally be substituted by G, or a C₂-C₃₀heteroarylen group, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
E is -OR69, -SR⁶⁹, -NR⁶⁵R⁶⁶, -COR⁶⁸, -COOR⁶⁷, -CONR⁶⁵R⁶⁶, -CN, or F,
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁₋C₁₈alkyl, or C₁-C₁₈alkoxy; a C₁-C₁₈alkyl group; or a C₁-C₁₈alkyl group, which is interrupted by - O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁₋C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl, with the proviso that
one of the substituents B¹, B², B³, B⁴ in formula (la) and one of the substituents B⁵, B⁶, B⁷ and B⁸ in formula (lb) represents N;
and R¹⁶ is different from H, if o is 0, p is 0, q is 0 and r is 0.

2. The compound according to claim 1, which is a compound of the formula 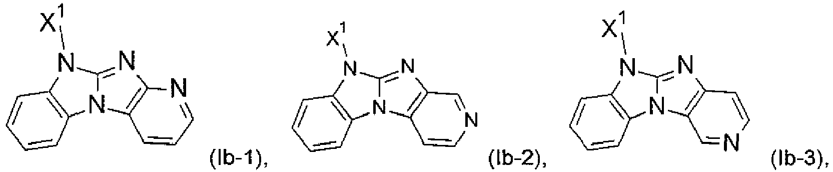 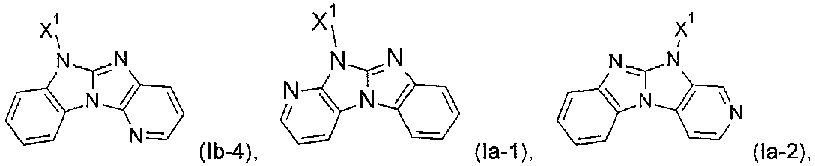 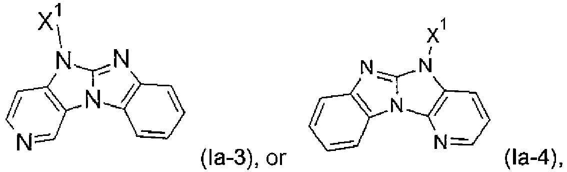 wherein
X¹ is defined in claim 1.

3. The compound according to claim 1 or 2, wherein
X¹ is a group of formula -(A¹)ₒ-(A²)ₚ-(A³)_{q}-(A⁴)ᵣ-R¹⁶,
o is 0, or 1, p is 0, or 1, q is 0, or 1, r is 0, or 1,
A¹, A², A³ and A⁴ are independently of each other a group of formula 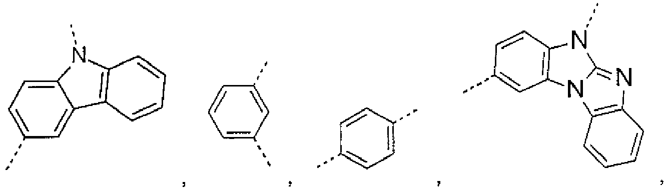 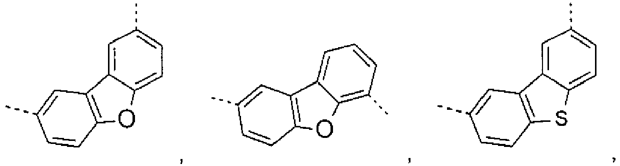 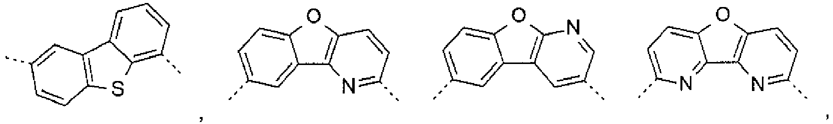 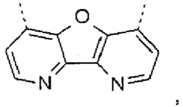 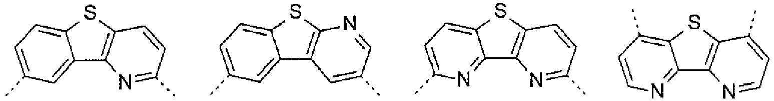 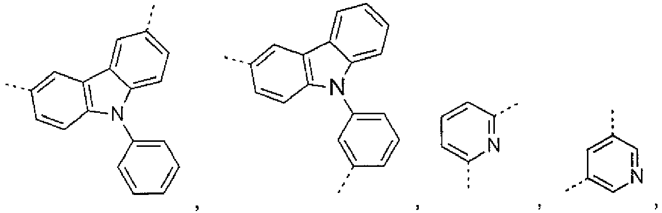 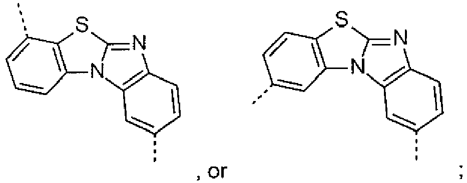 R¹⁶ is a group of formula

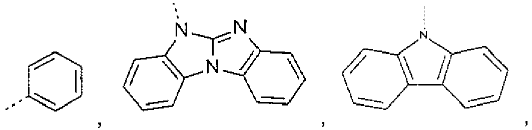

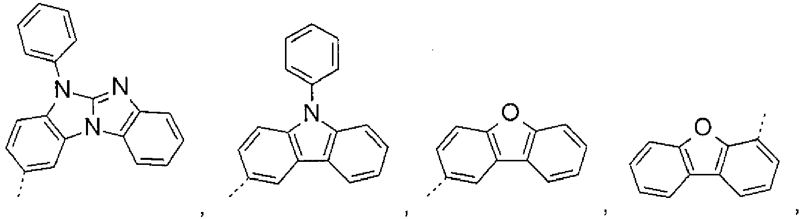

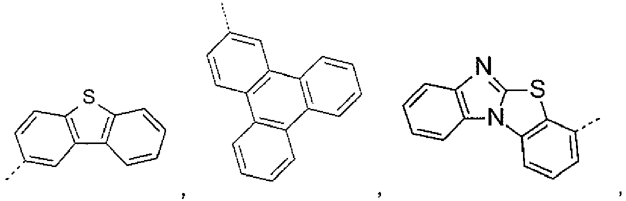

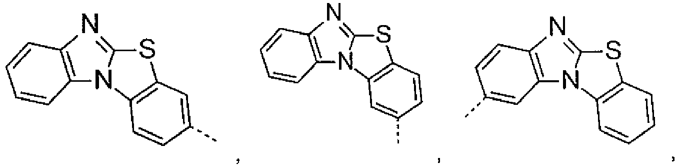

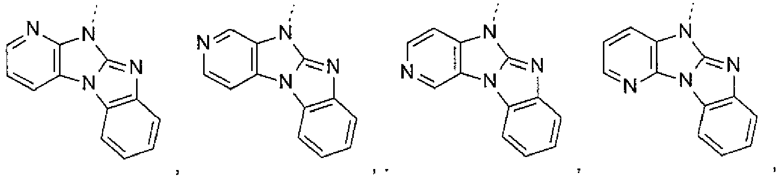

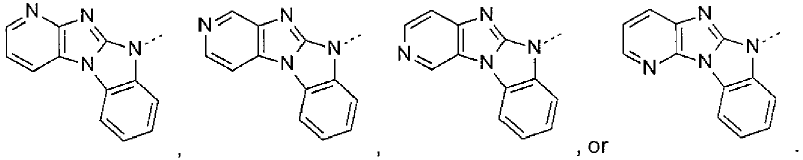

4. The compound according to any of claims 1 to 3, wherein
X¹ is a group of formula -(A¹)ₒ-(A²)ₚ-(A³)_{q}-(A⁴)ᵣ-R¹⁶, wherein
-(A¹)ₒ-(A²)ₚ-(A³)_{q}-(A⁴)ᵣ- is a group of formula 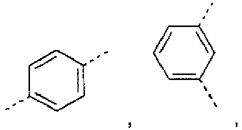 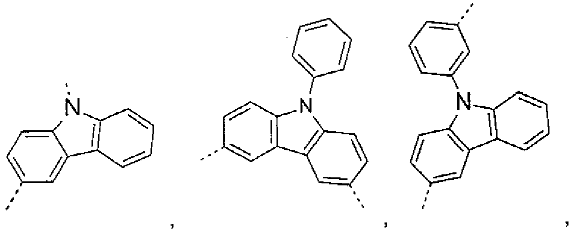 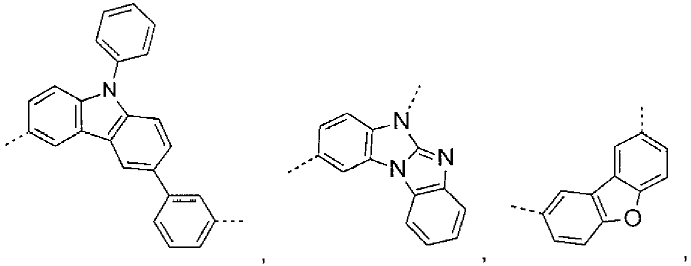 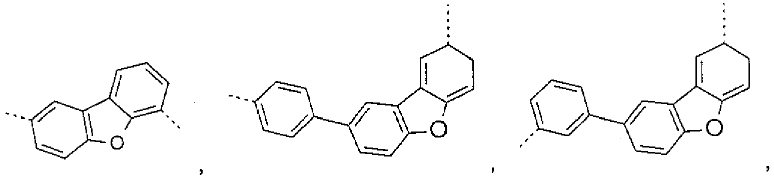 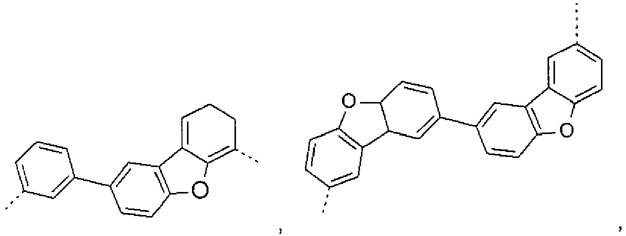 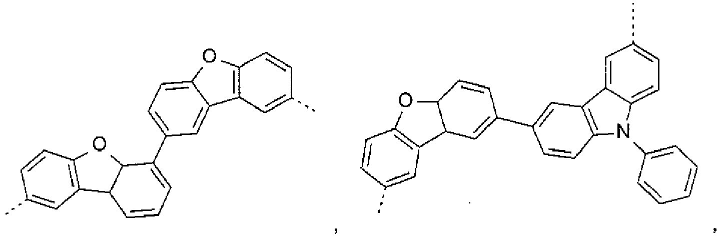 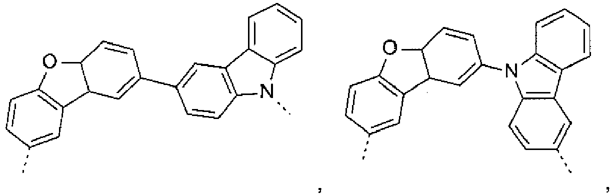 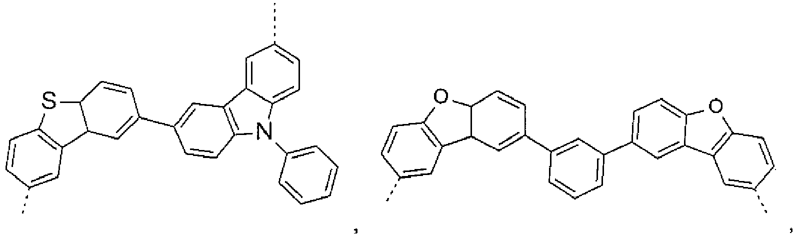 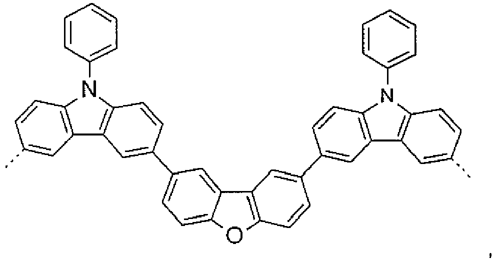 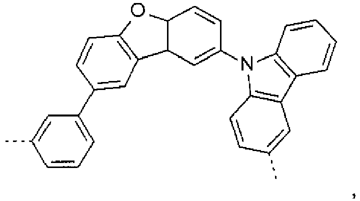 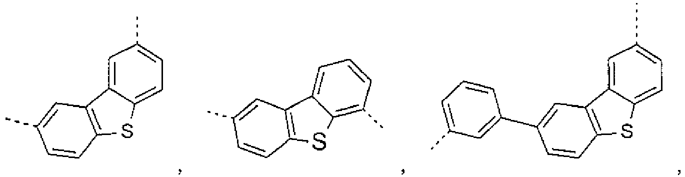 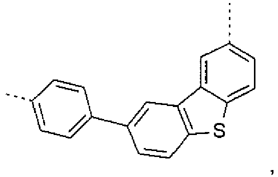 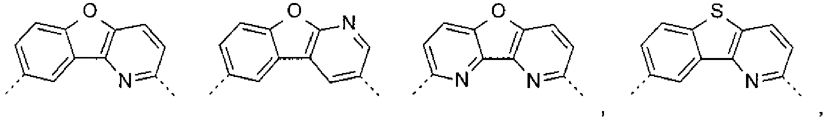 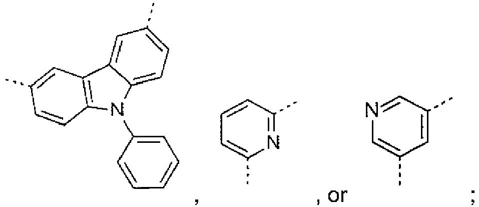 R¹⁶ is a group of formula

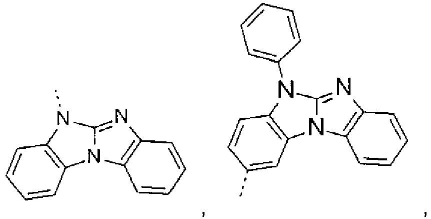

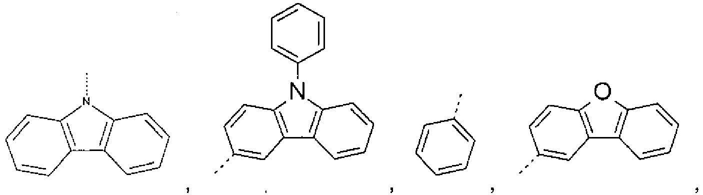

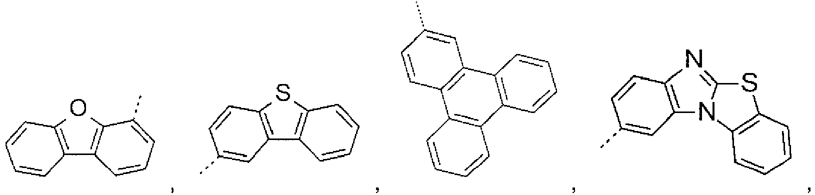

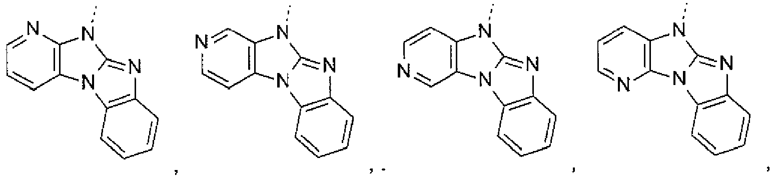

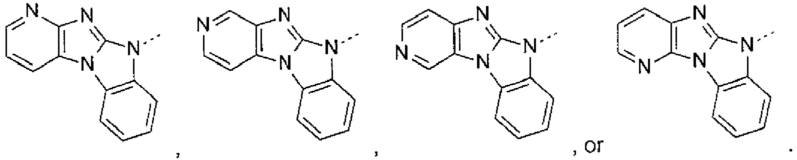

5. The compound according to claim 2, which is a compound of the formula 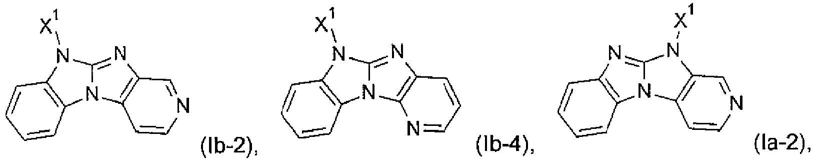 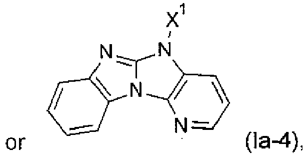 wherein
X¹ is a group of formula -(A¹)ₒ-(A²)ₚ-(A³)_{q}-(A⁴)ᵣ-R¹⁶,
o is 0, or 1, p is 0, or 1, q is 0, or 1, r is 0, or 1,
A¹, A², A³ and A⁴ are independently of each other a group of formula 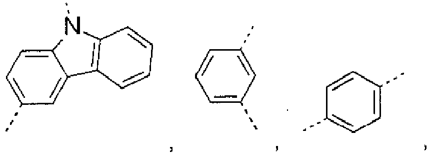 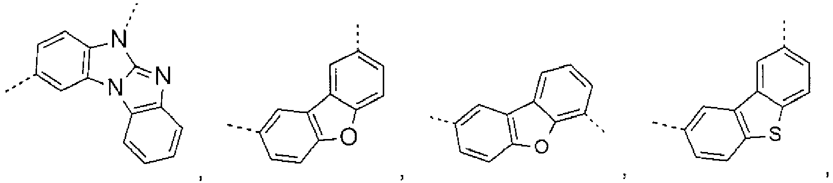 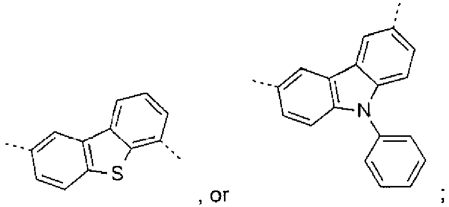 and
R¹⁶ is a group of formula

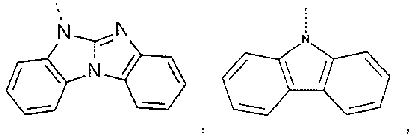

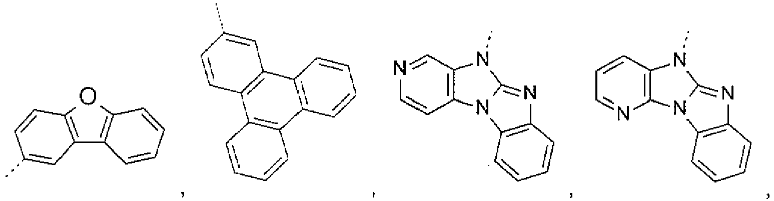

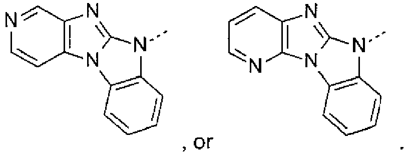

6. An electronic device, comprising a compound according to any of claims 1 to 5.

7. The electronic device according to claim 6, which is an electroluminescent device.

8. A charge transport layer, a charge/exciton blocker layer, or an emitting layer comprising a compound according to any of claims 1 to 5.

9. The emitting layer according to claim 8, comprising a compound according to any of claims 1 to 5 as host material in combination with a phosphorescent emitter.

10. An apparatus selected from the group consisting of stationary visual display units; mobile visual display units; illumination units; keyboards; items of clothing; furniture; wallpaper, comprising the organic electronic device according to claim 6, or 7, or the charge transport layer, the charge/exciton blocker layer, or the emitting layer according to claim 8.

11. Use of the compounds of formula I according to any of claims 1 to 5 for electrophotographic photoreceptors, photoelectric converters, organic solar cells, switching elements, organic light emitting field effect transistors, image sensors, dye lasers and electroluminescent devices.

## Patentansprüche

1. Verbindung der Formel 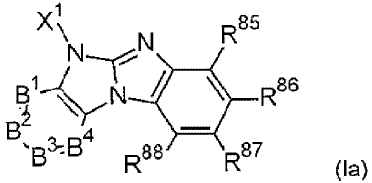 oder 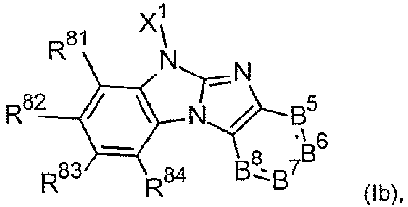 wobei
B¹ für N oder CR⁸¹ steht,
B² für N oder CR⁸² steht,
B³ für N oder CR⁸³ steht,
B⁴ für N oder CR⁸⁴ steht,
B⁵ für N oder CR⁸⁵ steht,
B⁶ für N oder CR⁸⁶ steht,
B⁷für N oder CR⁸⁷ steht,
B⁸ für N oder CR⁸⁸ steht,
R⁸¹, R⁸², R⁸³, R⁸⁴, R⁸⁵, R⁸⁶, R⁸⁷ und R⁸⁸ für H stehen,
X¹ für eine Gruppe der Formel -(A¹)ₒ-(A²)ₚ-(A³)_{q}-(A⁴)ᵣ-R¹⁶ steht,
o für 0 oder 1 steht, p für 0 oder 1 steht, q für 0 oder 1 steht, r für 0 oder 1 steht,
R¹⁶ für H, -NR¹⁰R¹¹ oder -Si (R¹²) (R¹³) (R¹⁴), eine C₆-C₂₄-Arylgruppe, die gegebenenfalls durch G substituiert sein kann, oder eine C₂-C₃₀-Heteroarylgruppe, die gegebenenfalls durch G substituiert sein kann, steht,
R¹⁰ und R¹¹ unabhängig voneinander für eine C₆-C₂₄-Arylgruppe, die gegebenenfalls durch G substituiert sein kann, oder eine C₂-C₃₀-Heteroaryl-gruppe, die gegebenenfalls durch G substituiert sein kann, stehen,
R¹², R¹³ und R¹⁴ unabhängig voneinander für eine C₁-C₂₅-Alkylgruppe, die gegebenenfalls durch E substituiert und oder durch D unterbrochen sein kann, eine C₆-C₂₄-Arylgruppe, die gegebenenfalls durch G substituiert sein kann, oder eine C₂-C₃₀-Hetero-arylgruppe, die gegebenenfalls durch G substituiert sein kann, stehen,
A¹, A², A³ und A⁴ unabhängig voneinander für eine C₆-C₂₄-Arylengruppe, die gegebenenfalls durch G substituiert sein kann, oder eine C₂-C₃₀-Hetero-arylengruppe, die gegebenenfalls durch G substituiert sein kann, stehen,
D für -CO-, -COO-, -S-, -SO-, -SO₂-, -O-, -NR⁶⁵-, -SiR⁷⁰R⁷¹-, -POR⁷²-, -CR⁶³=CR⁶⁴- oder -C=C- steht,
E für -OR⁶⁹, -SR⁶⁹, -NR⁶⁵R⁶⁶, -COR⁶⁸, -COOR⁶⁷, -CONR⁶⁵R⁶⁶, -CN oder F steht,
G für E oder eine C₁-C₁₈-Alkylgruppe, eine C₆-C₂₄-Arylgruppe, eine C₆-C₂₄-Arylgruppe, die durch F, C₁-C₁₈-Alkyl oder C₁-C₁₈-Alkyl, das durch O unterbrochen ist, substituiert ist, eine C₂-C₃₀-Hetero-arylgruppe oder eine C₂-C₃₀-Heteroarylgruppe, die durch F, C₁-C₁₈-Alkyl oder C₁-C₁₈-Alkyl, das durch O unterbrochen ist, substituiert ist, steht,
R⁶³ und R⁶⁴ unabhängig voneinander für H, C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -O- unterbrochen ist, stehen,
R⁶⁵ und R⁶⁶ unabhängig voneinander für eine C₆-C₁₈-Arylgruppe, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, stehen oder
R⁶⁵ und R⁶⁶ zusammen einen fünf- oder sechsgliedrigen Ring bilden,
R⁶⁷ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁶⁸ für H, eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁶⁹ für ein C₆-C₁₈-Aryl, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁷⁰ und R⁷¹ unabhängig voneinander für eine C₁-C₁₈-Alkylgruppe, eine C₆-C₁₈-Arylgruppe oder eine C₆-C₁₈-Arylgruppe, die durch C₁-C₁₈-Alkyl substituiert ist, stehen und
R⁷² für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht, mit der Maßgabe, dass
einer der Substituenten B¹, B², B³ und B⁴ in Formel (Ia) und einer der Substituenten B⁵, B⁶, B⁷ und B⁸ in Formel (Ib) für N steht
und R¹⁶ von H verschieden ist, wenn o für 0 steht, p für 0 steht, q für steht und r für 0 steht.

2. Verbindung nach Anspruch 1, bei der es sich um eine Verbindung der Formel 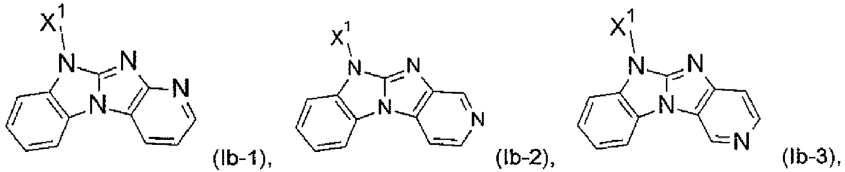 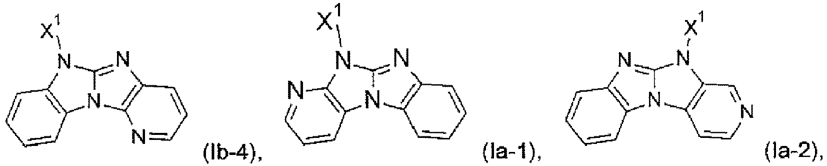 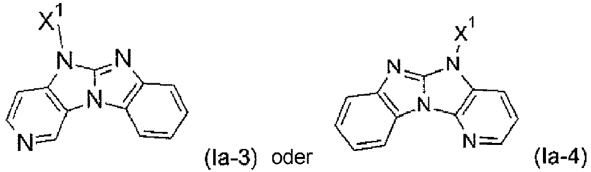 handelt, wobei
X¹ wie in Anspruch 1 definiert ist.

3. Verbindung nach Anspruch 1 oder 2, wobei
X¹ für eine Gruppe der Formel -(A¹)ₒ-(A²)ₚ-(A³)_{q}-(A⁴)ᵣ-R¹⁶ steht,
o für 0 oder 1 steht, p für 0 oder 1 steht, q für 0 oder 1 steht, r für 0 oder 1 steht,
A¹, A², A³ und A⁴ unabhängig voneinander für eine Gruppe der Formel 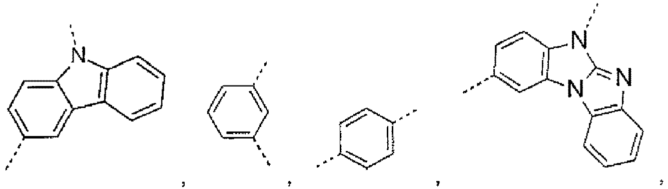 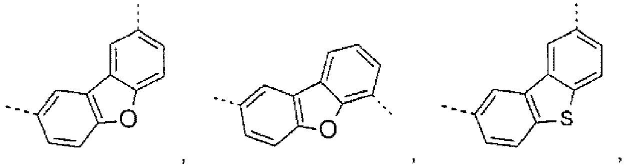 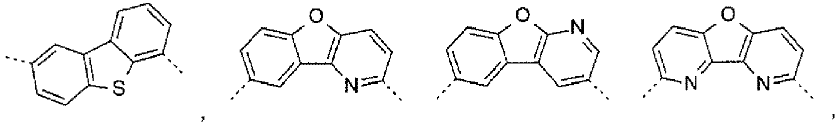 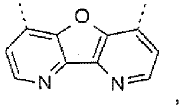 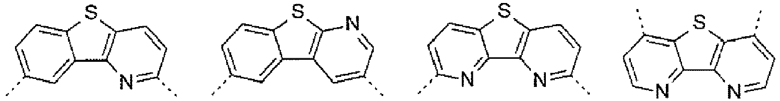 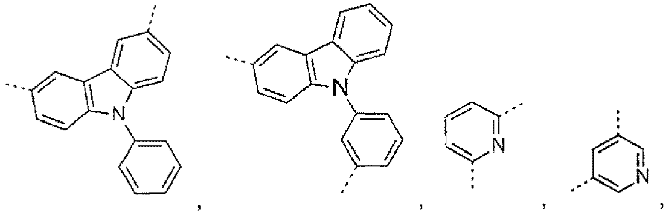 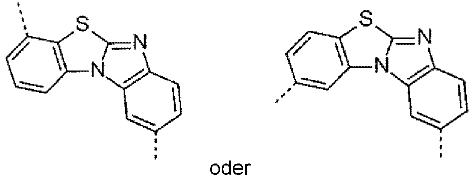 stehen,
R¹⁶ für eine Gruppe der Formel 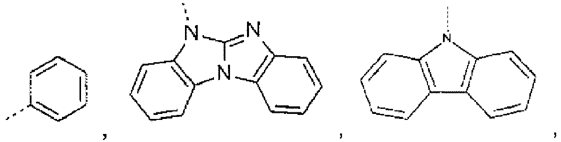 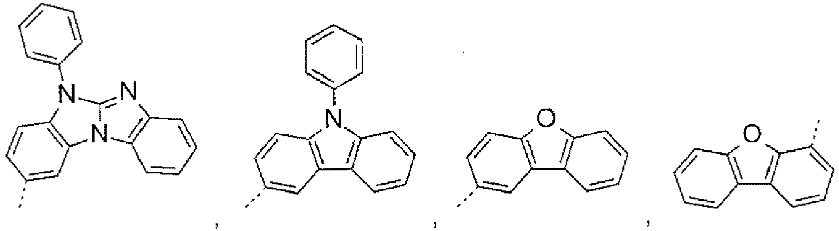 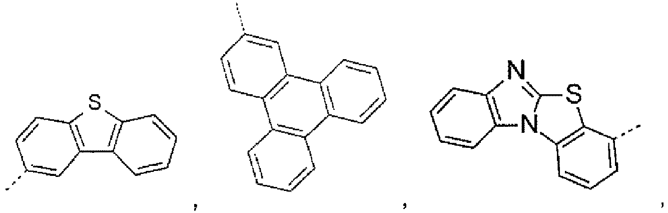 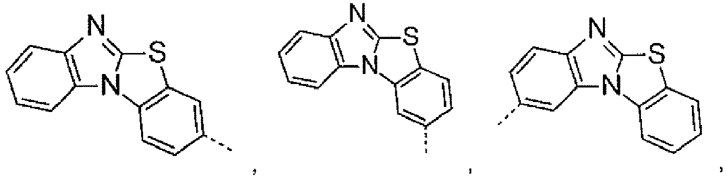 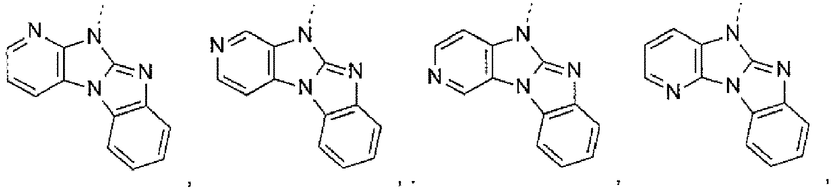 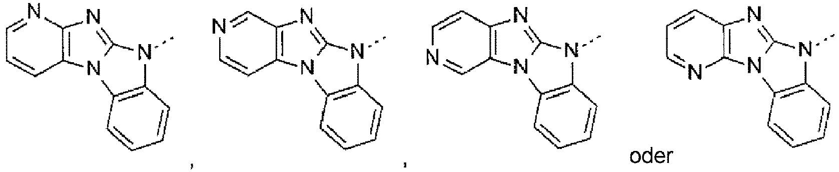 steht.

4. Verbindung nach einem der Ansprüche 1 bis 3, wobei X¹ für eine Gruppe der Formel -(A¹)ₒ-(A²)ₚ-(A³)_{q}-(A⁴)ᵣ-R¹⁶ steht, wobei
-(A¹)ₒ-(A²)ₚ-(A³)_{q}-(A⁴)ᵣ-R¹⁶ für eine Gruppe der Formel 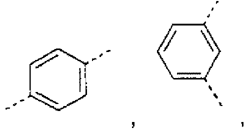 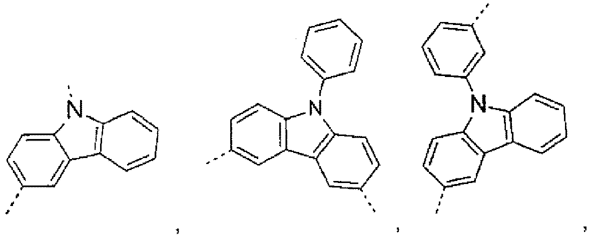 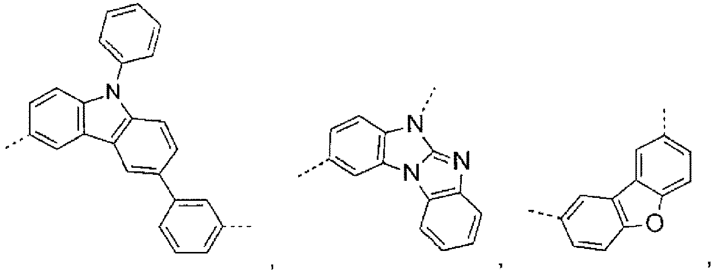 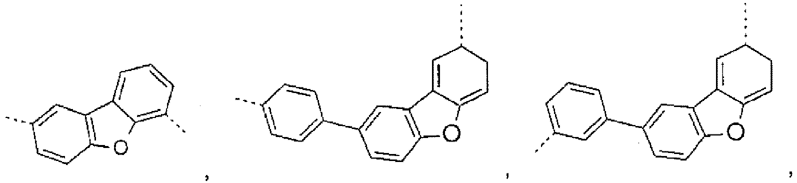 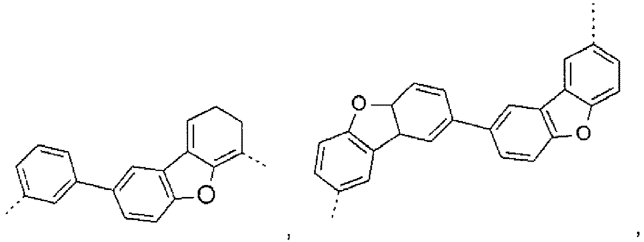 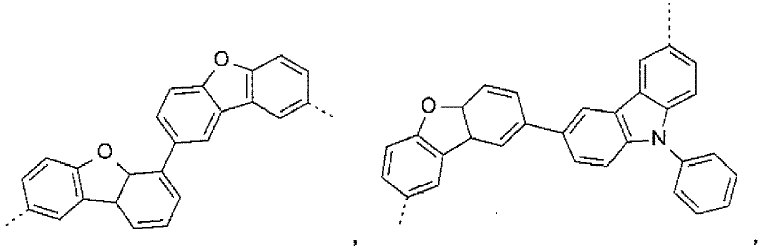 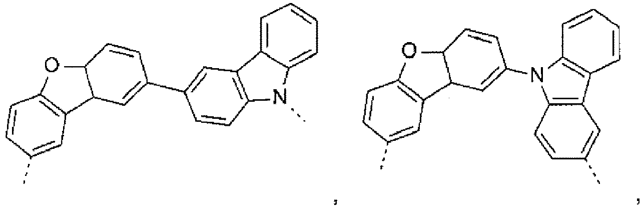 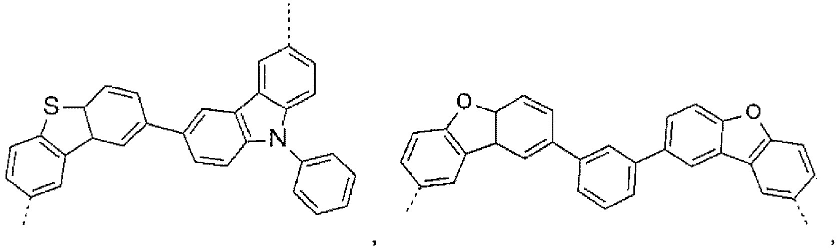 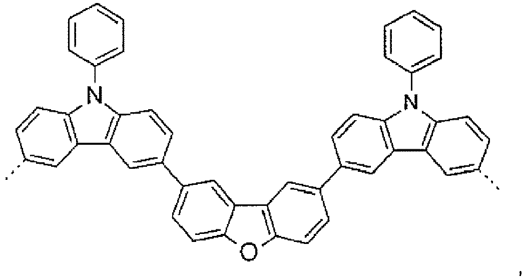 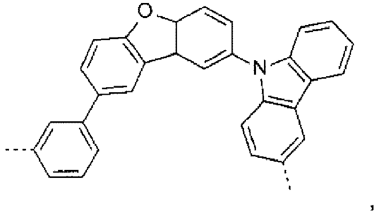 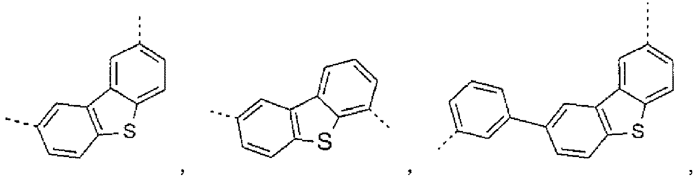 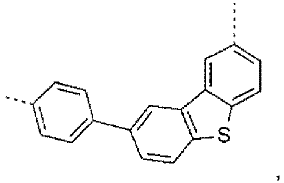 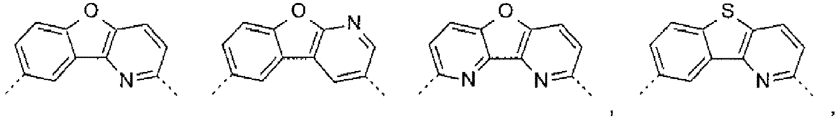 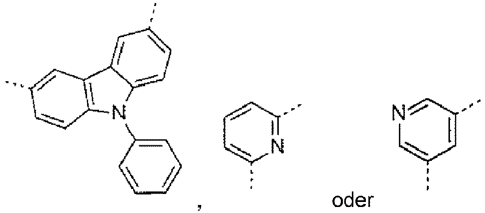 steht;
R¹⁶ für eine Gruppe der Formel 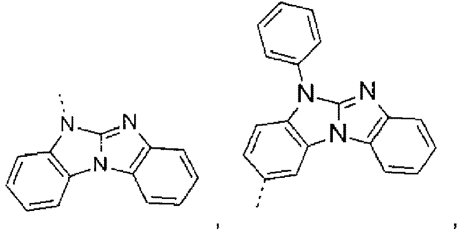 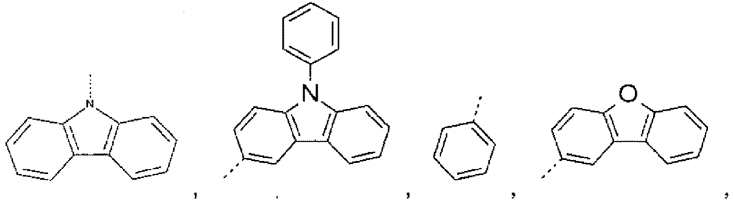 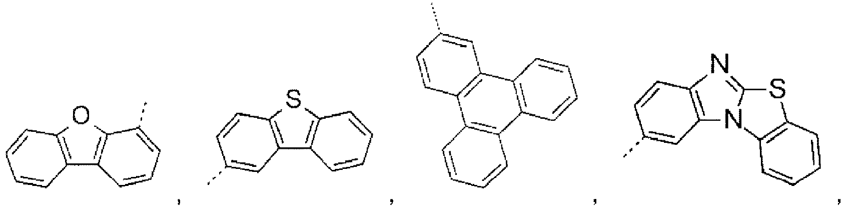 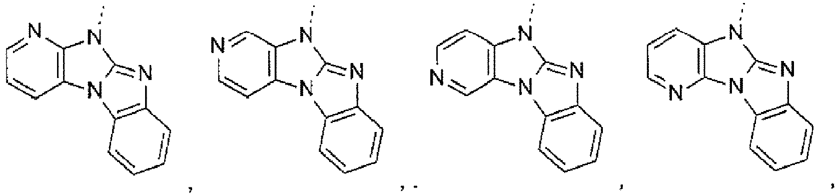 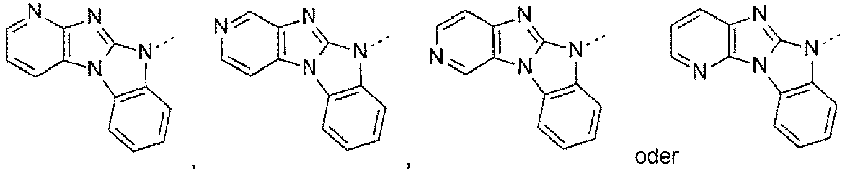 steht.

5. Verbindung nach Anspruch 2, bei der es sich um eine Verbindung der Formel 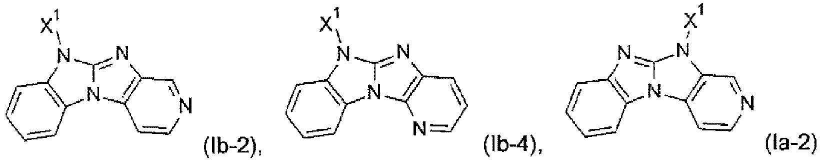 oder 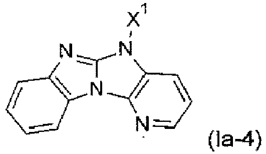 handelt, wobei
X¹ für eine Gruppe der Formel -(A¹)ₒ-(A²)ₚ-(A³)_{q}-(A⁴)ᵣ-R¹⁶ steht,
o für 0 oder 1 steht, p für 0 oder 1 steht, q für 0 oder 1 steht, r für 0 oder 1 steht,
A¹, A², A³ und A⁴ unabhängig voneinander für eine Gruppe der Formel 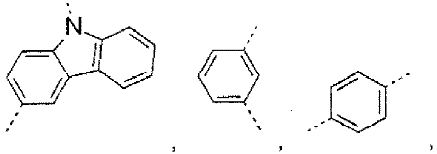 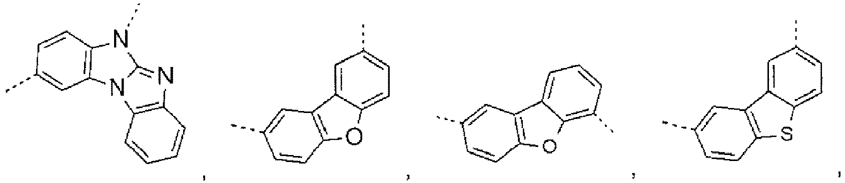 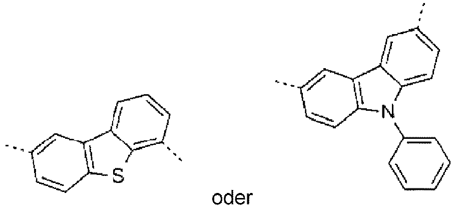 stehen und
R¹⁶ für eine Gruppe der Formel 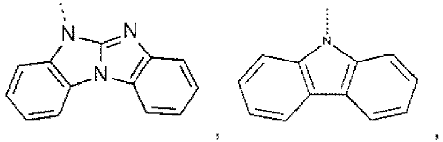 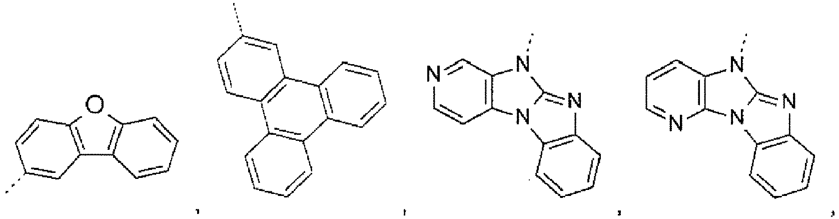 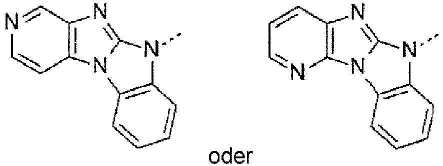 steht.

6. Elektronische Vorrichtung, umfassend eine Verbindung nach einem der Ansprüche 1 bis 5.

7. Elektronische Vorrichtung nach Anspruch 6, bei der es sich um eine Elektrolumineszenzvorrichtung handelt.

8. Ladungstransportschicht, Ladungs-/Exzitonen-Blockerschicht oder Emissionsschicht, umfassend eine Verbindung nach einem der Ansprüche 1 bis 5.

9. Emissionsschicht nach Anspruch 8, umfassend eine Verbindung nach einem der Ansprüche 1 bis 5 als Wirtsmaterial in Kombination mit einem phosphoreszierenden Emitter.

10. Apparatur, ausgewählt aus der Gruppe bestehend aus stationären Bildschirmen; mobilen Bildschirmen; Beleuchtungseinheiten; Tastaturen; Kleidungsstücken; Möbeln; Tapeten, umfassend die organische elektronische Vorrichtung nach Anspruch 6 oder 7 oder die Ladungstransportschicht, die Ladungs-/Exzitonen-Blockerschicht oder die Emissionsschicht nach Anspruch 8.

11. Verwendung der Verbindungen der Formel I nach einem der Ansprüche 1 bis 5 für elektrophotographische Photorezeptoren, photoelektrische Umwandler, organische Solarzellen, Schaltelemente, organische lichtemittierende Feldeffekttransistoren, Bildsensoren, Farbstofflaser und Elektrolumineszenzvorrichtungen.

## Revendications

1. Composé de formule 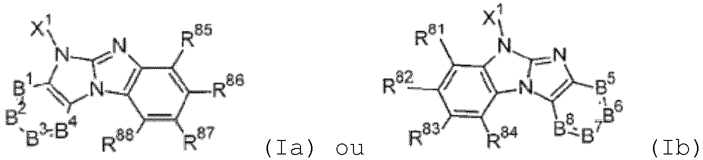
B¹ représentant N ou CR⁸¹,
B² représentant N ou CR⁸²,
B³ représentant N ou CR⁸³,
B⁴ représentant N ou CR⁸⁴,
B⁵ représentant N ou CR⁸⁵,
B⁶ représentant N ou CR⁸⁶,
B⁷ représentant N ou CR⁸⁷,
B⁸ représentant N ou CR⁸⁸,
R⁸¹, R⁸², R⁸³, R⁸⁴, R⁸⁵, R⁸⁶, R⁸⁷ et R⁸⁸ représentant H ;
X¹ représentant un groupe de formule -(A¹)ₒ-(A²)ₚ-(A³)_{q}-(A⁴)ᵣ-R¹⁶,
o représentant 0 ou 1, p représentant 0 ou 1, q représentant 0 ou 1, r représentant 0 ou 1,
R¹⁶ représentant H, -NR¹⁰R¹¹ ou -Si (R¹²) (R¹³) (R¹⁴) , un groupe C₆₋₂₄-aryle, pouvant éventuellement être substitué par G ; ou un groupe C₂₋₃₀-hétéroaryle, pouvant éventuellement être substitué par G ;
R¹⁰ et R¹¹ représentant indépendamment l'un de l'autre un groupe C₆₋₂₄-aryle, pouvant éventuellement être substitué par G ; ou un groupe C₂₋₃₀-hétéroaryle, pouvant éventuellement être substitué par G ;
R¹², R¹³ et R¹⁴ représentant indépendamment les uns des autres un groupe C₁₋₂₅-alkyle, pouvant éventuellement être substitué par E et/ou interrompu par D ; un groupe C₆₋₂₄-aryle, pouvant éventuellement être substitué par G ; ou un groupe C₂₋₃₀-hétéroaryle, pouvant éventuellement être substitué par G ;
A¹, A², A³ et A⁴ représentant indépendamment les uns des autres un groupe C₆₋₂₄-arylène, pouvant éventuellement être substitué par G, ou un groupe C₂₋₃₀-hétéroarylène, pouvant éventuellement être substitué par G ;
D représentant -CO-, -COO-, -S-, -SO-, -SO₂-, - O-, -NR⁶⁵-, SiR⁷⁰R⁷¹- , -POR⁷²-, -CR⁶³=C⁶⁴- ou -C=C-,
E représentant -OR⁶⁹, -SR⁶⁹, -NR⁶⁵R⁶⁶, -COR⁶⁸, - COOR⁶⁷, -CONR⁶⁵R⁶⁶, -CN ou F,
G représentant E ou un groupe C₁₋₁₈-alkyle, un groupe C₆₋₂₄-aryle, un groupe C₆₋₂₄-aryle, substitué par F, C₁₋₁₈-alkyle ou C₁₋₁₈-alkyle interrompu par O ; un groupe C₂₋₃₀-hétéroaryle ou un groupe C₂₋₃₀-hétéroaryle, substitué par F, C₁₋₁₈-alkyle ou C₁₋₁₈-alkyle interrompu par O ;
R⁶³ et R⁶⁴ représentant indépendamment l'un de l'autre H, C₆₋₁₈-aryle ; C₆₋₁₈-aryle substitué par C₁₋₁₈-alkyle ou C₁₋₁₈-alcoxyle ; C₁₋₁₈-alkyle ; ou C₁₋₁₈-alkyle interrompu par -O- ;
R⁶⁵ et R⁶⁶ représentant indépendamment l'un de l'autre un groupe C₆₋₁₈-aryle ; un C₆₋₁₈-aryle substitué par C₁₋₁₈-alkyle ou C₁₋₁₈-alcoxyle ; un groupe C₁₋₁₈-alkyle ; ou un groupe C₁₋₁₈-alkyle interrompu par -O- ; ou
R⁶⁵ et R⁶⁶ formant ensemble un cycle à 5 ou 6 chaînons,
R⁶⁷ représentant un groupe C₆₋₁₈-aryle ; un groupe C₆₋₁₈-aryle, substitué par C₁₋₁₈-alkyle ou C₁₋₁₈-alcoxyle ; un groupe C₁₋₁₈-alkyle ; ou un groupe C₁₋₁₈-alkyle interrompu par -O-,
R⁶⁸ représentant H ; un groupe C₆₋₁₈-aryle ; un groupe C₆₋₁₈-aryle substitué par C₁₋₁₈-alkyle ou C₁₋₁₈-alcoxyle ; un groupe C₁₋₁₈-alkyle ; ou un groupe C₁₋₁₈-alkyle interrompu par -O-,
R⁶⁹ représentant C₆₋₁₈-aryle ; C₆₋₁₈-aryle, substitué par C₁₋₁₈-alkyle ou C₁₋₁₈-alcoxyle ; un groupe C₁₋₁₈-alkyle ; ou un groupe C₁₋₁₈-alkyle interrompu par -O-,
R⁷⁰ et R⁷¹ représentant indépendamment l'un de l'autre un groupe C₁₋₁₈-alkyle, un groupe C₆₋₁₈-aryle ou un groupe C₆₋₁₈-aryle substitué par C₁₋₁₈-alkyle et
R⁷² représentant un groupe C₁₋₁₈-alkyle, un groupe C₆₋₁₈-aryle ou un groupe C₆₋₁₈-aryle substitué par C₁₋₁₈-alkyle, à la condition que
un des substituants B¹, B², B³, B⁴ dans la formule (Ia) et un des substituants B⁵, B⁶, B⁷, B⁸ dans la formule (Ib) représentent N ;
et R¹⁶ est différent de H, si o représente 0, p représente 0, q représente 0 et r représente 0.

2. Composé selon la revendication 1, le composé étant de formule 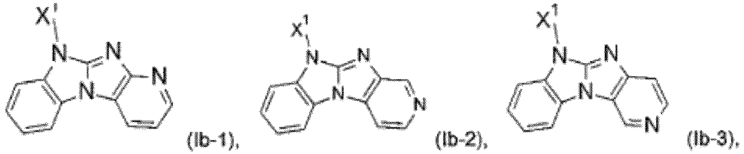 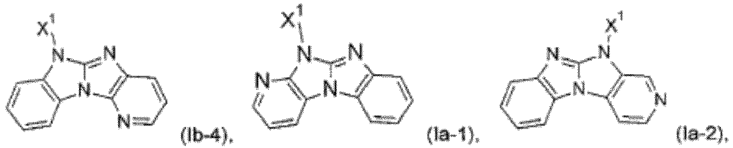 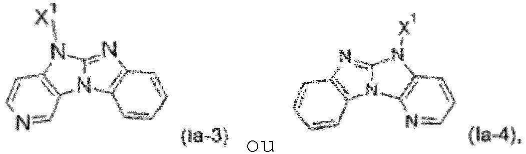 X¹ étant défini selon la revendication 1.

3. Composé selon la revendication 1 ou 2, X¹ représentant un groupe de formule -(A¹)ₒ-(A²)ₚ-(A³)_{q}-(A⁴)ᵣ-R¹⁶,
o représentant 0 ou 1, p représentant 0 ou 1, q représentant 0 ou 1, r représentant 0 ou 1,
A¹, A², A³ et A⁴ représentant indépendamment les uns des autres un groupe de formule 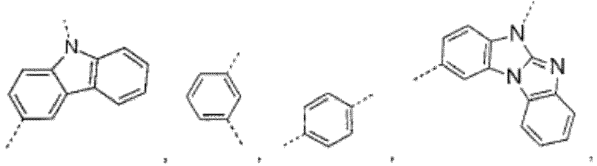 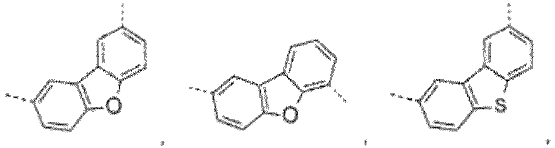 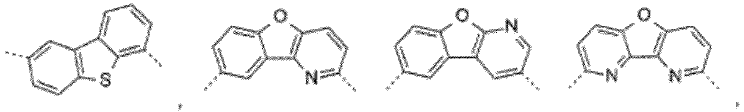 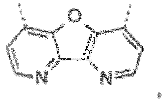 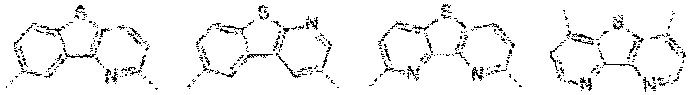 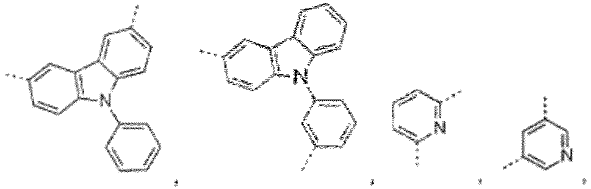 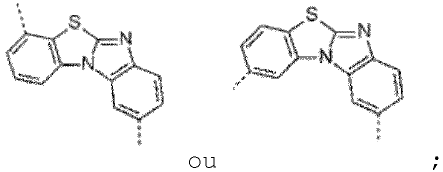 R¹⁶ représentant un groupe de formule

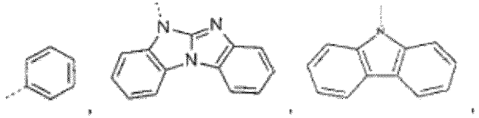

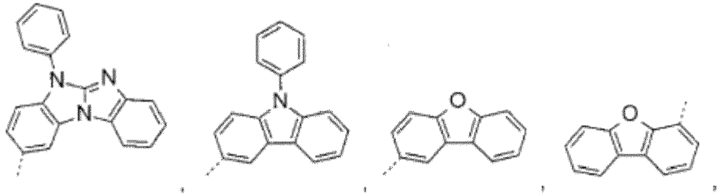

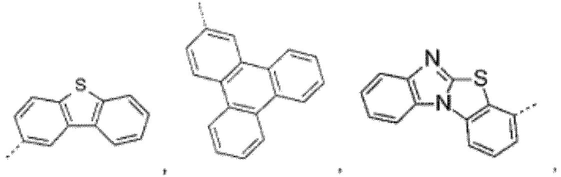

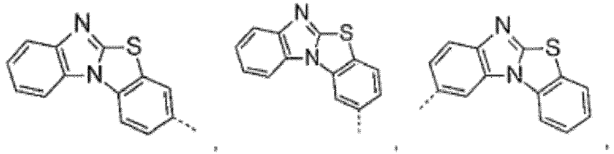

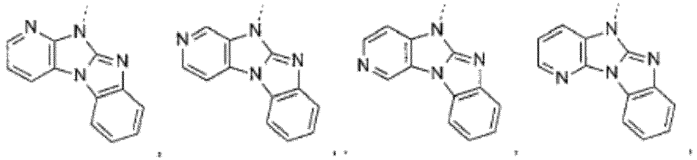

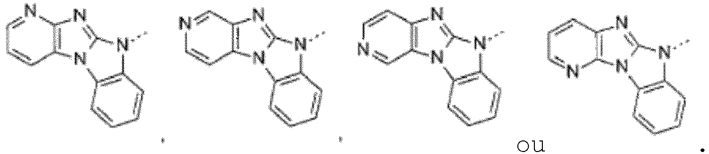

4. Composé selon l'une quelconque des revendications 1 à 3, X¹ représentant un groupe de formule - (A¹)ₒ-(A²)ₚ-(A³)_{q}-(A⁴)ᵣ-R¹⁶, -(A¹)ₒ-(A²)ₚ-(A³)_{q}-(A⁴)ᵣ-R¹⁶ représentant un groupe de formule 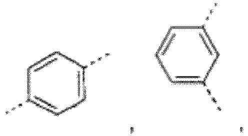 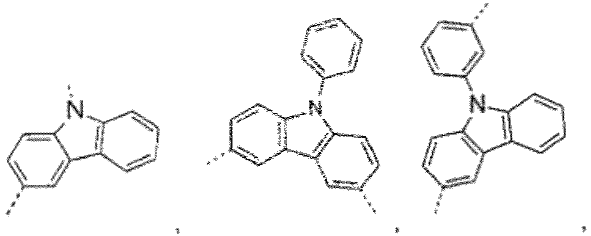 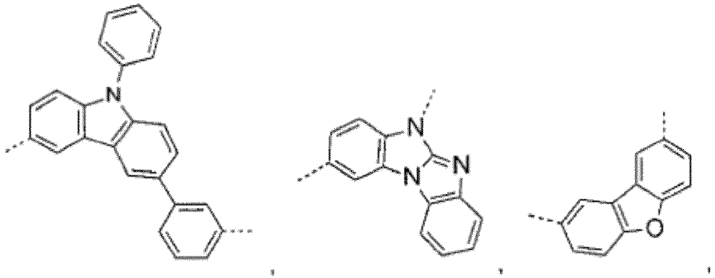 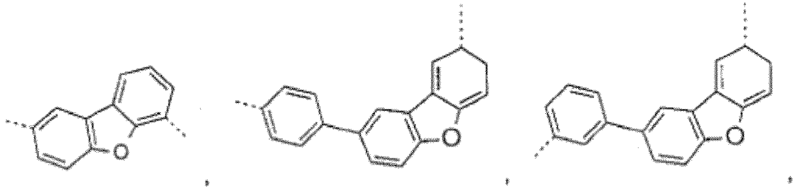 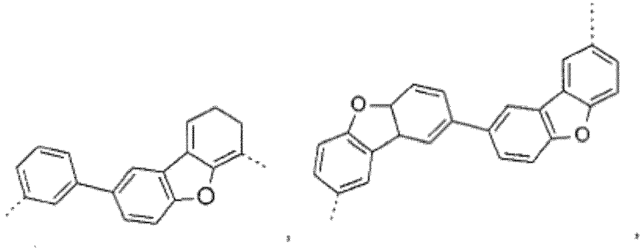 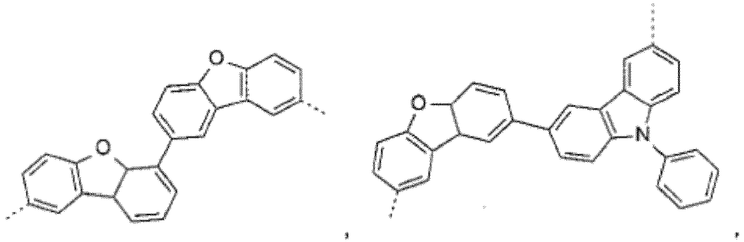 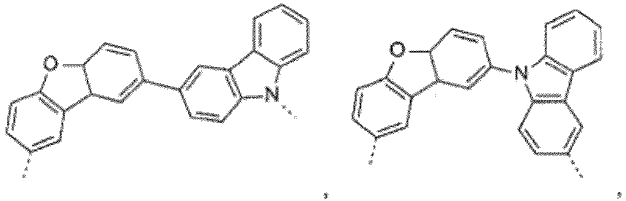 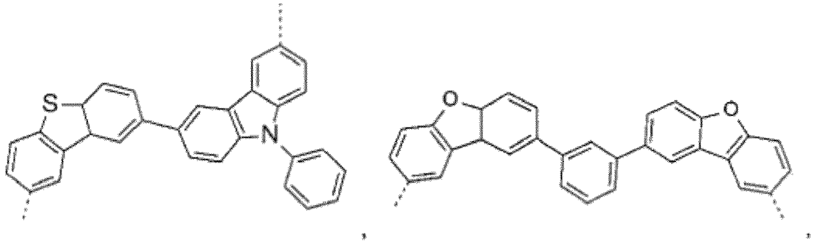 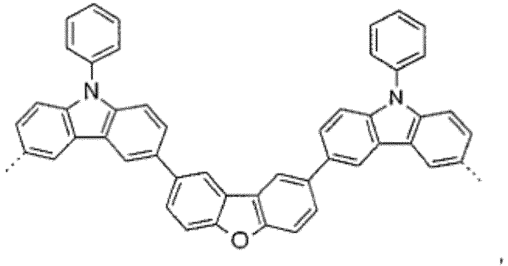 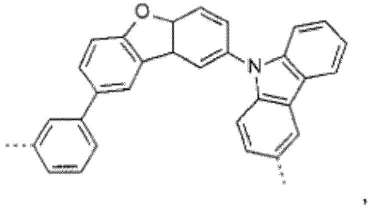 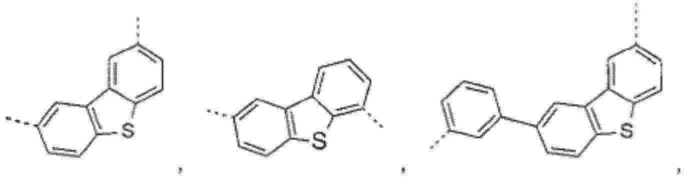 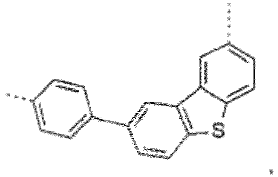 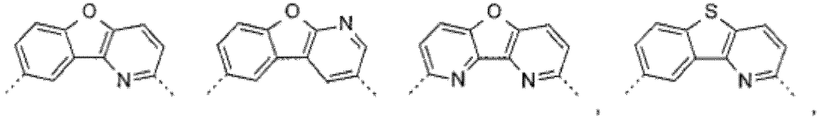 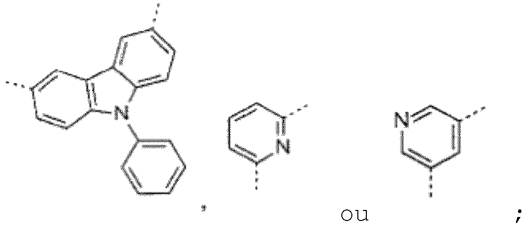 R¹⁶ représentant un groupe de formule 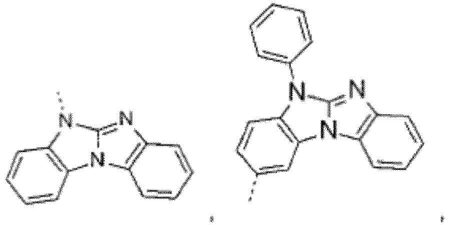 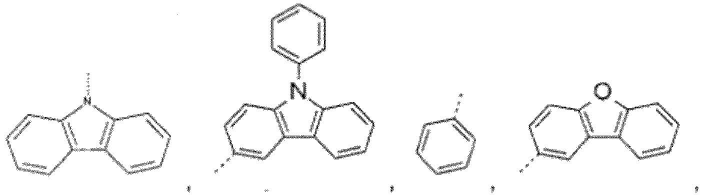 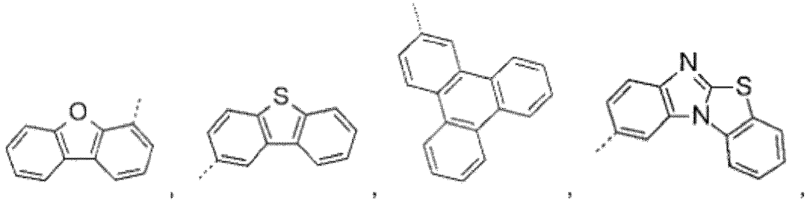 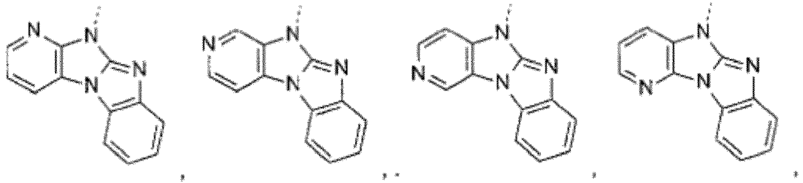 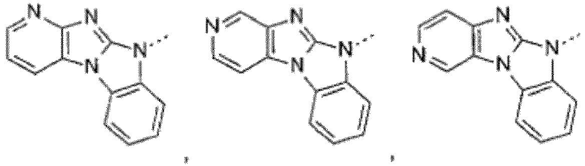 ou

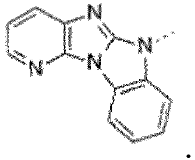

5. Composé selon la revendication 2, le composé étant de formule 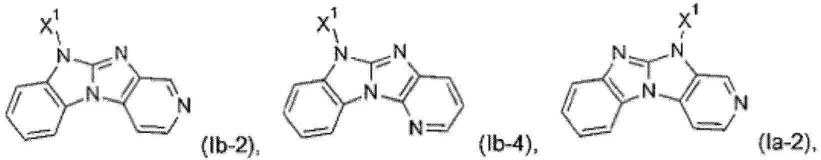 ou 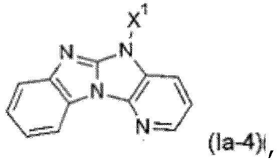
X¹ représentant un groupe de formule -(A¹)ₒ-(A²)ₚ-(A³)_{q}-(A⁴)ᵣ-R¹⁶,
o représentant 0 ou 1, p représentant 0 ou 1, q représentant 0 ou 1, r représentant 0 ou 1,
A¹, A², A³ et A⁴ représentant indépendamment les uns des autres un groupe de formule 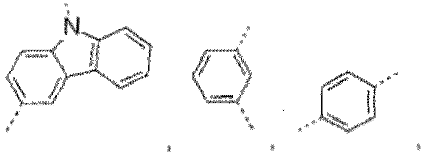 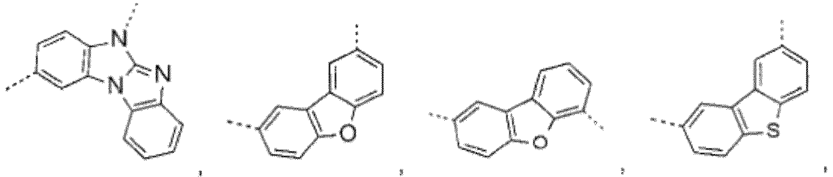 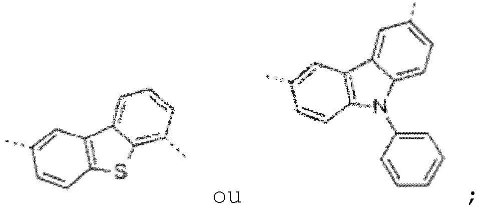 et
R¹⁶ représentant un groupe de formule

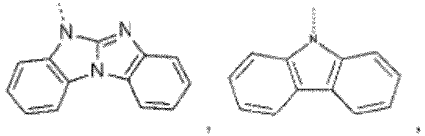

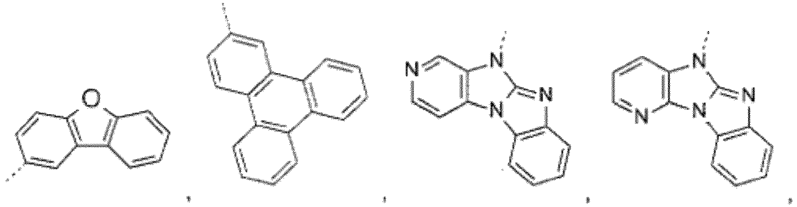

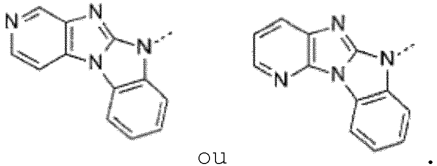

6. Dispositif électronique comprenant un composé selon l'une quelconque des revendications 1 à 5.

7. Dispositif électronique selon la revendication 6, qui est un dispositif électroluminescent.

8. Couche de transport de charge, couche de blocage de charge/exciton ou couche émettrice comprenant un composé selon l'une quelconque des revendications 1 à 5.

9. Couche émettrice selon la revendication 8, comprenant un composé selon l'une quelconque des revendications 1 à 5 en tant que matériau hôte en combinaison avec un émetteur phosphorescent.

10. Appareil choisi dans le groupe constitué par les unités d'affichage visuel stationnaires ; les unités d'affichage visuel mobiles ; les unités d'éclairage ; les claviers ; les articles vestimentaires ; les meubles ; une tapisserie comprenant le dispositif électronique organique selon la revendication 6 ou 7 ou la couche de transport de charge, la couche de blocage de charge/exciton ou la couche émettrice selon la revendication 8.

11. Utilisation des composés de formule I selon l'une quelconque des revendications 1 à 5 pour des photorécepteurs électrophotographiques, des convertisseurs photoélectriques, des cellules solaires organiques, des éléments de commutation, des transistors à effet de champ luminescents organiques, des détecteurs d'image, des lasers à colorant et des dispositifs électroluminescents.
